# EUROPÄISCHE PATENTSCHRIFT

(11) **EP 1 765 788 B1**
(45) Veröffentlichungstag und Bekanntmachung des Hinweises auf die Patenterteilung: **03.06.2009**
(21) Anmeldenummer: 05729376.3
(22) Anmeldetag: 04.04.2005
(51) Int. Cl.: C07D 231/56

(54) **3-AMINOINDAZOLE DERIVATE UND IHRE VERWENDUNG ZUR BEHANDLUNG SGK-BEDINGTEN KRANKHEITEN**
3-AMINOINDAZOLE DERIVATIVES AND THEIR USE FOR THE TREATMENT OF SGK-ASSOCIATED DISEASES
DÉRIVÉS DE 3-AMINOINDAZOLE ET LEUR UTILISATION POUR LE TRAITEMENT DES MALADIES ASSOCIÉES À LA SGK

(30) Priorität: 15.06.2004 DE 102004028862
(43) Veröffentlichungstag der Anmeldung: 28.03.2007
(73) Patentinhaber: Merck Patent GmbH, 64293 Darmstadt (DE)
(72) Erfinder: DORSCH, Dieter, 64372 Ober-Ramstadt (DE); BURGDORF, Lars, Thore, 60389 Frankfurt am Main (DE); GERICKE, Rolf, 64342 Seeheim-Jugenheim (DE); BEIER, Norbert, 64354 Reinheim (DE); MEDERSKI, Werner, 64673 Zwingenberg (DE); LANG, Florian, 72076 Tuebingen (DE)
(86) Internationale Anmeldenummer: PCT/EP2005/003513
(87) Internationale Veröffentlichungsnummer: WO 2005/123688

(56) Entgegenhaltungen:
- WO-A-03/064397
- WO-A-03/097610
- WO-A-20/04022544
- WO-A-20/05011681
- DE-B- 1 280 878

## Beschreibung

Der Erfindung lag die Aufgabe zugrunde, neue Verbindungen mit wertvollen Eigenschaften aufzufinden, insbesondere solche, die zur Herstellung von Arzneimitteln verwendet werden können.

Die vorliegende Erfindung betrifft Verbindungen, bei denen die Hemmung, Regulierung und/oder Modulation der Signaltransduktion von Kinasen, insbesondere der zellvolumenregulierten humanen Kinase h-sgk (human serum and glucocorticoid dependent kinase oder SGK) eine Rolle spielt, ferner pharmazeutische Zusammensetzungen, die diese Verbindungen enthalten, sowie die Verwendung der Verbindungen zur Behandlung SGKbedingter Krankheiten.

Die SGK mit den Isoformen SGK-1, SGK-2 und SGK-3 sind eine Serin/Threonin-Proteinkinase Familie (WO 02/17893).

Die erfindungsgemäßen Verbindungen sind vorzugsweise selektive Inhibitoren der SGK-1. Ferner können sie Inhibitoren der SGK-2 und/oder SGK-3 sein.

Im einzelnen betrifft die vorliegende Erfindung Verbindungen, die die Signaltransduktion der SGK hemmen, regulieren und/oder modulieren, Zusammensetzungen, die diese Verbindungen enthalten, sowie Verfahren zu ihrer Verwendung zur Behandlung von SGK-bedingten Krankheiten und Leiden wie Diabetes (z.B. Diabetes mellitus, diabetische Nephropathie, diabetische Neuropathie, diabetische Angiopathie und Mikroangiopathie), Fettsucht, metabolisches Syndrom (Dyslipidämie), systemische und pulmonale Hypertonie, Herzkreislauferkrankungen (z.B. kardiale Fibrosen nach Myokardinfarkt, Herzhypertrophie und Herzinsuffizienz, Arteriosklerose) und Nierenerkrankungen (z.B. Glomerulosklerose, Nephrosklerose, Nephritis, Nephropathie, Störung der Elektrolytausscheidung), allgemein bei jeglicher Art von Fibrosen und entzündlichen Prozessen (z.B. Leberzirrhose, Lungenfibrose, fibrosierende Pankreatitis, Rheumatismus und Arthrosen, Morbus Crohn, chronische Bronchitis, Strahlenfibrose, Sklerodermitis, zystische Fibrose, Narbenbildung, Morbus Alzheimer).

Die erfindungsgemäßen Verbindungen können auch das Wachstum von Tumorzellen und Tumormetastasen hemmen und sind deshalb für die Tumortherapie geeignet.

Die erfindungsgemäßen Verbindungen finden weiterhin Verwendung zur Behandlung von Koagulopathien, wie z.B. Dysfibrinogenämie, Hypoprokonvertinämie, Hämophilie B, Stuart-Prower-Defekt, ProthrombinKomplex-Mangel, Verbrauchskoagulopathie, Hyperfibrinolyse, Immunokoagulopathie oder komplexer Koagulopathien, wie auch bei neuronaler Erregbarkeit, z.B. Epilepsie. Die erfindungsgemäßen Verbindungen können auch bei der Behandlung eines Glaukoms oder Katarakt therapeutisch eingesetzt werden.

Die erfindungsgemäßen Verbindungen finden ferner Verwendung bei der Behandlung bakterieller Infektionen sowie in einer antiinfektiösen Therapie. Die erfindungsgemäßen Verbindungen können auch zur Steigerung der Lernfähigkeit und Aufmerksamkeit therapeutisch eingesetzt werden. Darüberhinaus wirken die erfindungsgemäßen Verbindungen der Zellalterung entgegen und steigern somit die Lebenserwartung und die Fitness im Alter.

Die erfindungsgemäßen Verbindungen finden ferner Verwendung bei der Behandlung von Tinitus.

Die Identifikation von kleinen Verbindungen, die die Signaltransduktion der SGK spezifisch hemmen, regulieren und/oder modulieren, ist daher wünschenswert und ein Ziel der vorliegenden Erfindung.

Es wurde gefunden, daß die erfindungsgemäßen Verbindungen und ihre Salze bei guter Verträglichkeit sehr wertvolle pharmakologische Eigenschaften besitzen.

Insbesondere zeigen sie inhibierende Eigenschaften bei der SGK.

Gegenstand der vorliegenden Erfindung sind deshalb erfindungsgemäße Verbindungen als Arzneimittel und/oder Arzneimittelwirkstoffe bei der Behandlung und/oder Prophylaxe der genannten Erkrankungen und die Verwendung von erfindungsgemäßen Verbindungen zur Herstellung eines Pharmazeutikums für die Behandlung und/oder Prophylaxe der genannten Erkrankungen wie auch ein Verfahren zur Behandlung der genannten Erkrankungen umfassend die Verabreichung eines oder mehrerer erfindungsgemäßer Verbindungen an einen Patienten mit Bedarf an einer derartigen Verabreichung.

Der Wirt oder Patient kann jeglicher Säugerspezies angehören, z. B. einer Primatenspezies, besonders Menschen; Nagetieren, einschließlich Mäusen, Ratten und Hamstern; Kaninchen; Pferden, Rindern, Hunden, Katzen usw. Tiermodelle sind für experimentelle Untersuchungen von Interesse, wobei sie ein Modell zur Behandlung einer Krankheit des Menschen zur Verfügung stellen.

Zur Identifizierung eines Signalübertragungswegs und zum Nachweis von Wechselwirkungen zwischen verschiedenen Signalübertragungswegen wurden von verschiedenen Wissenschaftlern geeignete Modelle oder Modellsysteme entwickelt, z.B. Zellkulturmodelle (z.B. Khwaja et al., EMBO, 1997, 16, 2783-93) und Modelle transgener Tiere (z.B. White et al., Oncogene, 2001, 20, 7064-7072). Zur Bestimmung bestimmter Stufen in der Signalübertragungskaskade können wechselwirkende Verbindungen genutzt werden, um das Signal zu modulieren (z.B. Stephens et al., Biochemical J., 2000, 351, 95-105). Die erfindungsgemäßen Verbindungen können auch als Reagenzien zur Testung kinaseabhängiger Signalübertragungswege in Tieren und/oder Zellkulturmodellen oder in den in dieser Anmeldung genannten klinischen Erkrankungen verwendet werden.

Die Messung der Kinaseaktivität ist eine dem Fachmann wohlbekannte Technik. Generische Testsysteme zur Bestimmung der Kinaseaktivität mit Substraten, z.B. Histon (z.B. Alessi et al., FEBS Lett. 1996, 399, 3, Seiten 333-338) oder dem basischen Myelinprotein sind in der Literatur beschrieben (z.B. Campos-Gonzälez, R. und Glenney, Jr., J.R. 1992, J. Biol. Chem. 267, Seite 14535).

Zur Identifikation von Kinase-Inhibitoren stehen verschiedene Assay-Systeme zur Verfügung. Beim Scintillation-Proximity-Assay (Sorg et al., J. of. Biomolecular Screening, 2002, 7, 11-19) und dem FlashPlate-Assay wird die radioaktive Phosphorylierung eines Proteins oder Peptids als Substrat mit γATP gemessen. Bei Vorliegen einer inhibitorischen Verbindung ist kein oder ein vermindertes radioaktives Signal nachweisbar. Ferner sind die Homogeneous Time-resolved Fluorescence Resonance Energy Transfer- (HTR-FRET-) und Fluoreszenzpolarisations- (FP-) Technologien als Assay-Verfahren nützlich (Sills et al., J. of Biomolecular Screening, 2002, 191-214).

Andere nicht radioaktive ELISA-Assay-Verfahren verwenden spezifische Phospho-Antikörper (Phospho-AK). Der Phospho-AK bindet nur das phosphorylierte Substrat. Diese Bindung ist mit einem zweiten Peroxidasekonjugierten Anti-Schaf-Antikörper durch Chemolumineszenz nachweisbar (Ross et al., Biochem. J., 2002, 366, 977-981).

### STAND DER TECHNIK

Andere Indazolderivate sind als Proteinkinase-Inhibitoren in der WO 03/064397 beschrieben.

In der WO 00/62781 ist die Verwendung von Arzneimitteln enthaltend Hemmstoffe der zellvolumenregulierten humanen Kinase H-SGK beschrieben.

Die Verwendung von Kinase-Inhibitoren in der antiinfektiösen Therapie ist von C.Doerig in Cell. Mol. Biol. Lett. Vol.8, No. 2A, 2003, 524-525 beschrieben.

Die Verwendung von Kinase-Inhibitoren bei Fettsucht ist von N.Perrotti in J. Biol. Chem. 2001, März 23; 276(12):9406-9412 beschrieben.

In nachstehenden Literaturstellen wird die Verwendung von SGK-Hemmern bei der Behandlung von Krankheiten nahegelegt und/oder beschrieben:
1: Chung EJ, Sung YK, Farooq M, Kim Y, Im S, Tak WY, Hwang YJ, Kim Yl, Han HS, Kim JC, Kim MK. Gene expression profile analysis in human hepatocellular carcinoma by cDNA microarray. Mol Cells. 2002;14:382-7.
2: Brickley DR, Mikosz CA, Hagan CR, Conzen SD. Ubiquitin modification of serum and glucocorticoid-induced protein kinase-1(SGK-1). J Biol Chem. 2002;277:43064-70.
3: Fillon S, Klingel K, Warntges S, Sauter M, Gabrysch S, Pestel S, Tanneur V; Waldegger S, Zipfel A, Viebahn R, Haussinger D, Broer S, Kandolf R, Lang F. Expression of the serine/threonine kinase hSGK1 in chronic viral hepatitis. Cell Physiol Biochem. 2002;12:47-54.
4: Brunet A, Park J, Tran H, Hu LS, Hemmings BA, Greenberg ME. Protein kinase SGK mediates survival signals by phosphorylating the forkhead transcription factor FKHRL1 1 (FOX03a). Mol Cell Biol 2001;21:952-65
5: Mikosz CA, Brickley DR, Sharkey MS, Moran TW, Conzen SD. Glucocorticoid receptor-mediated protection from apoptosis is associated with induction of the serine/threonine survival kinase gene, sgk-1. J Biol Chem. 2001;276:16649-54.
6: Zuo Z, Urban G, Scammell JG, Dean NM, McLean TK, Aragon I, Honkanen RE. Ser/Thr protein phosphatase type 5 (PP5) is a negative regulator of glucocorticoid receptor-mediated growth arrest. Biochemistry. 1999;38:8849-57.
7: Buse P, Tran SH, Luther E, Phu PT, Aponte GW, Firestone GL. Cell cycle and hormonal control of nuclear-cytoplasmic localization of the serum- and glucocorticoid-inducible protein kinase, Sgk, in mammary tumor cells. A novel convergence point of anti-proliferative and proliferative cell signalling pathways. J Biol Chem. 1999;274:7253-63.

### ZUSAMMENFASSUNG DER ERFINDUNG

Die Erfindung betrifft Verbindungen der Formel I 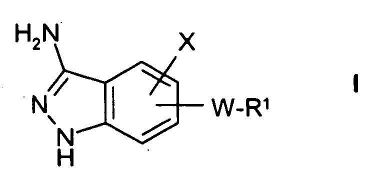 worin
- W: -[C(R³)₂]ₙ-, -[C(R³)₂]ₙCONH[C(R³)₂]ₙ-, -[C(R³)₂]ₙNR³CO[C(R³)²]nNR³-, -[C(R³)₂]ₙO[C(R³)₂]ₙ-, -[C(R³)]ₙNR³[C(R³)₂]ₙ-, -[C(R³)₂]ₙN[C(R³)₂R¹][C(R³)₂]ₙ-, -[C(R³)₂]ₙNR³C(R³)₂]ₙCONR³[C(R³)₂]ₙ-, -[C(R³)₂]ₙCO[C(R³)₂]ₙ-, -[C(R³)₂]ₙCONHNH[C(R³)₂]ₙ-, -(C(R³)₂]ₙNHCO[C(R³)₂]ₙ-, -C(R³)=C(R³)CONR³(C(R³)₂]ₙ oder -C(R³)=N-NR³CO[C(R³)]ₙ,
- X: H,
- R¹: Phenyl oder Naphthyl, das ein-, zwei- oder dreifach durch Hal, A, -(CH₂)ₙOH, OA, Phenyl, Phenoxy, Benzyloxy, Pyridin-4-ylmethyl-, Piperidin-4-oxy-, NO₂, CN, -(CH₂)ₙCOOR³, O-(CH₂)ₙCOOR³ SO₂A, NHCOA, (CH₂)ₙNH₂, (CH₂)ₙNHA, (CH₂)ₙNA₂, -[C(R³)₂]ₙCONH₂, (CH₂)ₙCONHNH₂ oder O-(CH₂)ₙCONHNH₂ substituiert sein kann,
oder
einen unsubstituierten oder ein- oder zweifach durch NH₂, A, OH, CH₂OH, COOH und/oder COOA substituierten ein- oder zweikernigen gesättigten oder ungesättigten Heterocyclus mit 1 bis 4 N-, O- und/oder S-Atomen,
- R³: H oder A,
- A: unverzweigtes oder verzweigtes Alkyl mit 1-6 C-Atomen, worin 1-7 H-Atome durch F ersetzt sein können,
- Hal: F, Cl, Br oder I,
- n: 0, 1 oder 2,
bedeuten,
sowie ihre pharmazeutisch verwendbaren Salze, E,Z-Isomere, Diastereomere, Enantiomere, Mono-, Dihydrate und Alkoholate, einschließlich deren Mischungen in allen Verhältnissen.

Gegenstand der Erfindung sind die Verbindungen der Formel **I** und ihre Salze sowie ein Verfahren zur Herstellung von Verbindungen der Formel I nach den Ansprüchen 1-2 sowie ihrer pharmazeutisch verwendbaren Salze, E,Z-Isomere, Diastereomere, Enantiomere, Mono-, Dihydrate und Alkoholate, einschließlich deren Mischungen in allen Verhältnissen, dadurch gekennzeichnet, daß man
a) zur Herstellung von Verbindungen der Formel I, worin
   W
   i) -[C(R³)₂]ₙCONH(C(R³)₂]ₙ- oder
   ii) -[C(R³)₂]ₙCONHNH[C(R³)₂]ₙ-
      bedeutet,
      eine Verbindung der Formel II 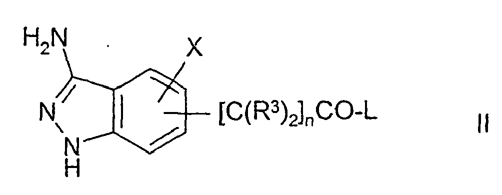 worin
      X, R³ und n die in Anspruch 1 angegebene Bedeutung haben,
      und L Cl, Br, I oder eine freie oder reaktionsfähig funktionell
      abgewandelte OH-Gruppe bedeutet,
   mit
   i) einer Verbindung der Formel IIIa

      H₂N[C(R³)₂]ₙ-R¹ IIIa

      worin
      R¹, R³ und n die in Anspruch 1 angegebenen Bedeutungen haben,
      oder
   ii) einer Verbindung der Formel IIIb

      H₂N-NH[C(R³)₂]ₙ-R¹ IIIb

      worin
      R¹, R³ und n die in Anspruch 1 angegebenen Bedeutungen haben,
   umsetzt,
   oder
b) zur Herstellung von Verbindungen der Formel I, worin
   W -[C(R³)₂]ₙNHCO[C(R³)₂]ₙ- bedeutet,
   eine Verbindung der Formel IV 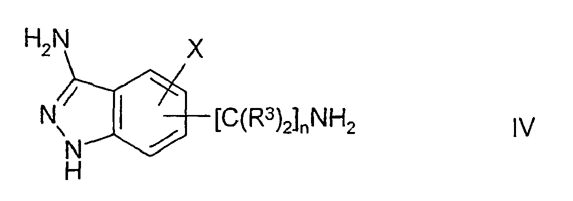 worin X, R³ und n die in Anspruch 1 angegebenen Bedeutungen
   haben,
   mit einer Verbindung der Formel V

   L-CO-[C(R³)₂]ₙ-R¹ V

   worin
   L Cl, Br, I oder eine freie oder reaktionsfähig funktionell abgewandelte OH-Gruppe bedeutet und
   R¹, R³ und n die in Anspruch 1 angegebenen Bedeutungen haben,
   umsetzt,
   oder
c) zur Herstellung von Verbindungen der Formel I, worin
   W -[C(R³)₂]ₙ-, -[C(R³)₂]ₙO[C(R³)₂]ₙ, -[C(R³)₂]ₙCO[C(R³)₂]ₙ- -[C(R³)₂]ₙNR³[C(R³)₂]ₙ- oder -[C(R³)₂]ₙNR¹[C(R³)₂]ₙ-
   bedeutet,
   eine Verbindung der Formel VI 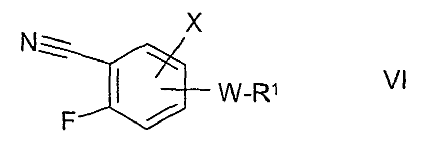 worin
   R¹, W und X die in Anspruch 1 angegebenen Bedeutungen haben,
   mit Hydrazin umsetzt,
   oder
d) zur Herstellung von Verbindungen der Formel I, worin
   W -[C(R³)₂]ₙNHCONH[C(R³)₂]ₙ- bedeutet,
   eine Verbindung der Formel IV mit einer Verbindung der Formel VII

   O=C=N-[C(R³)₂]ₙ-R¹ VII

   worin R¹, R³ und n die in Anspruch 1 angegebenen Bedeutungen haben,
   umsetzt,
   oder
e) daß man sie aus einem ihrer funktionellen Derivate durch Behandeln mit einem solvolysierenden oder hydrogenolysierenden Mittel in Freiheit setzt,
   und/oder
   eine Base oder Säure der Formel I in eines ihrer Salze umwandelt.

Gegenstand der Erfindung sind auch die Stereoisomeren (E, Z-Isomeren) sowie die Hydrate und Solvate dieser Verbindungen. Unter Solvate der Verbindungen werden Anlagerungen von inerten Lösungsmittelmolekülen an die Verbindungen verstanden, die sich aufgrund ihrer gegenseitigen Anziehungskraft ausbilden. Solvate sind z.B. Mono- oder Dihydrate oder Alkoholate.

Unter pharmazeutisch verwendbaren Derivaten versteht man z.B. die Salze der erfindungsgemäßen Verbindungen als auch sogenannte Prodrug-Verbindungen.

Unter Prodrug-Derivaten versteht man mit z. B. Alkyl- oder Acylgruppen, Zuckern oder Oligopeptiden abgewandelte Verbindungen der Formel I, die im Organismus rasch zu den wirksamen erfindungsgemäßen Verbindungen gespalten werden.

Hierzu gehören auch bioabbaubare Polymerderivate der erfindungsgemäßen Verbindungen, wie dies z. B. in Int. J. Pharm. 115, 61-67 (1995) beschrieben ist.

Der Ausdruck "wirksame Menge" bedeutet die Menge eines Arzneimittels oder eines pharmazeutischen Wirkstoffes, die eine biologische oder medizinische Antwort in einem Gewebe, System, Tier oder Menschen hervorruft, die z.B. von einem Forscher oder Mediziner gesucht oder erstrebt wird.

Darüberhinaus bedeutet der Ausdruck "therapeutisch wirksame Menge" eine Menge, die, verglichen zu einem entsprechenden Subjekt, das diese Menge nicht erhalten hat, folgendes zur Folge hat: verbesserte Heilbehandlung, Heilung, Prävention oder Beseitigung einer Krankheit, eines Krankheitsbildes, eines Krankheitszustandes, eines Leidens, einer Störung oder von Nebenwirkungen oder auch die Verminderung des Fortschreitens einer Krankheit, eines Leidens oder einer Störung.

Die Bezeichnung "therapeutisch wirksame Menge" umfaßt auch die Mengen, die wirkungsvoll sind, die normale physiologische Funktion zu erhöhen.

Gegenstand der Erfindung sind auch Mischungen der erfindungsgemäßen Verbindungen der Formel I, z.B. Gemische zweier Diastereomerer z.B. im Verhältnis 1:1, 1:2, 1:3, 1:4, 1:5, 1:10, 1:100 oder 1:1000.

Besonders bevorzugt handelt es sich dabei um Mischungen stereoisomerer Verbindungen.

Für alle Reste, die mehrfach auftreten, wie z.B. R², R³ oder n, gilt, daß deren Bedeutungen unabhängig voneinander sind.

Vor- und nachstehend haben die Reste bzw. Parameter W, X und R¹ die bei der Formel I angegebenen Bedeutungen, falls nicht ausdrücklich etwas anderes angegeben ist.

A bedeutet Alkyl, ist unverzweigt (linear) oder verzweigt, und hat 1, 2, 3, 4, 5, 6, 7, 8, 9 oder 10 C-Atome. A bedeutet vorzugsweise Methyl, weiterhin Ethyl, Propyl, Isopropyl, Butyl, Isobutyl, sek.-Butyl oder tert.-Butyl, ferner auch Pentyl, 1-, 2- oder 3-Methylbutyl, 1,1- , 1,2- oder 2,2-Dimethylpropyl, 1-Ethylpropyl, Hexyl, 1- , 2- , 3- oder 4-Methylpentyl, 1,1- , 1,2- , 1,3- , 2,2- , 2,3- oder 3,3-Dimethylbutyl, 1- oder 2-Ethylbutyl, 1-Ethyl-1-methylpropyl, 1-Ethyl-2-methylpropyl, 1,1,2- oder 1,2,2-Trimethylpropyl, weiter bevorzugt z.B. Trifluormethyl.

A bedeutet ganz besonders bevorzugt Alkyl mit 1, 2, 3, 4, 5 oder 6 C-Atomen, vorzugsweise Methyl, Ethyl, Propyl, Isopropyl, Butyl, Isobutyl, sek.-Butyl, tert.-Butyl, Pentyl, Hexyl, Trifluormethyl, Pentafluorethyl oder 1,1,1-Trifluorethyl.

Cycloalkyl bedeutet vorzugsweise Cyclopropyl, Cyclobutyl, Cylopentyl, Cyclohexyl oder Cycloheptyl.

W bedeutet besonders bevorzugt (CH₂)ₙ, CONH(CH₂)ₙ, NHCO(CH₂)ₙ, CONHNH, O, O(CH₂)ₙ, NHCO, CONH, CO, NH(CH₂)ₙ, N[(CH₂)ₙR¹](CH₂)ₙ, NHCO-(CH₂)ₙNH, NHCONH, CONHCHR³, -CH=CH-CONH(CH₂)ₙ oder -CH=N-NHCO(CH₂)ₙ.

W bedeutet ganz besonders bevorzugt eine Bindung, CONHCH₂, NHCOCH₂, CONHNH, O, OCH₂, NHCO, CONH, CONH(CH₂)₂, CO, NHCH₂, N(CH₂R¹)CH₂, NHCO-CH₂NH, NHCONH, NHCO(CH₂)₂, CONHCHR³, -CH=CH-CONH(CH₂)ₙ oder -CH=N-NHCO(CH₂)ₙ.

X bedeutet vorzugsweise H.

Gesättigter Carbocyclus bedeutet vorzugsweise Cyclopentyl oder Cyclohexyl. Ungesättigter Carbocyclus bedeutet vorzugsweise Phenyl, Naphthyl oder Biphenyl, besonders bevorzugt Phenyl. In einer weiteren Ausführungsform bedeutet R¹ besonders bevorzugt Phenyl oder Naphthyl, das ein-, zwei- oder dreifach durch Hal, A, -(CH₂)ₙOH, OA, Phenyl, Phenoxy, Benzyloxy, Pyridin-4-ylmethyl-, Piperidin-4-oxy-, NO₂, CN, -(CH₂)ₙCOOR³, O-(CH₂)ₙCOOR³, SO₂A, NHCOA, (CH₂)ₙNH₂, (CH₂)nNHA, (CH₂)ₙNA₂, -[C(R²)₂]ₙCONH₂, (CH₂)ₙCONHNH₂ oder O-(CH₂)ₙCONHNH₂ substituiert sein kann.

In einer anderen Ausführungsform bedeutet R¹ vorzugsweise einen unsubstituierten oder ein- oder zweifach durch NH₂, A, OH, CH₂OH, COOH und/oder COOA substituierten ein- oder zweikernigen gesättigten oder ungesättigten Heterocyclus mit 1 bis 4 N-, O- und/oder S-Atomen.

Unsubstituierter ein- oder zweikerniger gesättigter oder ungesättigter Heterocyclus mit 1 bis 4 N-, O- und/oder S-Atomen bedeutet vorzugsweise z.B. 2- oder 3-Furyl, 2- oder 3-Thienyl, 1-, 2- oder 3-Pyrrolyl, 1-, 2, 4- oder 5-Imidazolyl, 1-, 3-, 4- oder 5-Pyrazolyl, 2-, 4- oder 5-Oxazolyl, 3-, 4- oder 5-Isoxazolyl, 2-, 4- oder 5-Thiazolyl, 3-, 4- oder 5-Isothiazolyl, 2-, 3- oder 4-Pyridyl, 2-, 4-, 5- oder 6-Pyrimidinyl, weiterhin bevorzugt 1,2,3-Triazol-1-, -4- oder -5-yl, 1,2,4-Triazol-1-, -3- oder 5-yl, 1- oder 5-Tetrazolyl, 1,2,3-Oxadiazol-4- oder -5-yl, 1,2,4-Oxadiazol-3- oder -5-yl, 1,3,4-Thiadiazol-2-oder -5-yl, 1,2,4-Thiadiazol-3- oder -5-yl, 1,2,3-Thiadiazol-4- oder -5-yl, 3-oder 4-Pyridazinyl, Pyrazinyl, 1-, 2-, 3-, 4-, 5-, 6- oder 7-Indolyl, 4- oder 5-Isoindolyl, 1-, 2-, 4- oder 5-Benzimidazolyl, 1-, 2-, 3-, 4-, 5-, 6- oder 7-Indazolyl, 1-, 3-, 4-, 5-, 6- oder 7-Benzopyrazolyl, 2-, 4-, 5-, 6- oder 7-Benzoxazolyl, 3-, 4-, 5-, 6- oder 7- Benzisoxazolyl, 2-, 4-, 5-, 6- oder 7-Benzothiazolyl, 2-, 4-, 5-, 6- oder 7-Benzisothiazolyl, 4-, 5-, 6- oder 7-Benz-2,1,3-oxadiazolyl, 2-, 3-, 4-, 5-, 6-, 7- oder 8-Chinolyl, 1-, 3-, 4-, 5-, 6-, 7-oder 8-lsochinolyl, 3-, 4-, 5-, 6-, 7- oder 8-Cinnolinyl, 2-, 4-, 5-, 6-, 7- oder 8-Chinazolinyl, 5- oder 6-Chinoxalinyl, 2-, 3-, 5-, 6-, 7- oder 8-2H-Benzo[1,4]oxazinyl, weiter bevorzugt 1,3-Benzodioxol-5-yl, 1,4-Benzodioxan-6-yl, 2,1,3-Benzothiadiazol-4- oder -5-yl oder 2,1,3-Benzoxadiazol-5-yl, 2,3-Dihydro-2-, -3-, -4- oder -5-furyl, 2,5-Dihydro-2-, -3-, -4- oder 5-furyl, Tetrahydro-2- oder -3-furyl, 1,3-Dioxolan-4-yl, Tetrahydro-2- oder -3-thienyl, 2,3-Dihydro-1-, -2-, -3-, -4- oder -5-pyrrolyl, 2,5-Dihydro-1-, -2-, -3-, -4- oder -5-pyrrolyl, 1-, 2- oder 3-Pyrrolidinyl, Tetrahydro-1-, -2- oder -4-imidazolyl, 2,3-Dihydro-1-, -2-, -3-, -4- oder -5-pyrazolyl, Tetrahydro-1-, -3- oder -4-pyrazolyl, 1,4-Dihydro-1-, -2-, -3- oder -4-pyridyl, 1,2,3,4-Tetrahydro-1-, -2-, -3-, -4-, -5- oder -6-pyridyl, 1-, 2-, 3- oder 4-Piperidinyl, 2-, 3- oder 4-Morpholinyl, Tetrahydro-2-, -3- oder -4-pyranyl, 1,4-Dioxanyl, 1,3-Dioxan-2-, -4- oder -5-yl, Hexahydro-1-, -3- oder -4-pyridazinyl, Hexahydro-1-, -2-, -4-oder -5-pyrimidinyl, 1-, 2- oder 3-Piperazinyl, 1,2,3,4-Tetrahydro-1-, -2-, -3-, -4-, -5-, -6-, -7- oder -8-chinolyl, 1,2,3,4-Tetrahydro-1-,-2-,-3-, -4-, -5-, -6-, -7- oder -8-isochinolyl, 2-, 3-, 5-, 6-, 7- oder 8- 3,4-Dihydro-2H-benzo[1,4]oxazinyl, weiter bevorzugt 2,3-Methylendioxyphenyl, 3,4-Methylendioxyphenyl, 2,3-Ethylendioxyphenyl, 3,4-Ethylendioxyphenyl, 3,4-(Difluormethylendioxy)phenyl, 2,3-Dihydrobenzofuran-5- oder 6-yl, 2,3-(2-Oxo-methylendioxy)-phenyl oder auch 3,4-Dihydro-2H-1,5-benzodioxepin-6- oder -7-yl, ferner bevorzugt 2,3-Dihydrobenzofuranyl oder 2,3-Dihydro-2-oxo-furanyl.

R¹ bedeutet besonders bevorzugt auch unsubstituiertes oder ein- oder zweifach durch NH₂, A, OH, CH₂OH, COOH und/oder COOA substituiertes 2- oder 3-Furyl, 2- oder 3-Thienyl, 1-, 2- oder 3-Pyrrolyl, 1-, 2, 4- oder 5-Imidazolyl, 1-, 3-, 4- oder 5-Pyrazolyl, 2-, 4- oder 5-Oxazolyl, 3-, 4- oder 5-Isoxazolyl, 2-, 4- oder 5-Thiazolyl, 3-, 4- oder 5-Isothiazolyl, 2-, 3- oder 4-Pyridyl, 2-, 4-, 5- oder 6-Pyrimidinyl, 3- oder 4-Pyridazinyl, Pyrazinyl, 1-, 2-, 3-, 4-, 5-, 6- oder 7-Indolyl, 4- oder 5-Isoindolyl, 1-, 2-, 4- oder 5-Benzimidazolyl, 1-, 2-, 3-, 4-, 5-, 6- oder 7-Indazolyl, 2-, 4-, 5-, 6- oder 7-Benzothiazolyl, 2-, 4-, 5-, 6- oder 7-Benzisothiazolyl, 4-, 5-, 6- oder 7-Benz-2,1,3-oxadiazolyl, 2-, 3-, 4-, 5-, 6-, 7- oder 8-Chinolyl, 1-, 3-, 4-, 5-, 6-, 7- oder 8-Isochinolyl, 3-, 4-, 5-, 6-, 7- oder 8-Cinnolinyl, 2-, 4-, 5-, 6-, 7- oder 8-Chinazolinyl, 5- oder 6-Chinoxalinyl, 2-, 3-, 5-, 6-, 7- oder 8-2H-Benzo[1,4]oxazinyl, 1,3-Benzodioxol-5-yl, 1,4-Benzodioxan-6-yl, 2,1,3-Benzothiadiazol-4- oder -5-yl oder 2,1,3-Benzoxadiazol-5-yl, 2-, 3- oder 4-Piperidinyl, 2-, 3- oder 4-Morpholinyl, 1-, 2- oder 3-Piperazinyl, 2,3-Methylendioxyphenyl, 3,4-Methylendioxyphenyl, 2,3-Ethylendioxyphenyl, 3,4-Ethylendioxyphenyl, 3,4-(Difluormethylendioxy)phenyl, 2,3-Dihydrobenzofuran-5- oder 6-yl, 2,3-(2-Oxo-methylendioxy)-phenyl, 3,4-Dihydro-2H-1,5-benzodioxepin-6- oder -7-yl, 2,3-Dihydrobenzofuranyl oder 2,3-Dihydro-2-oxo-furanyl.

R¹ bedeutet weiterhin vorzugsweise Phenyl oder Naphthyl, das ein-, zwei- oder dreifach durch Hal, A, -(CH₂)ₙOH, OA, Phenyl, Phenoxy, Benzyloxy, Pyridin-4-ylmethyl-, Piperidin-4-oxy-, NO₂, CN, -(CH₂)ₙCOOR³, O-(CH₂)ₙCOOR³, SO₂A, NHCOA, (CH₂)ₙNH₂, (CH₂)ₙNHA, (CH₂)nNA₂, -[C(R²)₂]ₙCONH₂, (CH₂)ₙCONHNH₂ oder O-(CH₂)ₙCONHNH₂ substituiert sein kann,
oder einen unsubstituierten oder ein- oder zweifach durch NH₂, A, OH, CH₂OH COOH und/oder COOA substituierten ein- oder zweikernigen gesättigten oder ungesättigten Heterocyclus mit 1 bis 4 N-, O- und/oder S-Atomen.

Die Verbindungen der Formel I können ein oder mehrere chirale Zentren besitzen und daher in verschiedenen stereoisomeren Formen vorkommen. Die Formel I umschließt alle diese Formen.

Dementsprechend sind Gegenstand der Erfindung insbesondere diejenigen Verbindungen der Formel I, in denen mindestens einer der genannten Reste eine der vorstehend angegebenen bevorzugten Bedeutungen hat. Einige bevorzugte Gruppen von Verbindungen können durch die folgenden Teilformeln Ia ausgedrückt werden, die der Formel I entsprechen und worin die nicht näher bezeichneten Reste die bei der Formel I angegebene Bedeutung haben, worin jedoch
- in Ia: W -[C(R³)₂]ₙ-, -[C(R³)₂]ₙCONH[C(R³)₂]ₙ-, -[C(R³)₂]ₙNR-³CO[C(R³)₂]ₙNR³-, -[C(R³)₂]ₙO[C(R³)₂]ₙ-, -[C(R³)₂]ₙNR³[C(R³)₂]ₙ-, -[C(R³)₂]ₙN[C(R³)₂R¹][C(R³)]ₙ-, -[C(R³)₂]ₙNR³C(R³)₂]ₙCONR³[C(R³)₂]ₙ-, -[C(R³)₂]ₙCO[C(R³)₂]ₙ-, -[C(R³)₂]ₙCONHNH[C(R³)₂]_{n,} -[C(R³)_{2]n}NHCO[C(R³)₂]ₙ-, -C(R³)=C(R³)CONR³C(R²)₂]ₙ oder -C(R³)=N-NR³CO[C(R³)₂]ₙ,
X H,
R¹ Phenyl oder Naphthyl, das ein-, zwei- oder dreifach durch Hal, A, -(CH₂)ₙOH, OA, Phenyl, Phenoxy, Benzyloxy, Pyridin-4-ylmethyl-, Piperidin-4-oxy-, NO₂, CN, -(CH₂)ₙCOOR³, O-(CH₂)ₙCOOR³, SO₂A, NHCOA, (CH₂)ₙNH₂, (CH₂)ₙNHA, (CH₂)ₙNA₂, -[C(R²)₂]ₙCONH₂, (CH₂)ₙCONHNH₂ oder O-(CH₂)ₙCONHNH₂ substituiert sein kann,
oder
einen unsubstituierten oder ein- oder zweifach durch NH₂, A, OH, CH₂OH, COOH und/oder COOA substituierten ein- oder zweikernigen gesättigten oder ungesättigten Heterocyclus mit 1 bis 4 N-, O- und/oder S-Atomen,
R³ H oder A,
A unverzweigtes oder verzweigtes Alkyl mit 1-6 C-Atomen, worin 1-7 H-Atome durch F ersetzt sein können,
Hal F, Cl, Br oder I,
n 0, 1 oder 2,
sowie ihre pharmazeutisch verwendbaren Salze, E,Z-Isomere, Diastereomere, Enantiomere, Mono-, Dihydrate und Alkoholate, einschließlich deren Mischungen in allen Verhältnissen.

Die erfindungsgemäßen Verbindungen und auch die Ausgangsstoffe zu ihrer Herstellung werden im übrigen nach an sich bekannten Methoden hergestellt, wie sie in der Literatur (z.B. in den Standardwerken wie Houben-Weyl, Methoden der organischen Chemie, Georg-Thieme-Verlag, Stuttgart) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können, falls erwünscht, auch in situ gebildet werden, so daß man sie aus dem Reaktionsgemisch nicht isoliert, sondern sofort weiter zu den erfindungsgemäßen Verbindungen umsetzt.

Die Ausgangsverbindungen sind in der Regel bekannt. Sind sie neu, so können sie aber nach an sich bekannten Methoden hergestellt werden.

Verbindungen der Formel I, worin
W i) -(C(R³)₂]ₙCONH(C(R³)₂]ₙ- oder
ii) -[C(R³)₂]ₙCONHNH[C(R³)₂]ₙ-
   bedeutet, können vorzugsweise erhalten werden, indem man Verbindungen der Formel II mit Verbindungen der Formel IIIa bzw. IIIb umsetzt.

Die Umsetzung erfolgt in der Regel in einem inerten Lösungsmittel, in Gegenwart eines säurebindenden Mittels vorzugsweise einer organischen Base wie DIPEA, Triethylamin, Dimethylanilin, Pyridin oder Chinolin oder eines Überschusses der Carboxykomponente der Formel II.

Als inerte Lösungsmittel eignen sich z.B. Kohlenwasserstoffe wie Hexan, Petrolether, Benzol, Toluol oder Xylol; chlorierte Kohlenwasserstoffe wie Trichlorethylen, 1,2-Dichlorethan,Tetrachlorkohlenstoff, Chloroform oder Dichlormethan; Alkohole wie Methanol, Ethanol, Isopropanol, n-Propanol, n-Butanol oder tert.-Butanol; Ether wie Diethylether, Diisopropylether, Tetrahydrofuran (THF) oder Dioxan; Glykolether wie Ethylenglykolmonomethyl- oder -monoethylether (Methylglykol oder Ethylglykol), Ethylenglykoldimethylether (Diglyme); Ketone wie Aceton oder Butanon; Amide wie Acetamid, Dimethylacetamid oder Dimethylformamid (DMF); Nitrile wie Acetonitril; Sulfoxide wie Dimethylsulfoxid (DMSO); Schwefelkohlenstoff; Carbonsäuren wie Ameisensäure oder Essigsäure; Nitroverbindungen wie Nitromethan oder Nitrobenzol; Ester wie Ethylacetat oder Gemische der genannten Lösungsmittel.

Auch der Zusatz eines Alkali- oder Erdalkalimetall-hydroxids, -carbonats oder -bicarbonats oder eines anderen Salzes einer schwachen Säure der Alkali- oder Erdalkalimetalle, vorzugsweise des Kaliums, Natriums, Calciums oder Cäsiums kann günstig sein.

Die Reaktionszeit liegt je nach den angewendeten Bedingungen zwischen einigen Minuten und 14 Tagen, die Reaktionstemperatur zwischen etwa -30° und 140°, normalerweise zwischen -10° und 90°, insbesondere zwischen etwa 0° und etwa 70°.

In den Verbindungen der Formel II bedeutet L vorzugsweise Cl, Br, I oder eine freie oder eine reaktionsfähig abgewandelte OH-Gruppe wie z.B. ein aktivierter Ester, ein Imidazolid oder Alkylsulfonyloxy mit 1-6 C-Atomen (bevorzugt Methylsulfonyloxy oder Trifluormethylsulfonyloxy) oder Arylsulfonyloxy mit 6-10 C-Atomen (bevorzugt Phenyl- oder p-Tolylsulfonyloxy). Derartige Reste zur Aktivierung der Carboxygruppe in typischen Acylierungsreaktionen sind in der Literatur (z.B. in den Standardwerken wie Houben-Weyl, Methoden der organischen Chemie, Georg-Thieme-Verlag, Stuttgart;) beschrieben.

Aktivierte Ester werden zweckmäßig in situ gebildet, z. B. durch Zusatz von HOBt oder N-Hydroxysuccinimid.

Verbindungen der Formel I, worin W -[C(R³)₂]ₙNHCO[C(R³)₂]ₙ- bedeutet, können weiter vorzugsweise erhalten werden, indem man Verbindungen der Formel IV mit Verbindungen der Formel V umsetzt.

Die Umsetzung erfolgt in der Regel in einem inerten Lösungsmittel und unter Bedingungen wie oben angegeben.

In den Verbindungen der Formel V bedeutet L vorzugsweise Cl, Br, I oder eine freie oder eine reaktionsfähig abgewandelte OH-Gruppe wie z.B. ein aktivierter Ester, ein Imidazolid oder Alkylsulfonyloxy mit 1-6 C-Atomen (bevorzugt Methylsulfonyloxy oder Trifluormethylsulfonyloxy) oder Arylsulfonyloxy mit 6-10 C-Atomen (bevorzugt Phenyl- oder p-Tolylsulfonyloxy).

Verbindungen der Formel I, worin
W -[C(R³)₂]ₙ-, -[C(R³)₂]ₙO[C(R³)₂]ₙ-, -[C(R³)₃]ₙNR³[C(R³)₂]ₙ- oder -[C(R³)₂]ₙNR¹[C(R³)₂]ₙ- bedeutet,
können weiter vorzugsweise erhalten werden, indem man Verbindungen der Formel VI mit Hydrazin umsetzt.

Die Umsetzung erfolgt in der Regel in einem inerten Lösungsmittel und unter Bedingungen wie oben angegeben.

Verbindungen der Formel I, worin
W -[C(R³)₂]ₙNHCONH[C(R³)₂]ₙ- bedeutet,
können weiter vorzugsweise erhalten werden, indem man Verbindungen der Formel IV mit Verbindungen der Formel VII umsetzt.

Die Umsetzung erfolgt in der Regel in einem inerten Lösungsmittel und unter Bedingungen wie oben angegeben.

Verbindungen der Formel I können ferner erhalten werden, indem man Verbindungen der Formel I aus einem ihrer funktionellen Derivate durch Behandeln mit einem solvolysierenden oder hydrogenolysierenden Mittel in Freiheit setzt.

Bevorzugte Ausgangsstoffe für die Solvolyse bzw. Hydrogenolyse sind solche, die sonst der Formel I entsprechen, aber anstelle einer oder mehrerer freier Amino- und/oder Hydroxygruppen entsprechende geschützte Amino- und/oder Hydroxygruppen enthalten, vorzugsweise solche, die anstelle eines H-Atoms, das mit einem N-Atom verbunden ist, eine Aminoschutzgruppe tragen, insbesondere solche, die anstelle einer HN-Gruppe eine R'-N-Gruppe tragen, worin R' eine Aminoschutzgruppe bedeutet, und/oder solche, die anstelle des H-Atoms-einer Hydroxygruppe eine Hydroxyschutzgruppe tragen, z.B. solche, die der Formel I entsprechen, jedoch anstelle einer Gruppe -COOH eine Gruppe -COOR" tragen, worin R" eine Hydroxyschutzgruppe bedeutet.

Es können auch mehrere - gleiche oder verschiedene - geschützte Amino- und/oder Hydroxygruppen im Molekül des Ausgangsstoffes vorhanden sein. Falls die vorhandenen Schutzgruppen voneinander verschieden sind, können sie in vielen Fällen selektiv abgespalten werden.

Der Ausdruck "Aminoschutzgruppe" ist allgemein bekannt und bezieht sich auf Gruppen, die geeignet sind, eine Aminogruppe vor chemischen Umsetzungen zu schützen (zu blockieren), die aber leicht entfernbar sind, nachdem die gewünschte chemische Reaktion an anderen Stellen des Moleküls durchgeführt worden ist. Typisch für solche Gruppen sind insbesondere unsubstituierte oder substituierte Acyl-, Aryl-, Aralkoxymethyl- oder Aralkylgruppen. Da die Aminoschutzgruppen nach der gewünschten Reaktion (oder Reaktionsfolge) entfernt werden, ist ihre Art und Größe im übrigen nicht kritisch; bevorzugt werden jedoch solche mit 1-20, insbesondere 1-8 C-Atomen. Der Ausdruck "Acylgruppe" ist im Zusammenhang mit dem vorliegenden Verfahren in weitestem Sinne aufzufassen. Er umschließt von aliphatischen, araliphatischen, aromatischen oder heterocyclischen Carbonsäuren oder Sulfonsäuren abgeleitete Acylgruppen sowie insbesondere Alkoxycarbonyl-, Aryloxycarbonyl- und vor allem Aralkoxycarbonylgruppen. Beispiele für derartige Acylgruppen sind Alkanoyl wie Acetyl, Propionyl, Butyryl; Aralkanoyl wie Phenylacetyl; Aroyl wie Benzoyl oder Toluyl; Aryloxyalkanoyl wie POA; Alkoxycarbonyl wie Methoxycarbonyl, Ethoxycarbonyl, 2,2,2-Trichlorethoxycarbonyl, BOC (tert.-Butyloxycarbonyl), 2-lodethoxycarbonyl; Aralkyloxycarbonyl wie CBZ ("Carbobenzoxy"), 4-Methoxybenzyloxycarbonyl, FMOC; Arylsulfonyl wie Mtr. Bevorzugte Aminoschutzgruppen sind BOC und Mtr, ferner CBZ, Fmoc, Benzyl und Acetyl.

Der Ausdruck "Hydroxyschutzgruppe" ist ebenfalls allgemein bekannt und bezieht sich auf Gruppen, die geeignet sind, eine Hydroxygruppe vor chemischen Umsetzungen zu schützen, die aber leicht entfernbar sind, nachdem die gewünschte chemische Reaktion an anderen Stellen des Moleküls durchgeführt worden ist. Typisch für solche Gruppen sind die oben genannten unsubstituierten oder substituierten Aryl-, Aralkyl- oder Acylgruppen, ferner auch Alkylgruppen. Die Natur und Größe der Hydroxyschutzgruppen ist nicht kritisch, da sie nach der gewünschten chemischen Reaktion oder Reaktionsfolge wieder entfernt werden; bevorzugt sind Gruppen mit 1-20, insbesondere 1-10 C-Atomen. Beispiele für Hydroxyschutzgruppen sind u.a. Benzyl, 4-Methoxybenzyl, p-Nitrobenzoyl, p-Toluolsulfonyl, tert.-Butyl und Acetyl, wobei Benzyl und tert.-Butyl besonders bevorzugt sind.

Das In-Freiheit-Setzen der Verbindungen der Formel I aus ihren funktionellen Derivaten gelingt - je nach der benutzten Schutzgruppe - z. B. mit starken Säuren, zweckmäßig mit TFA oder Perchlorsäure, aber auch mit anderen starken anorganischen Säuren wie Salzsäure oder Schwefelsäure, starken organischen Carbonsäuren wie Trichloressigsäure oder Sulfonsäuren wie Benzol- oder p-Toluolsulfonsäure. Die Anwesenheit eines zusätzlichen inerten Lösungsmittels ist möglich, aber nicht immer erforderlich. Als inerte Lösungsmittel eignen sich vorzugsweise organische, beispielsweise Carbonsäuren wie Essigsäure, Ether wie Tetrahydrofuran oder Dioxan, Amide wie DMF, halogenierte Kohlenwasserstoffe wie Dichlormethan, ferner auch Alkohole wie Methanol, Ethanol oder Isopropanol, sowie Wasser. Ferner kommen Gemische der vorgenannten Lösungsmittel in Frage. TFA wird vorzugsweise im Überschuß ohne Zusatz eines weiteren Lösungsmittels verwendet, Perchlorsäure in Form eines Gemisches aus Essigsäure und 70 %iger Perchlorsäure im Verhältnis 9:1. Die Reaktionstemperaturen für die Spaltung liegen zweckmäßig zwischen etwa 0 und etwa 50°, vorzugsweise arbeitet man zwischen 15 und 30° (Raumtemperatur).

Die Gruppen BOC, OBut und Mtr können z. B. bevorzugt mit TFA in Dichlormethan oder mit etwa 3 bis 5n HCl in Dioxan bei 15-30° abgespalten werden, die FMOC-Gruppe mit einer etwa 5- bis 50 %igen Lösung von Dimethylamin, Diethylamin oder Piperidin in DMF bei 15-30°.

Hydrogenolytisch entfernbare Schutzgruppen (z. B. CBZ, Benzyl oder die Freisetzung der Amidinogruppe aus ihrem Oxadiazolderivat)) können z. B. durch Behandeln mit Wasserstoff in Gegenwart eines Katalysators (z. B. eines Edelmetallkatalysators wie Palladium, zweckmäßig auf einem Träger wie Kohle) abgespalten werden. Als Lösungsmittel eignen sich dabei die oben angegebenen, insbesondere z. B. Alkohole wie Methanol oder Ethanol oder Amide wie DMF. Die Hydrogenolyse wird in der Regel bei Temperaturen zwischen etwa 0 und 100° und Drucken zwischen etwa 1 und 200 bar, bevorzugt bei 20-30° und 1-10 bar durchgeführt. Eine Hydrogenolyse der CBZ-Gruppe gelingt z. B. gut an 5 bis 10 %igem Pd/C in Methanol oder mit Ammomiumformiat (anstelle von Wasserstoff) an Pd/C in Methanol/DMF bei 20-30°.

Als inerte Lösungsmittel eignen sich z.B. Kohlenwasserstoffe wie Hexan, Petrolether, Benzol, Toluol oder Xylol; chlorierte Kohlenwasserstoffe wie Trichlorethylen, 1,2-Dichlorethan,Tetrachlorkohlenstoff, Trifluormethylbenzol, Chloroform oder Dichlormethan; Alkohole wie Methanol, Ethanol, Isopropanol, n-Propanol, n-Butanol oder tert.-Butanol; Ether wie Diethylether, Diisopropylether, Tetrahydrofuran (THF) oder Dioxan; Glykolether wie Ethylenglykolmonomethyl- oder -monoethylether (Methylglykol oder Ethylglykol), Ethylenglykoldimethylether (Diglyme); Ketone wie Aceton oder Butanon; Amide wie Acetamid, Dimethylacetamid, N-Methylpyrrolidon (NMP) oder Dimethylformamid (DMF); Nitrile wie Acetonitril; Sulfoxide wie Dimethylsulfoxid (DMSO); Schwefelkohlenstoff; Carbonsäuren wie Ameisensäure oder Essigsäure; Nitroverbindungen wie Nitromethan oder Nitrobenzol; Ester wie Ethylacetat oder Gemische der genannten Lösungsmittel.

Ester können z.B. mit Essigsäure oder mit NaOH oder KOH in Wasser, Wasser-THF oder Wasser-Dioxan bei Temperaturen zwischen 0 und 100° verseift werden.

Ferner kann man freie Aminogruppen in üblicher Weise mit einem Säurechlorid oder -anhydrid acylieren oder mit einem unsubstituierten oder substituierten Alkylhalogenid alkylieren, oder mit CH₃-C(=NH)-OEt umsetzen, zweckmäßig in einem inerten Lösungsmittel wie Dichlormethan oder THF und /oder in Gegenwart einer Base wie Triethylamin oder Pyridin bei Temperaturen zwischen -60 und +30°.

Gegenstand der Erfindung sind auch die Zwischenverbindungen ausgewählt aus der Gruppe 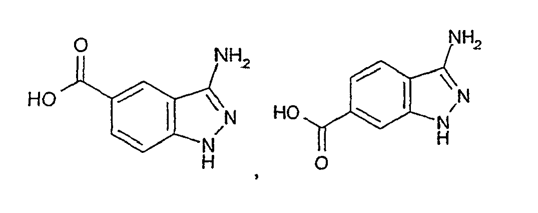 sowie deren Salze.

### Pharmazeutische Salze und andere Formen

Die genannten erfindungsgemäßen Verbindungen lassen sich in ihrer endgültigen Nichtsalzform verwenden. Andererseits umfaßt die vorliegende Erfindung auch die Verwendung dieser Verbindungen in Form ihrer pharmazeutisch unbedenklichen Salze, die von verschiedenen organischen und anorganischen Säuren und Basen nach fachbekannten Vorgehensweisen abgeleitet werden können. Pharmazeutisch unbedenkliche Salzformen der Verbindungen der Formel I werden größtenteils konventionell hergestellt. Sofern die Verbindung der Formel I eine Carbonsäuregruppe enthält, läßt sich eines ihrer geeigneten Salze dadurch bilden, daß man die Verbindung mit einer geeigneten Base zum entsprechenden Basenadditionssalz umsetzt. Solche Basen sind zum Beispiel Alkalimetallhydroxide, darunter Kaliumhydroxid, Natriumhydroxid und Lithiumhydroxid; Erdalkalimetallhydroxide wie Bariumhydroxid und Calciumhydroxid; Alkalimetallalkoholate, z.B. Kaliumethanolat und Natriumpropanolat; sowie verschiedene organische Basen wie Piperidin, Diethanolamin und N-Methylglutamin. Die Aluminiumsalze der Verbindungen der Formel I zählen ebenfalls dazu. Bei bestimmten Verbindungen der Formel I lassen sich Säureadditionssalze dadurch bilden, daß man diese Verbindungen mit pharmazeutisch unbedenklichen organischen und anorganischen Säuren, z.B. Halogenwasserstoffen wie Chlorwasserstoff, Bromwasserstoff oder Jodwasserstoff, anderen Mineralsäuren und ihren entsprechenden Salzen wie Sulfat, Nitrat oder Phosphat und dergleichen sowie Alkyl- und Monoarylsulfonaten wie Ethansulfonat, Toluolsulfonat und Benzolsulfonat, sowie anderen organischen Säuren und ihren entsprechenden Salzen wie Acetat, Trifluoracetat, Tartrat, Maleat, Succinat, Citrat, Benzoat, Salicylat, Ascorbat und dergleichen behandelt. Dementsprechend zählen zu pharmazeutisch unbedenklichen Säureadditionssalzen der Verbindungen der Formel I die folgenden: Acetat, Adipat, Alginat, Arginat, Aspartat, Benzoat, Benzolsulfonat (Besylat), Bisulfat, Bisulfit, Bromid, Butyrat, Kampferat, Kampfersulfonat, Caprylat, Chlorid, Chlorbenzoat, Citrat, Cyclopentanpropionat, Digluconat, Dihydrogenphosphat, Dinitrobenzoat, Dodecylsulfat, Ethansulfonat, Fumarat, Galacterat (aus Schleimsäure), Galacturonat, Glucoheptanoat, Gluconat, Glutamat, Glycerophosphat, Hemisuccinat, Hemisulfat, Heptanoat, Hexanoat, Hippurat, Hydrochlorid, Hydrobromid, Hydroiodid, 2-Hydroxyethansulfonat, Iodid, Isethionat, Isobutyrat, Lactat, Lactobionat, Malat, Maleat, Malonat, Mandelat, Metaphosphat, Methansulfonat, Methylbenzoat, Monohydrogenphosphat, 2-Naphthalinsulfonat, Nicotinat, Nitrat, Oxalat, Oleat, Pamoat, Pectinat, Persulfat, Phenylacetat, 3-Phenylpropionat, Phosphat, Phosphonat, Phthalat, was jedoch keine Einschränkung darstellt.

Weiterhin zählen zu den Basensalzen der erfindungsgemäßen Verbindungen Aluminium-, Ammonium-, Calcium-, Kupfer-, Eisen(III)-, Eisen(II)-, Lithium-, Magnesium-, Mangan(III)-, Mangan(II), Kalium-, Natrium- und Zinksalze, was jedoch keine Einschränkung darstellen soll. Bevorzugt unter den oben genannten Salzen sind Ammonium; die Alkalimetallsalze Natrium und Kalium,sowie die Erdalkalimetalsalze Calcium und Magnesium. Zu Salzen der Verbindungen der Formel I, die sich von pharmazeutisch unbedenklichen organischen nicht-toxischen Basen ableiten, zählen Salze primärer, sekundärer und tertiärer Amine, substituierter Amine, darunter auch natürlich vorkommender substituierter Amine, cyclischer Amine sowie basischer Ionenaustauscherharze, z.B. Arginin, Betain, Koffein, Chlorprocain, Cholin, N,N'-Dibenzylethylendiamin (Benzathin), Dicyclohexylamin, Diethanolamin, Diethylamin, 2-Diethylaminoethanol, 2-Dimethylaminoethanol, Ethanolamin, Ethylendiamin, N-Ethylmorpholin, N-Ethylpiperidin, Glucamin, Glucosamin, Histidin, Hydrabamin, Iso-propylamin, Lidocain, Lysin, Meglumin, N-Methyl-D-glucamin, Morpholin, Piperazin, Piperidin, Polyaminharze, Procain, Purine, Theobromin, Triethanolamin, Triethylamin, Trimethylamin, Tripropylamin sowie Tris-(hydroxymethyl)-methylamin (Tromethamin), was jedoch keine Einschränkung darstellen soll.

Verbindungen der vorliegenden Erfindung, die basische stickstoffhaltige Gruppen enthalten, lassen sich mit Mitteln wie (C₁-C₄) Alkylhalogeniden, z.B. Methyl-, Ethyl-, Isopropyl- und tert:-Butylchlorid, -bromid und -iodid; Di(C₁-C₄)Alkylsulfaten, z.B. Dimethyl-, Diethyl- und Diamylsulfat; (C₁₀-C₁₈)Alkylhalogeniden, z.B. Decyl-, Dodecyl-, Lauryl-, Myristyl- und Stearylchlorid, -bromid und -iodid; sowie Aryl-(C₁-C₄)Alkylhalogeniden, z.B. Benzylchlorid und Phenethylbromid, quarternisieren. Mit solchen Salzen können sowohl wasser- als auch öllösliche erfindungsgemäße Verbindungen hergestellt werden.

Zu den oben genannten pharmazeutischen Salzen, die bevorzugt sind, zählen Acetat, Trifluoracetat, Besylat, Citrat, Fumarat, Gluconat, Hemisuccinat, Hippurat, Hydrochlorid, Hydrobromid, Isethionat, Mandelat, Meglumin, Nitrat, Oleat, Phosphonat, Pivalat, Natriumphosphat, Stearat, Sulfat, Sulfosalicylat, Tartrat, Thiomalat, Tosylat und Tromethamin, was jedoch keine Einschränkung darstellen soll.

Die Säureadditionssalze basischer Verbindungen der Formel I werden dadurch hergestellt, daß man die freie Basenform mit einer ausreichenden Menge der gewünschten Säure in Kontakt bringt, wodurch man auf übliche Weise das Salz darstellt. Die freie Base läßt sich durch In-Kontakt-Bringen der Salzform mit einer Base und Isolieren der freien Base auf übliche Weise regenerieren. Die freien Basenformen unterscheiden sich in gewissem Sinn von ihren entsprechenden Salzformen in bezug auf bestimmte physikalische Eigenschaften wie Löslichkeit in polaren Lösungsmitteln; im Rahmen der Erfindung entsprechen die Salze jedoch sonst ihren jeweiligen freien Basenformen.

Wie erwähnt werden die pharmazeutisch unbedenklichen Basenadditionssalze der Verbindungen der Formel I mit Metallen oder Aminen wie Alkalimetallen und Erdalkatimetallen oder organischen Aminen gebildet. Bevorzugte Metalle sind Natrium, Kalium, Magnesium und Calcium. Bevorzugte organische Amine sind N,N'-Dibenzylethylendiamin, Chlorprocain, Cholin, Diethanolamin, Ethylendiamin, N-Methyl-D-glucamin und Procain.

Die Basenadditionssalze von erfindungsgemäßen sauren Verbindungen werden dadurch hergestellt, daß man die freie Säureform mit einer ausreichenden Menge der gewünschten Base in Kontakt bringt, wodurch man das Salz auf übliche Weise darstellt. Die freie Säure läßt sich durch In-Kontakt-Bringen der Salzform mit einer Säure und Isolieren der freien Säure auf übliche Weise regenerieren. Die freien Säureformen unterscheiden sich in gewissem Sinn von ihren entsprechenden Salzformen in bezug auf bestimmte physikalische Eigenschaften wie Löslichkeit in polaren Lösungsmitteln; im Rahmen der Erfindung entsprechen die Salze jedoch sonst ihren jeweiligen freien Säureformen.

Enthält eine erfindungsgemäße Verbindung mehr als eine Gruppe, die solche pharmazeutisch unbedenklichen Salze bilden kann, so umfaßt die Erfindung auch mehrfache Salze. Zu typischen mehrfachen Salzformen zählen zum Beispiel Bitartrat, Diacetat, Difumarat, Dimeglumin, Diphosphat, Dinatrium und Trihydrochlorid, was jedoch keine Einschränkung darstellen soll.

Im Hinblick auf das oben Gesagte sieht man, daß unter dem Ausdruck "pharmazeutisch unbedenkliches Salz" im vorliegenden Zusammenhang ein Wirkstoff zu verstehen ist, der eine Verbindung der Formel I in der Form eines ihrer Salze enthält, insbesondere dann, wenn diese Salzform dem Wirkstoff im Vergleich zu der freien Form des Wirkstoffs oder irgendeiner anderen Salzform des Wirkstoffs, die früher verwendet wurde, verbesserte pharmakokinetische Eigenschaften verleiht. Die pharmazeutisch unbedenkliche Salzform des Wirkstoffs kann auch diesem Wirkstoff erst eine gewünschte pharmakokinetische Eigenschaft verleihen, über die er früher nicht verfügt hat, und kann sogar die Pharmakodynamik dieses Wirkstoffs in bezug auf seine therapeutische Wirksamkeit im Körper positiv beeinflussen.

Erfindungsgemäße Verbindungen der Formel I können aufgrund ihrer Molekülstruktur chiral sein und können dementsprechend in verschiedenen enantiomeren Formen auftreten. Sie können daher in racemischer oder in optisch aktiver Form vorliegen.

Da sich die pharmazeutische Wirksamkeit der Racemate bzw. der Stereoisomeren der erfindungsgemäßen Verbindungen unterscheiden kann, kann es wünschenswert sein, die Enantiomere zu verwenden. In diesen Fällen kann das Endprodukt oder aber bereits die Zwischenprodukte in enantiomere Verbindungen, durch dem Fachmann bekannte chemische oder physikalische Maßnahmen, aufgetrennt oder bereits als solche bei der Synthese eingesetzt werden.

Im Falle racemischer Amine werden aus dem Gemisch durch Umsetzung mit einem optisch aktiven Trennmittel Diastereomere gebildet. Als Trennmittel eignen sich z.B. optisch aktiven Säuren, wie die R- und S-Formen von Weinsäure, Diacetylweinsäure, Dibenzoylweinsäure, Mandelsäure, Äpfelsäure, Milchsäure, geeignet N-geschützte Aminosäuren (z.B. N-Benzoylprolin oder N-Benzolsulfonylprolin) oder die verschiedenen optisch aktiven Camphersulfonsäuren. Vorteilhaft ist auch eine chromatographische Enantiomerentrennung mit Hilfe eines optisch aktiven Trennmittels (z.B. Dinitrobenzoylphenylglycin, Cellulosetriacetat oder andere Derivate von Kohlenhydraten oder auf Kieselgel fixierte chiral derivatisierte Methacrylatpolymere). Als Laufmittel eignen sich hierfür wäßrige oder alkoholische Lösungsmittelgemische wie z.B. Hexan/Isopropanol/ Acetonitril z.B. im Verhältnis 82:15:3.

Gegenstand der Erfindung ist ferner die Verwendung der Verbindungen und/oder ihrer physiologisch unbedenklichen Salze zur Herstellung eines Arzneimittels (pharmazeutische Zubereitung), insbesondere auf nichtchemischem Wege. Hierbei können sie zusammen mit mindestens einem festen, flüssigen und/oder halbflüssigen Träger- oder Hilfsstoff und gegebenenfalls in Kombination mit einem oder mehreren weiteren Wirkstoffen in eine geeignete Dosierungsform gebracht werden.

Gegenstand der Erfindung sind ferner Arzneimittel, enthaltend mindestens eine erfindungsgemäße Verbindung und/oder ihre pharmazeutisch verwendbaren Salze, E,Z-Isomere, Diastereomere, Enantiomere, Mono-, Dihydrate und Alkoholate, einschließlich deren Mischungen in allen Verhältnissen, sowie gegebenenfalls Träger- und/oder Hilfsstoffe.

Pharmazeutische Formulierungen können in Form von Dosiseinheiten, die eine vorbestimmte Menge an Wirkstoff pro Dosiseinheit enthalten, dargereicht werden. Eine solche Einheit kann beispielsweise 0,5 mg bis 1 g, vorzugsweise 1 mg bis 700 mg, besonders bevorzugt 5 mg bis 100 mg einer erfindungsgemäßen Verbindung enthalten, je nach dem behandelten Krankheitszustand, dem Verabreichungsweg und dem Alter, Gewicht und Zustand des Patienten, oder pharmazeutische Formulierungen können in Form von Dosiseinheiten, die eine vorbestimmte Menge an Wirkstoff pro Dosiseinheit enthalten, dargereicht werden. Bevorzugte Dosierungseinheitsformulierungen sind solche, die eine Tagesdosis oder Teildosis, wie oben angegeben, oder einen entsprechenden Bruchteil davon eines Wirkstoffs enthalten. Weiterhin lassen sich solche pharmazeutischen Formulierungen mit einem der im pharmazeutischen Fachgebiet allgemein bekannten Verfahren herstellen.

Pharmazeutische Formulierungen lassen sich zur Verabreichung über einen beliebigen geeigneten Weg, beispielsweise auf oralem (einschließlich buccalem bzw. sublingualem), rektalem, nasalem, topischem (einschließlich buccalem, sublingualem oder transdermalem), vaginalem oder parenteralem (einschließlich subkutanem, intramuskulärem, intravenösem oder intradermalem) Wege, anpassen. Solche Formulierungen können mit allen im pharmazeutischen Fachgebiet bekannten Verfahren hergestellt werden, indem beispielsweise der Wirkstoff mit dem bzw. den Trägerstoff(en) oder Hilfsstoff(en) zusammengebracht wird.

An die orale Verabreichung angepaßte pharmazeutische Formulierungen können als separate Einheiten, wie z.B. Kapseln oder Tabletten; Pulver oder Granulate; Lösungen oder Suspensionen in wäßrigen oder nichtwäßrigen Flüssigkeiten; eßbare Schäume oder Schaumspeisen; oder Öl-in-Wasser-Flüssigemulsionen oder Wasser-in-Öl-Flüssigemulsionen dargereicht werden.

So läßt sich beispielsweise bei der oralen Verabreichung in Form einer Tablette oder Kapsel die Wirkstoffkomponente mit einem oralen, nicht-toxischen und pharmazeutisch unbedenklichen inerten Trägerstoff, wie z.B. Ethanol, Glycerin, Wasser u.ä. kombinieren. Pulver werden hergestellt, indem die Verbindung auf eine geeignete feine Größe zerkleinert und mit einem in ähnlicher Weise zerkleinerten pharmazeutischen Trägerstoff, wie z.B. einem eßbaren Kohlenhydrat wie beispielsweise Stärke oder Mannit vermischt wird. Ein Geschmacksstoff, Konservierungsmittel, Dispersionsmittel und Farbstoff können ebenfalls vorhanden sein.

Kapseln werden hergestellt, indem ein Pulvergemisch wie oben beschrieben hergestellt und geformte Gelatinehüllen damit gefüllt werden. Gleit- und Schmiermittel wie z.B. hochdisperse Kieselsäure, Talkum, Magnesiumstearat, Kalziumstearat oder Polyethylenglykol in Festform können dem Pulvergemisch vor dem Füllvorgang zugesetzt werden. Ein Sprengmittel oder Lösungsvermittler, wie z.B. Agar-Agar, Kalziumcarbonat oder Natriumcarbonat, kann ebenfalls zugesetzt werden, um die Verfügbarkeit des Medikaments nach Einnahme der Kapsel zu verbessern.

Außerdem können, falls gewünscht oder notwendig, geeignete Bindungs-, Schmier- und Sprengmittel sowie Farbstoffe ebenfalls in das Gemisch eingearbeitet werden. Zu den geeigneten Bindemitteln gehören Stärke, Gelatine, natürliche Zucker, wie z.B. Glukose oder Beta-Lactose, Süßstoffe aus Mais, natürliche und synthetische Gummi, wie z.B. Akazia, Traganth oder Natriumalginat, Carboxymethylzellulose, Polyethylenglykol, Wachse, u.ä. Zu den in diesen Dosierungsformen verwendeten Schmiermitteln gehören Natriumoleat, Natriumstearat, Magnesiumstearat, Natriumbenzoat, Natriumacetat, Natriumchlorid u.ä. Zu den Sprengmitteln gehören, ohne darauf beschränkt zu sein, Stärke, Methylzellulose, Agar, Bentonit, Xanthangummi u.ä. Die Tabletten werden formuliert, indem beispielsweise ein Pulvergemisch hergestellt, granuliert oder trockenverpreßt wird, ein Schmiermittel und ein Sprengmittel zugegeben werden und das Ganze zu Tabletten verpreßt wird. Ein Pulvergemisch wird hergestellt, indem die in geeigneter Weise zerkleinerte Verbindung mit einem Verdünnungsmittel oder einer Base, wie oben beschrieben, und gegebenenfalls mit einem Bindemittel, wie z.B. Carboxymethylzellulose, einem Alginat, Gelatine oder Polyvinylpyrrolidon, einem Lösungsverlangsamer, wie z.B. Paraffin, einem Resorptionsbeschleuniger, wie z.B. einem quaternären Salz und/oder einem Absorptionsmittel, wie z.B. Bentonit, Kaolin oder Dikalziumphosphat, vermischt wird. Das Pulvergemisch läßt sich granulieren, indem es mit einem Bindemittel, wie z.B. Sirup, Stärkepaste, Acadia-Schleim oder Lösungen aus Zellulose- oder Polymermaterialen benetzt und durch ein Sieb gepreßt wird. Als Alternative zur Granulierung kann man das Pulvergemisch durch eine Tablettiermaschine laufen lassen, wobei ungleichmäßig geformte Klumpen entstehen, die in Granulate aufgebrochen werden. Die Granulate können mittels Zugabe von Stearinsäure, einem Stearatsalz, Talkum oder Mineralöl gefettet werden, um ein Kleben an den Tablettengußformen zu verhindern. Das gefettete Gemisch wird dann zu Tabletten verpreßt. Die erfindungsgemäßen Verbindungen können auch mit einem freifließenden inerten Trägerstoff kombiniert und dann ohne Durchführung der Granulierungs- oder Trockenverpressungsschritte direkt zu Tabletten verpreßt werden. Eine durchsichtige oder undurchsichtige Schutzschicht, bestehend aus einer Versiegelung aus Schellack, einer Schicht aus Zucker oder Polymermaterial und einer Glanzschicht aus Wachs, kann vorhanden sein. Diesen Beschichtungen können Farbstoffe zugesetzt werden, um zwischen unterschiedlichen Dosierungseinheiten unterscheiden zu können.

Orale Flüssigkeiten, wie z.B. Lösung, Sirupe und Elixiere, können in Form von Dosierungseinheiten hergestellt werden, so daß eine gegebene Quantität eine vorgegebene Menge der Verbindung enthält. Sirupe lassen sich herstellen, indem die Verbindung in einer wäßrigen Lösung mit geeignetem Geschmack gelöst wird, während Elixiere unter Verwendung eines nichttoxischen alkoholischen Vehikels hergestellt werden. Suspensionen können durch Dispersion der Verbindung in einem nicht-toxischen Vehikel formuliert werden. Lösungsvermittler und Emulgiermittel, wie z.B. ethoxylierte Isostearylalkohole und Polyoxyethylensorbitolether, Konservierungsmittel, Geschmackszusätze, wie z.B. Pfefferminzöl oder natürliche Süßstoffe oder Saccharin oder andere künstliche Süßstoffe, u.ä. können ebenfalls zugegeben werden.

Die Dosierungseinheitsformulierungen für die orale Verabreichung können gegebenenfalls in Mikrokapseln eingeschlossen werden. Die Formulierung läßt sich auch so herstellen, daß die Freisetzung verlängert oder retardiert wird, wie beispielsweise durch Beschichtung oder Einbettung von partikulärem Material in Polymere, Wachs u.ä.

Die erfindungsgemäßen Verbindungen sowie Salze, Solvate und physiologisch funktionelle Derivate davon lassen sich auch in Form von Liposomenzuführsystemen, wie z.B. kleinen unilamellaren Vesikeln, großen unilamellaren Vesikeln und multilamellaren Vesikeln, verabreichen. Liposomen können aus verschiedenen Phospholipiden, wie z.B. Cholesterin, Stearylamin oder Phosphatidylcholinen, gebildet werden

Die erfindungsgemäßen Verbindungen sowie die Salze, Solvate und physiologisch funktionellen Derivate davon können auch unter Verwendung monoklonaler Antikörper als individuelle Träger, an die die Verbindungsmoleküle gekoppelt werden, zugeführt werden. Die Verbindungen können auch mit löslichen Polymeren als zielgerichtete Arzneistoffträger gekoppelt werden. Solche Polymere können Polyvinylpyrrolidon, Pyran-Copolymer, Polyhydroxypropylmethacrylamidphenol, Polyhydroxyethylaspartamidphenol oder Polyethylenoxidpolylysin, substituiert mit Palmitoylresten, umfassen. Weiterhin können die Verbindungen an eine Klasse von biologisch abbaubaren Polymeren, die zur Erzielung einer kontrollierten Freisetzung eines Arzneistoffs geeignet sind, z.B. Polymilchsäure, Poiyepsilon-Caprolacton, Polyhydroxybuttersäure, Polyorthoester, Polyacetale, Polydihydroxypyrane, Polycyanoacrylate und quervernetzte oder amphipatische Blockcopolymere von Hydrogelen, gekoppelt sein.

An die transdermale Verabreichung angepaßte pharmazeutische Formulierungen können als eigenständige Pflaster für längeren, engen Kontakt mit der Epidermis des Empfängers dargereicht werden. So kann beispielsweise der Wirkstoff aus dem Pflaster mittels Iontophorese zugeführt werden, wie in Pharmaceutical Research, 3(6), 318 (1986) allgemein beschrieben.

An die topische Verabreichung angepaßte pharmazeutische Verbindungen können als Salben, Cremes, Suspensionen, Lotionen, Pulver, Lösungen, Pasten, Gele, Sprays, Aerosole öder Öle formuliert sein.

Für Behandlungen des Auges oder anderer äußerer Gewebe, z.B. Mund und Haut, werden die Formulierungen vorzugsweise als topische Salbe oder Creme appliziert. Bei Formulierung zu einer Salbe kann der Wirkstoff entweder mit einer paraffinischen oder einer mit Wasser mischbaren Cremebasis eingesetzt werden. Alternativ kann der Wirkstoff zu einer Creme mit einer Öl-in-Wasser-Cremebasis oder einer Wasser-in-Öl-Basis formuliert werden.

Zu den an die topische Applikation am Auge angepaßten pharmazeutischen Formulierungen gehören Augentropfen, wobei der Wirkstoff in einem geeigneten Träger, insbesondere einem wäßrigen Lösungsmittel, gelöst oder suspendiert ist.

An die topische Applikation im Mund angepaßte pharmazeutische Formulierungen umfassen Lutschtabletten, Pastillen und Mundspülmittel.

An die rektale Verabreichung angepaßte pharmazeutische Formulierungen können in Form von Zäpfchen oder Einläufen dargereicht werden.

An die nasale Verabreichung angepaßte pharmazeutische Formulierungen, in denen die Trägersubstanz ein Feststoff ist, enthalten ein grobes Pulver mit einer Teilchengröße beispielsweise im Bereich von 20-500 Mikrometern, das in der Art und Weise, wie Schnupftabak aufgenommen wird, verabreicht wird, d.h. durch Schnellinhalation über die Nasenwege aus einem dicht an die Nase gehaltenen Behälter mit dem Pulver. Geeignete Formulierungen zur Verabreichung als Nasenspray oder Nasentropfen mit einer Flüssigkeit als Trägersubstanz umfassen Wirkstofflösungen in Wasser oder Öl.

An die Verabreichung durch Inhalation angepaßte pharmazeutische Formulierungen umfassen feinpartikuläre Stäube oder Nebel, die mittels verschiedener Arten von unter Druck stehenden Dosierspendern mit Aerosolen, Verneblern oder Insufflatoren erzeugt werden können.

An die vaginale Verabreichung angepaßte pharmazeutische Formulierungen können als Pessare, Tampons, Cremes, Gele, Pasten, Schäume oder Sprayformulierungen dargereicht werden.

Zu den an die parenterale Verabreichung angepaßten pharmazeutischen Formulierungen gehören wäßrige und nichtwäßrige sterile Injektionslösungen, die Antioxidantien, Puffer, Bakteriostatika und Solute, durch die die Formulierung isotonisch mit dem Blut des zu behandelnden Empfängers gemacht wird, enthalten; sowie wäßrige und nichtwäßrige sterile Suspensionen, die Suspensionsmittel und Verdicker enthalten können. Die Formulierungen können in Einzeldosis- oder Mehrfachdosisbehältern, z.B. versiegelten Ampullen und Fläschchen, dargereicht und in gefriergetrocknetem (lyophilisiertem) Zustand gelagert werden, so daß nur die Zugabe der sterilen Trägerflüssigkeit, z.B. Wasser für Injektionszwecke, unmittelbar vor Gebrauch erforderlich ist. Rezepturmäßig hergestellte Injektionslösungen und Suspensionen können aus sterilen Pulvern, Granulaten und Tabletten hergestellt werden.

Es versteht sich, daß die Formulierungen neben den obigen besonders erwähnten Bestandteilen andere im Fachgebiet übliche Mittel mit Bezug auf die jeweilige Art der Formulierung enthalten können; so können beispielsweise für die orale Verabreichung geeignete Formulierungen Geschmacksstoffe enthalten.

Eine therapeutisch wirksame Menge einer Verbindung der vorliegenden Erfindung hängt von einer Reihe von Faktoren ab, einschließlich z.B. dem Alter und Gewicht des Menschen oder Tiers, dem exakten Krankheitszustand, der der Behandlung bedarf, sowie seines Schweregrads, der Beschaffenheit der Formulierung sowie dem Verabreichungsweg, und wird letztendlich von dem behandelnden Arzt bzw. Tierarzt festgelegt. Jedoch liegt eine wirksame Menge einer erfindungsgemäßen Verbindung für die Behandlung im allgemeinen im Bereich von 0,1 bis 100 mg/kg Körpergewicht des Empfängers (Säugers) pro Tag und besonders typisch im Bereich von 1 bis 10 mg/kg Körpergewicht pro Tag. Somit läge für einen 70 kg schweren erwachsenen Säuger die tatsächliche Menge pro Tag für gewöhnlich zwischen 70 und 700 mg, wobei diese Menge als Einzeldosis pro Tag oder üblicher in einer Reihe von Teildosen (wie z.B. zwei, drei, vier, fünf oder sechs) pro Tag gegeben werden kann, so daß die Gesamttagesdosis die gleiche ist. Eine wirksame Menge eines Salzes oder Solvats oder eines physiologisch funktionellen Derivats davon kann als Anteil der wirksamen Menge der erfindungsgemäßen Verbindung *per se* bestimmt werden. Es läßt sich annehmen, daß ähnliche Dosierungen für die Behandlung der anderen, obenerwähnten Krankheitszustände geeignet sind. Gegenstand der Erfindung ist auch ein Set (Kit), bestehend aus getrennten Packungen von
(a) einer wirksamen Menge an einer erfindungsgemäßen Verbindung und/oder ihrer pharmazeutisch verwendbaren Salze, E,Z-Isomere, Diastereomere, Enantiomere, Mono-, Dihydrate und Alkoholate, einschließlich deren Mischungen in allen Verhältnissen, und
(b) einer wirksamen Menge eines weiteren Arzneimittelwirkstoffs.

Das Set enthält geeignete Behälter, wie Schachteln oder Kartons, individuelle Flaschen, Beutel oder Ampullen. Das Set kann z.B. separate Ampullen enthalten, in denen jeweils eine wirksame Menge an einer erfindungsgemäßen Verbindung und/oder ihrer pharmazeutisch verwendbaren Salze, E,Z-Isomere, Diastereomere, Enantiomere, Mono-, Dihydrate und Alkoholate, einschließlich deren Mischungen in allen Verhältnissen, und einer wirksamen Menge eines weiteren Arzneimittelwirkstoffs gelöst oder in lyophylisierter Form vorliegt.

### VERWENDUNG

Die vorliegenden Verbindungen eignen sich als pharmazeutische Wirkstoffe für Säugetiere, insbesondere für den Menschen, bei der Behandlung von SGK-bedingten Krankheiten.

Gegenstand der Erfindung ist somit die Verwendung von Verbindungen nach Anspruch 1, sowie ihrer pharmazeutisch verwendbaren Derivate, Solvate und Stereoisomere, einschließlich deren Mischungen in allen Verhältnissen, zur Herstellung eines Arzneimittels zur Behandlung von Krankheiten, bei denen die Hemmung, Regulierung und/oder Modulation der Signaltransduktion von Kinasen eine Rolle spielt. Bevorzugt ist hierbei SGK.

Bevorzugt ist die Verwendung von Verbindungen gemäß Anspruch 1, sowie ihrer pharmazeutisch verwendbaren Salze, E,Z-Isomere, Diastereomere, Enantiomere, Mono-, Dihydrate und Alkoholate, einschließlich deren Mischungen in allen Verhältnissen, zur Herstellung eines Arzneimittels zur Behandlung von Krankheiten, die durch Inhibierung der SGK durch die Verbindungen nach Anspruch 1 beeinflußt werden.

Die vorliegende Erfindung umfasst die Verwendung der erfindungsgemäßen Verbindungen nach Anspruch 1 und/oder ihre physiologisch unbedenklichen Salze und Solvate zur Herstellung eines Arzneimittels zur Behandlung oder Vorbeugung von Diabetes (z.B. Diabetes mellitus, diabetische Nephropathie, diabetische Neuropathie, diabetische Angiopathie und Mikroangiopathie), Fettsucht, metabolisches Syndrom (Dyslipidämie), systemische und pulmonale Hypertonie, Herzkreislauferkrankungen (z.B. kardiale Fibrosen nach Myokardinfarkt, Herzhypertrophie und Herzinsuffizienz, Arteriosklerose) und Nierenerkrankungen (z.B. Glomerulosklerose, Nephrosklerose, Nephritis, Nephropathie, Störung der Elektrolytausscheidung), allgemein bei jeglicher Art von Fibrosen und entzündlichen Prozessen (z.B. Leberzirrhose, Lungenfibrose, fibrosierende Pankreatitis, Rheumatismus und Arthrosen, Morbus Crohn, chronische Bronchitis, Strahlenfibrose, Sklerodermitis, zystische Fibrose, Narbenbildung, Morbus Alzheimer).

Die erfindungsgemäßen Verbindungen können auch das Wachstum von Krebs, Tumorzellen und Tumormetastasen hemmen und sind deshalb für die Tumortherapie geeignet.

Die erfindungsgemäßen Verbindungen finden weiterhin Verwendung zur Behandlung von Koagulopathien, wie z.B. Dysfibrinogenämie, Hypoprokonvertinämie, Hämophilie B, Stuart-Prower-Defekt, Prothrombin-Komplex-Mangel, Verbrauchskoagulopathie, Hyperfibrinolyse, Immunokoagulopathie oder komplexer Koagulopathien, wie auch bei neuronaler Erregbarkeit, z.B. Epilepsie. Die erfindungsgemäßen Verbindungen können auch bei der Behandlung eines Glaukoms oder Katarakt therapeutisch eingesetzt werden.

Die erfindungsgemäßen Verbindungen finden ferner Verwendung bei der Behandlung bakterieller Infektionen sowie in einer antiinfektiösen Therapie. Die erfindungsgemäßen Verbindungen können auch zur Steigerung der Lernfähigkeit und Aufmerksamkeit therapeutisch eingesetzt werden.

Bevorzugt ist die Verwendung von Verbindungen gemäß Anspruch 1, sowie ihrer pharmazeutisch verwendbaren Salze, E,Z-Isomere, Diastereomere, Enantiomere, Mono-, Dihydrate und Alkoholate, einschließlich deren Mischungen in allen Verhältnissen, zur Herstellung eines Arzneimittels zur Behandlung oder Vorbeugung von Diabetes, Fettsucht, metabolischem Syndrom (Dyslipidämie), systemischer und pulmonaler Hypertonie, Herzkreislauferkrankungen und Nierenerkrankungen, allgemein bei jeglicher Art von Fibrosen und entzündlichen Prozessen, Krebs, Tumorzellen, Tumormetastasen, Koagulopathien, neuronaler Erregbarkeit, Glaukom, Katarakt, bakteriellen Infektionen sowie in einer antiinfektiösen Therapie, zur Steigerung der Lernfähigkeit und Aufmerksamkeit.

Bei Diabetes handelt es sich vorzugsweise um Diabetes mellitus, diabetische Nephropathie, diabetische Neuropathie, diabetische Angiopathie und Mikroangiopathie.

Bei Herzkreislauferkrankurigen handelt es sich vorzugsweise um kardiale Fibrosen nach Myokardinfarkt, Herzhypertrophie, Herzinsuffizienz und Arteriosklerose.

Bei Nierenerkrankungen handelt es sich vorzugsweise um Glomerulosklerose, Nephrosklerose, Nephritis, Nephropathie und Störung der Elektrolytausscheidung.

Bei Fibrosen und entzündlichen Prozessen handelt es sich vorzugsweise um Leberzirrhose, Lungenfibrose, fibrosierende Pankreatitis, Rheumatismus und Arthrosen, Morbus Crohn, chronische Bronchitis, Strahlenfibrose, Sklerodermitis, zystische Fibrose, Narbenbildung, Morbus Alzheimer.

### ASSAYS

Die in den Beispielen beschriebenen erfindungsgemäßen Verbindungen wurden in den unten beschriebenen Assays geprüft, und es wurde gefunden, dass sie eine kinasehemmende Wirkung aufweisen. Weitere Assays sind aus der Literatur bekannt und könnten vom Fachmann leicht durchgeführt werden (siehe z.B. Dhanabal et al., Cancer Res. 59:189-197; Xin et al., J. Biol. Chem. 274:9116-9121; Sheu et al., Anticancer Res. 18:4435-4441; Ausprunk et al., Dev. Biol. 38:237-248; Gimbrone et al., J. Natl. Cancer lnst. 52:413-427; Nicosia et al., In Vitro 18:538- 549).

Vor- und nachstehend sind alle Temperaturen in °C angegeben. In den nachfolgenden Beispielen bedeutet "übliche Aufarbeitung": Man gibt, falls erforderlich, Wasser hinzu, stellt, falls erforderlich, je nach Konstitution des Endprodukts auf pH-Werte zwischen 2 und 10 ein, extrahiert mit Ethylacetat oder Dichlormethan, trennt ab, trocknet die organische Phase über Natriumsulfat, dampft ein und reinigt durch Chromatographie an Kieselgel und /oder durch Kristallisation. Rf-Werte an Kieselgel; Laufmittel: Ethylacetat/Methanol 9:1.
- Massenspektrometrie (MS):: EI (Elektronenstoß-Ionisation) M⁺
FAB (Fast Atom Bombardment) (M+H)⁺
ESI (Electrospray lonization) (M+H)⁺ (wenn nichts anderes angegeben)

### Beispiel 1

Die Herstellung von 3-Amino-1*H*-indazol-5-carbonsäure-3-chlor-benzylamid ("1") erfolgt analog nachstehendem Schema: 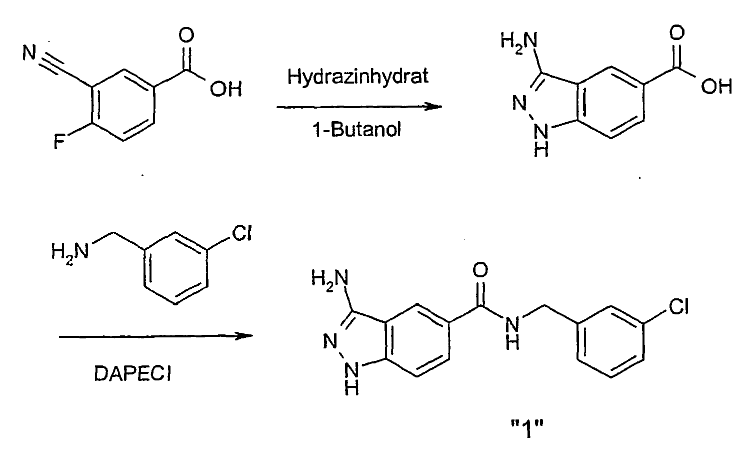
1. Eine Lösung von 22.0g (133 mmol) 3-Cyan-4-fluorbenzoesäure in 400 ml 1-Butanol wird mit 8 ml Hydrazinhydrat versetzt und 3 Stunden auf 110° C erhitzt. Das Reaktionsgemisch wird mit 1 N Natronlauge alkalisiert und mit Ethylacetat extrahiert. Die wässrige Phase wird mit 1 N HCl auf einen pH-Wert von ca. 4 gebracht und der entstandene Niederschlag abfiltriert: 3-Amino-1*H*-indazol-5-carbonsäure als farbloser Feststoff; ESI 178.
2. Eine Lösung von 88.6 mg (0.50 mmol) 3-Amino-1H-indazol-5-carbonsäure und 70.8 mg (0.50 mmol) 3-Chlorbenzylamin in 1 ml DMF wird mit 125 mg (0.65 mmol) *N*-(3-Dimethylaminopropyl)-*N*'-ethylcarbodiimidhydrochlorid (DAPECI) versetzt und 18 Stunden bei Raumtemperatur gerührt. Das Reaktionsgemisch wird auf gesättigte Natriumhydrogencarbonatlösung gegeben und der entstandene Niederschlag abfiltriert: 3-Amino-1*H*-indazol-5-carbonsäure-3-chlorbenzylamid ("1") als farbloser Feststoff; ESI 301.

### Beispiel 2

Die Herstellung von [3-(3-Amino-1*H*-indazol-5-yl)-phenoxy]-essigsäure ("2") erfolgt analog nachstehendem Schema:

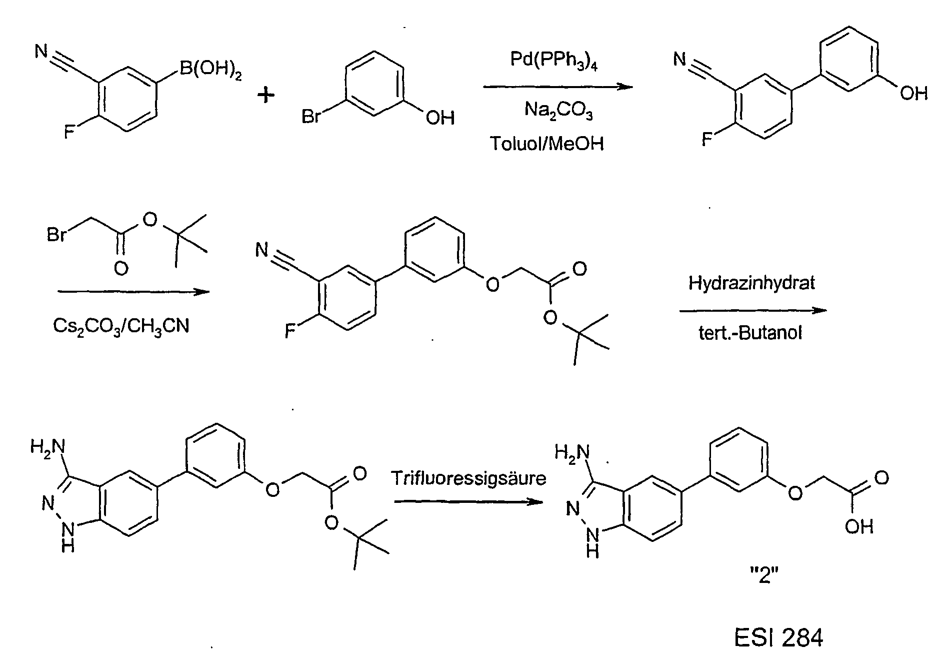

### Beispiel 3

Die Herstellung von N-(3-Amino-1*H*-indazol-5-yl)-2-(3-hydroxy-phenyl)-acetamid Hydrochlorid ("3") erfolgt analog nachstehendem Schema: 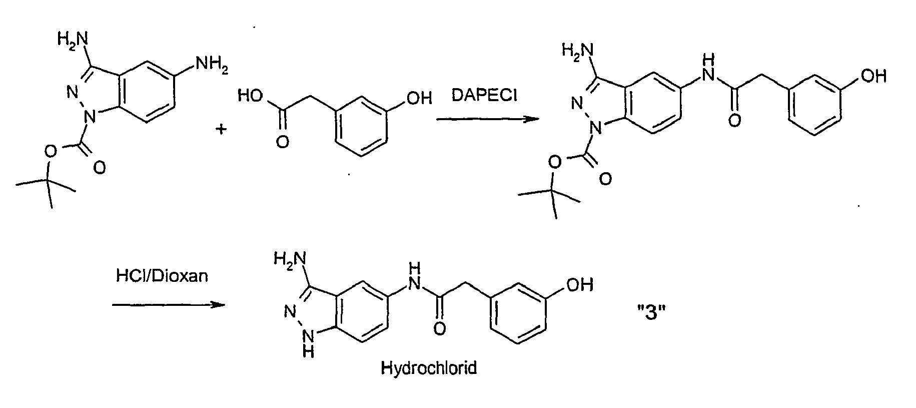
1. Eine Lösung von 149 mg (0.60 mmol) 3,5-Diaminoindazol-1-carbonsäure-tert.-butylester (Herstellung beschrieben in WO 03/064397), 91.3 mg (0.60 mmol) 3-Hydroxyphenylessigsäure in 3 ml DMF wird mit 153 mg (0.80 mmol) DAPECI versetzt und 18 Stunden bei Raumtemperatur gerührt. Das Reaktionsgemisch wird auf Wasser gegeben und der entstandene Niederschlag abfiltriert: 3-Amino-5-[2-(3-hydroxy-phenyl)-acetylamino]-indazol-1-carbonsäure-tert-butylester als farbloser Feststoff; ESI 383.
2. 110 mg (0.288 mmol) (3-Amino-5-[2-(3-hydroxy-phenyl)-acetylamino]-indazol-1-carbonsäure-tert-butylester wird mit 10 ml 4 N HCl in Dioxan versetzt und 18 Stunden bei Raumtemperatur gerührt. Das Reaktionsgemisch wird eingedampft, mit Diethylether digeriert und filtriert. Der Rückstand wird getrocknet: N-(3-Amino-1*H*-indazol-5-yl)-2-(3-hydroxyphenyl)-acetamid Hydrochlorid ("3") als farbloser Feststoff; ESI 283.

### Beispiel 3.1

Die Herstellung von N-(3-Amino-1*H*-indazol-5-ylmethyl)-3-chlorbenzamid Hydrochlorid ("3.1 ") erfolgt analog nachstehendem Schema: 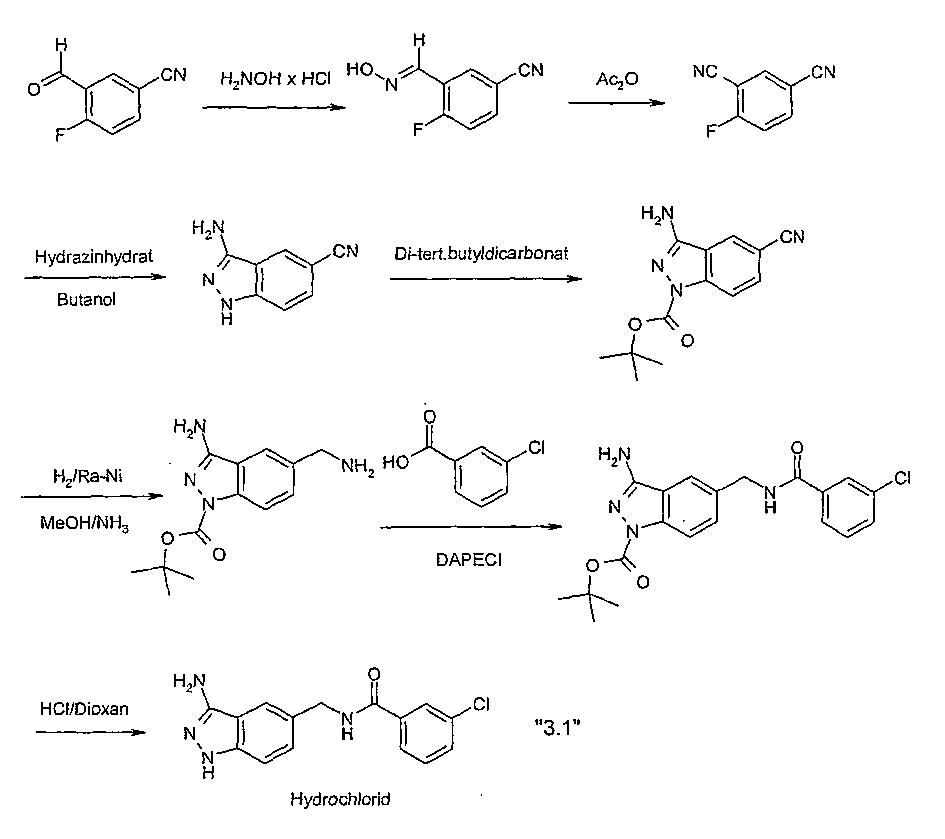
1. Zu einer Lösung von 4.0 g (58 mmol) Hydroxylammoniumchlorid in 20 ml Wasser wird eine Lösung von 4.5 g (80 mmol) Kaliumhydroxid in 20 ml Wasser gegeben und die entstandene Lösung auf 0 °C gekühlt. Zu dieser Lösung wird unter Rühren langsam 5.0 g (33.5 mmol) 4-Fluor-3-formyl-benzonitril gegeben. Man rührt noch 2 Stunden bei Raumtemperatur, gibt dann Essigsäure bis zum Erreichen eines pH-Wertes von 7-8 zu und filtriert den entstandenen Feststoff ab: 4-Fluor-3-(hydroxyimino-methyl)-benzonitril als farbloser Feststoff; ESI 165.
2. Eine Lösung von 5.0 g (30 mmol) 4-Fluor-3-(hydroxyimino-methyl)-benzonitril in 40 ml Essigsäureanhydrid wird 3 Stunden zum Sieden erhitzt. Überschüssiges Anhydrid wird abdestilliert und der Rückstand zwischen 200 ml tert-Butylmethylether und gesättigter Natriumhydrogencarbonatlösung verteilt. Die organische Phase wird über Natriumsulfat getrocknet, eingedampft, in Diethylether gelöst und mit Petrolether versetzt. Der entstandene Feststoff wird abfiltriert: 4-Fluorisophthalonitril als farbloser Feststoff; ESI 147.
3. Eine Lösung von 3.30 g (22.6 mmol) 4-Fluorisophthalonitril in 50 ml 1-Butanol wird mit 4.86 ml (100 mmol) Hydrazinhydrat versetzt und 1 Stunde auf 110 °C erhitzt. Man lässt das Reaktionsgemisch abkühlen und gibt 300 ml Wasser zu. Der entstandene Niederschlag wird abfiltriert und im Vakuum getrocknet: 3-Amino-1*H*-indazol-5-carbonitril als farbloser Feststoff; ESI 159.
4. Eine Lösung von 2.60 g (16.4 mmol) 3-Amino-1*H*-indazol-5-carbonitril in 200 ml THF wird mit 2.02 g (20.0 mmol) Triethylamin, 696 mg (5.70 mmol) 4-(Dimethylamino)-pyridin und 4.37 g (20.0 mmol) Di-tert.-butyldicarbonat versetzt und das Reaktionsgemisch 2 Stunden bei Raumtemperatur gerührt. Der größte Teil des THF wird abdestilliert und der Rückstand zwischen Ethylacetat und gesättigter Ammoniumchloridlösung verteilt. Die organische Phase wird über Natriumsulfat getrocknet, eingedampft und der Rückstand aus tert.-Butylmethylether/Petrolether umkristallisiert: 3-Amino-5-cyan-indazol-1-carbonsäure-tert.-butylester als farbloser Feststoff; ESI 259.
5. Eine Lösung von 3.70 g (14.3 mmol) 3-Amino-5-cyan-indazol-1-carbonsäure-tert.-butylester in methanolischer Ammoniaklösung wird mit 1.8 g wasserfeuchtem Raney-Nickel versetzt und bei 40 °C und einem Druck von 4 bar hydriert. Der Katalysator wird abfiltriert und das Filtrat eingedampft: 3-Amino-5-aminomethyl-indazol-1-carbonsäure-tert-butylester als farbloser Feststoff; ESI 263.
6. Eine Lösung von 157.0 mg (0.60 mmol) 3-Amino-5-aminomethylindazol-1-carbonsäure-tert.-butylester und 93.9 mg (0.60 mmol) 3-Chlorbenzoesäure in 3 ml DMF wird mit 153 mg (0.80 mmol) DAPECI versetzt und 18 Stunden bei Raumtemperatur gerührt. Das Reaktionsgemisch wird auf gesättigte Natriumhydrogencarbonatlösung gegeben und der entstandene Niederschlag abfiltriert. Der Rückstand wird an einer Kieselgelsäule mit Ethylacetat als Laufmittel chromatographiert: 3-Amino-5-[(3-chlor-benzoylamino)-methyl]-indazol-1-carbonsäure-tert.-butylester als farbloser Feststoff, ESI 401.
7. 100 mg (0.249 mmol) 3-Amino-5-[(3-chlor-benzoylamino)-methyl]-indazol-1-carbonsäure-tert.-butylester werden mit 10 ml 4 N HCl in Dioxan versetzt und 18 Stunden bei Raumtemperatur stehen lassen. Das Reaktionsgemisch wird eingedampft und im Vakuum getrocknet, mit Diethylether digeriert und filtriert. Der Rückstand wird im Vakuum getrocknet: N-(3-Amino-1*H*-indazol-5-ylmethyl)-3-chlorbenzamid Hydrochlorid ("3.1") als farbloser Feststoff; ESI 301.

### Beispiel 4

Die Herstellung von N-(3-Amino-1*H-*indazol-6-yl)-2-(3-chlor-4-methoxyphenyl)-acetamid ("4") erfolgt analog nachstehendem Schema: 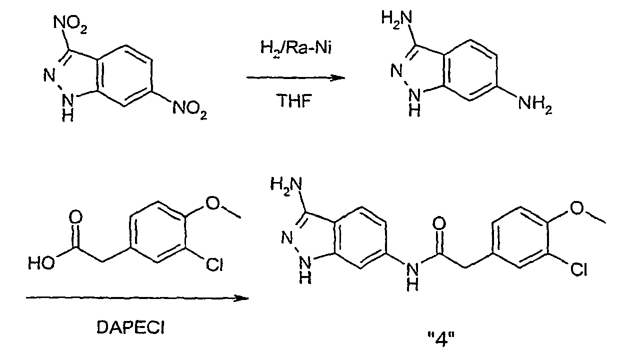
1. Eine Lösung von 1.71 g (8.22 mmol) 3,6-Dinitro-1 H-indazol [hergestellt nach J. Usarewicz et al., Pharmazie 47, 15 (1992)] in 100 ml THF wird mit 340 mg THF-feuchtem Raney-Nickel versetzt und bei Raumtemperatur und Normaldruck hydriert. Der Katalysator wird abfiltriert und das Filtrat eingedampft: 1*H*-Indazol-3,6-diamin als brauner Feststoff; ESI 149.
2. Eine Lösung von 47.1 mg (0.50 mmol) 1*H*-Indazol-3,6-diamin, 100 mg (0.50 mmol) 3-Chlor-4-methoxyphenylessigsäure in 1ml DMF wird mit 124 mg (0.65 mmol) DAPECI versetzt und 18 Stunden bei Raumtemperatur gerührt. Das Reaktionsgemisch wird auf gesättigte Natriumhydrogencarbonatlösung gegeben und der entstandene Niederschlag abfiltriert. Der Rückstand wird an einer Kieselgelsäule mit Ethylacetat als Laufmittel chromatographiert: N-(3-Amino-1*H*-indazol-6-yl)-2-(3-chlor-4-methoxyphenyl)-acetamid ("4") als farbloser Feststoff; ESI 331.

### Beispiel.4.1

Die Herstellung von 3-Amino-1*H*-indazol-6-carbonsäure-3-chlorbenzylamid ("4.1 ") erfolgt analog nachstehendem Schema: 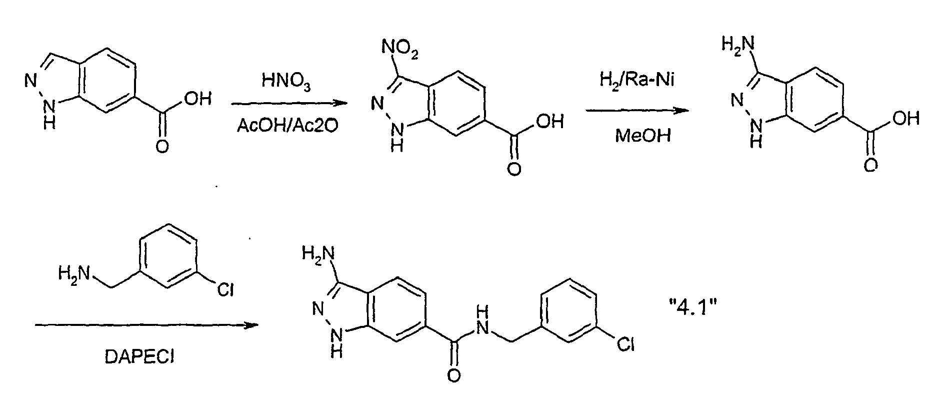
1. Eine Lösung von 4.80 g (29.6 mmol) 1*H*-Indazol-6-carbonsäure (Herstellung beschrieben in EP 0 242 167) in 40 ml Essigsäure wird auf 45 °C erwärmt. Zu dieser Lösung werden 1.95 ml (47.0 mmol) 100%ige Salpetersäure zugetropft. Man rührt eine Stunde bei 45 °C, gibt 10 ml Essigsäureanhydrid zu und rührt noch 18 Stunden bei dieser Temperatur. Nach Abkühlen auf Raumtemperatur wird das Reaktionsgemisch zunächst mit Wasser, dann mit 1 N NaOH bis zur alkalischen Reaktion versetzt. Nach 1 Stunde wird mit konz. HCl angesäuert. Der entstandene Niederschlag wird abfiltriert: 3-Nitro-1H-indazol-6-carbonsäure als gelblicher Feststoff; ESI 208.
2. Eine Lösung von 4.50 g (21.7 mmol) 3-Nitro-1*H*-indazol-6-carbonsäure in 100 ml Methanol wird mit 1.0 g methanolfeuchtem Raney-Nickel versetzt und bei Raumtemperatur und Normaldruck hydriert. Der Katalysator wird abfiltriert und das Filtrat eingedampft: 3-Amino-1*H*-indazol-6-carbonsäure als farbloser Feststoff; ESI 178.
3. Eine Lösung von 88.6 mg (0.50 mmol) 3-Amino-1*H*-indazol-6-carbonsäure und 70.8 mg (0.50 mmol) 3-Chlorbenzylamin in 1 ml DMF wird mit 125 mg (0.65 mmol) DAPECI versetzt und 18 Stunden bei Raumtemperatur gerührt. Das Reaktionsgemisch wird auf gesättigte Natriumhydrogencarbonatlösung gegeben und der entstandene Niederschlag abfiltriert: 3-Amino-1*H*-indazol-6-carbonsäure-3-chlor-benzylamid ("4.1") als farbloser Feststoff: ESI 301.

### Beispiel 5

Die Herstellung von 6-(4-Phenoxy-phenyl)-1*H*-indazol-3-ylamin ("5") erfolgt analog nachstehendem Schema:

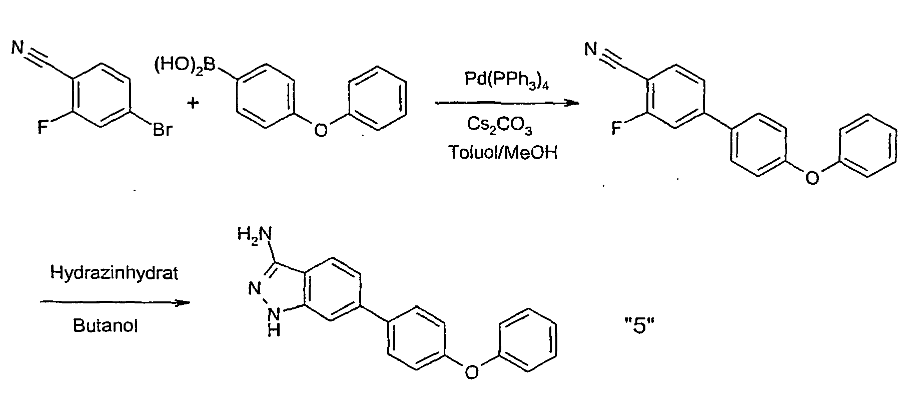

### Beispiel 6

Die Herstellung von 5-(3-Methansulfonyl-phenyl)-1*H*-indazol-3-ylamin ("6") erfolgt analog nachstehendem Schema:

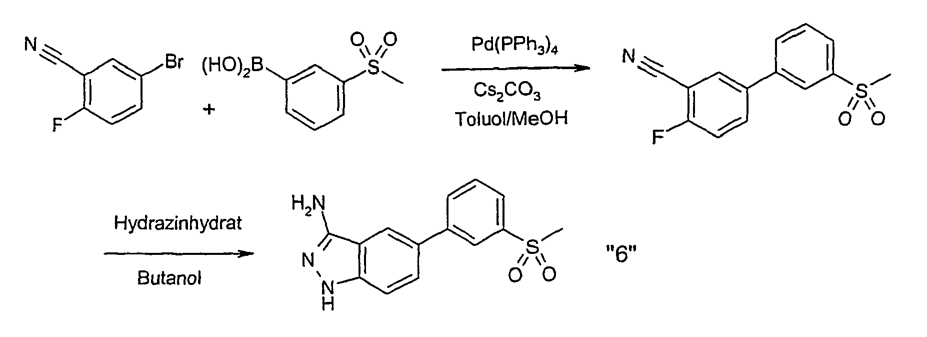

### Beispiel 6.1

Die Herstellung von 6-(3-Chlor-4-methoxy-benzyloxy)-1*H*-indazol-3-ylamin ("6.1") erfolgt analog nachstehendem Schema:

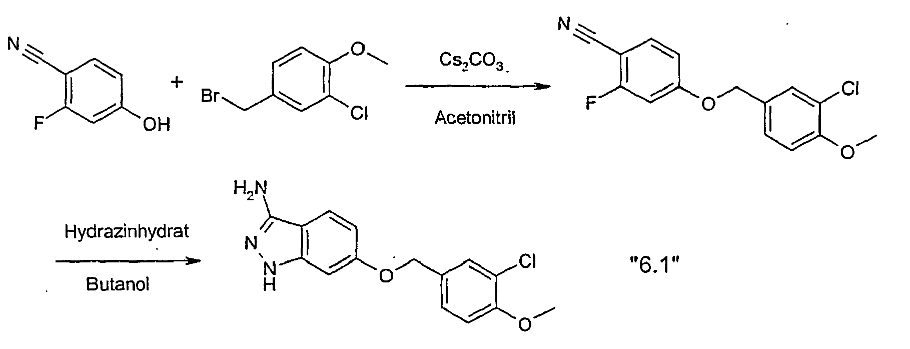

### Beispiel 6.2

Die Herstellung von N5-(3-Chlor-4-methoxy-benzyl)-1*H*-indazol-3 ,5-diamin ("6.2") und N5,N5-Bis-(3-chlor-4-methoxy-benzyl)-1*H*-indazol-3,5-diamin ("6.2a") erfolgt analog nachstehendem Schema:

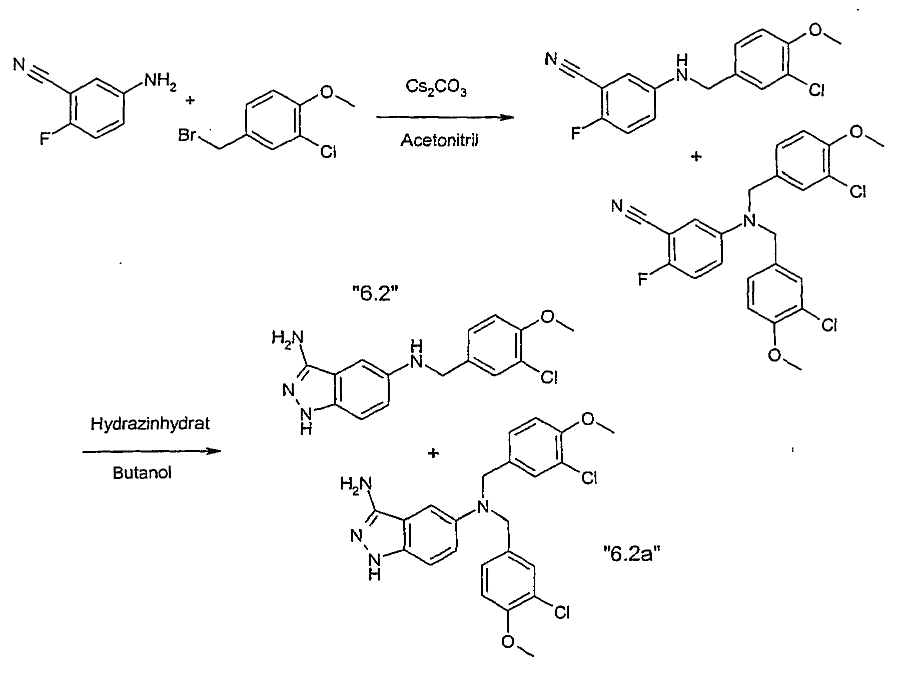

Die beiden Verbindungen werden durch präparative Dünnschichtchromatographie (Si60, Ethylacetat/Methanol 9:1) getrennt.

### Beispiel 6.3

Die Herstellung von 1-(3-Amino-1*H*-indazol-5-yl)-3-(4-benzyloxy-phenyl)-harnstoff ("6.3") erfolgt analog nachstehendem Schema:

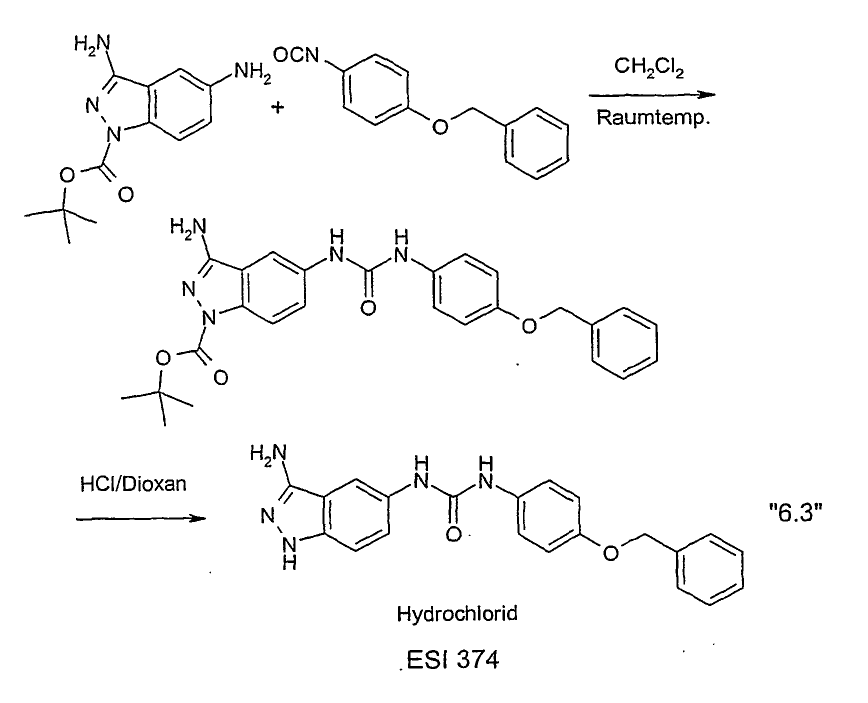

Analog erhält man die Verbindung 1-(3-Amino-1*H*-indazol-5-yl)-3-(3-chlorphenyl)-harnstoff ("6.3a"), Hydrochlorid; ESI 302.

### Beispiel 7

Die Herstellung von (E)-3-(3-Amino-1*H*-indazol-5-yl)-N-(3-hydroxy-benzyl)-acrylamid ("162") erfolgt analog nachstehendem Schema: 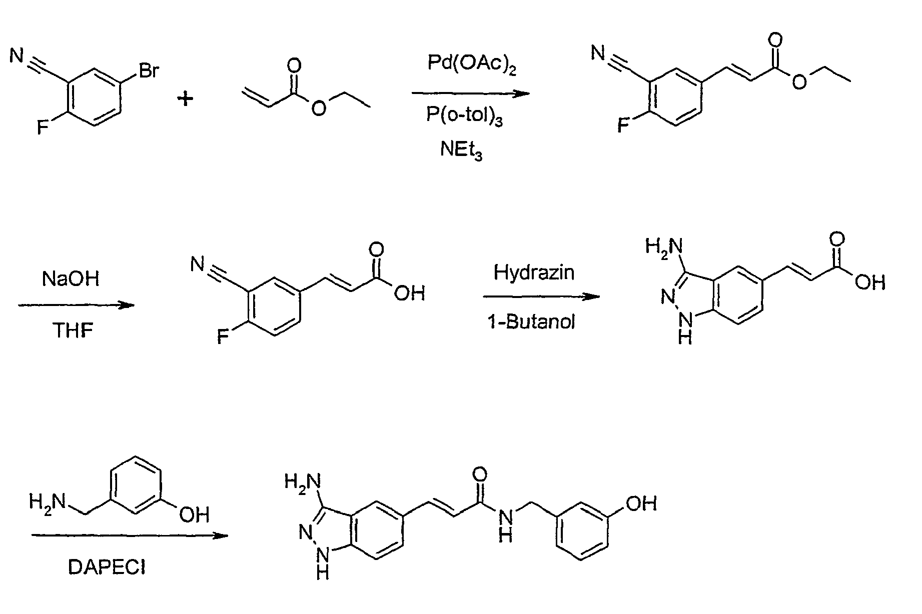
1. Eine Lösung von 10.0 g (50.0 mmol) 5-Brom-2-fluorbenzonitril in 70 ml Acetonitril wird mit 5.50 ml Ethylacrylat, 500 mg (2.2 mmol) Palladium(11)-acetat, 500 mg (2.2 mmol) Tri-o-tolylphosphin und 14 ml Triethylamin versetzt und 2 Tage unter Stickstoff zum Sieden erhitzt. Nach Abkühlen auf Raumtemperatur wird das Reaktionsgemisch mit Wasser versetzt und dreimal mit Toluol extrahiert. Die vereinigten organischen Phasen werden über Natriumsulfat getrocknet und eingedampft: 3-(3-Cyan-4-fluorphenyl)-acrylsäureethylester als farbloser Feststoff, ESI 220.
2. Eine Lösung von 10.9 g (49.8 mmol) 3-(3-Cyan-4-fluorphenyl)-acrylsäureethylester in 200 ml THF wird mit 60 ml 1 N wässriger Natronlauge versetzt und das Reaktionsgemisch 2 Tage bei Raumtemperatur gerührt. Das THF wird abdestilliert, der wässrige Rückstand mit konz. HCl angesäuert und mit Ethylacetat extrahiert. Die organische Phase wird über Natriumsulfat getrocknet und eingedampft: 3-(3-Cyan-4-fluorphenyl)-acrylsäure als farbloser Feststoff, ESI 192.
3. Eine Lösung von 6.40g (33.5 mmol) 3-(3-Cyan-4-fluorphenyl)-acrylsäure in 50 ml 1-Butanol wird mit 10 ml (206 mmol) Hydraziniumhydroxid versetzt und 3 Stunden auf 100° C erhitzt. Das Reaktionsgemisch wird im Vakuum auf ein Volumen von ca. 20 ml eingedampft und mit Wasser versetzt. Der entstandene Niederschlag wird abfiltriert und getrocknet: 3-(3-Amino-1*H*-indazol-5-yl)-acrylsäure als farbloser Feststoff, ESI 204.
4. Eine Lösung von 203 mg (1.00 mmol) 3-(3-Amino-1*H*-indazol-5-yl)-acrylsäure und 123 mg (1.00 mmol) 3-Hydroxybenzylamin in 2 ml DMF wird mit 287 mg (1.50 mmol) *N*-(3-Dimethylaminopropyl)-*N'*-ethylcarbodiimidhydrochlorid (DAPECI) versetzt und 18 Stunden bei Raumtemperatur gerührt. Das Reaktionsgemisch wird mit Wasser und 1 N HCl versetzt und mit Ethylacetat extrahiert. Die organische Phase wird über Natriumsulfat getrocknet, eingedampft und der Rückstand an einer Kieselgelsäule mit Ethylacetat/Methanol 9:1 chromatographiert. Man erhält 3-(3-Amino-1*H*-indazol-5-yl)-N-(3-hydroxybenzyl)-acrylamid ("162") als farblosen Feststoff, ESI 309.

### Beispiel 8

Die Herstellung von 3-(3-Amino-1*H*-indazol-5-yl)-N-(3-chlor-benzyl)-propionamid ("163") erfolgt analog nachstehendem Schema:

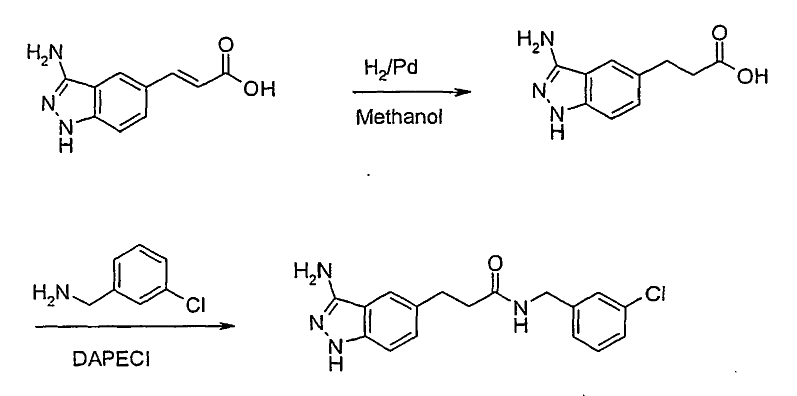

### Beispiel 9

Die Herstellung von 5-(5-Trifluormethyl-1*H*-imidazol-2-yl)-1*H*-indazol-3-ylamin ("164") erfolgt analog nachstehendem Schema: 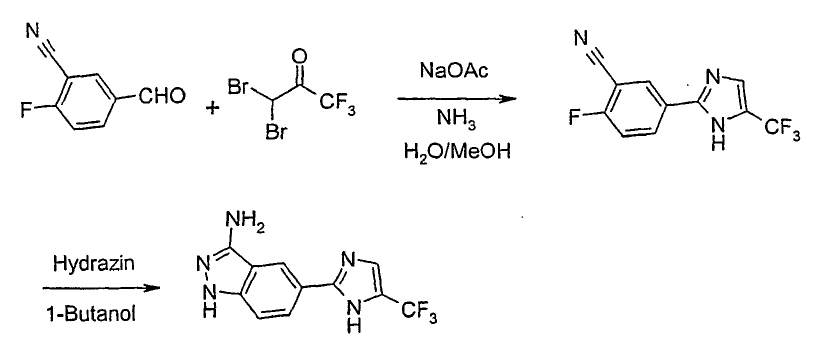
1. 16.2 g (60.0 mmol) 1,1-Dibrom-3,3,3-trifluoraceton werden mit 68 ml Wasser vermischt und 16.3 g (120 mmol) Natriumacetat-Trihydrat zugegeben. Diese Lösung wird auf 90° C erhitzt und in einer Portion zu einer vorher bereiteten Suspension von 8.95 g (60.0 mmol) 2-Fluor-4-formylbenzonitril in 300 ml Methanol und 84 ml konz. Ammoniak gegeben. Das Reaktionsgemisch wird 40 Stunden bei Raumtemperatur gerührt. Es wird auf ein Volumen von ca. 200 ml eingeengt und der entstandene Niederschlag abfiltriert: rohes 2-Fluor-5-(5-trifluormethyl-1*H-*imidazol-2-yl)-benzonitril als gelbbrauner Feststoff (ESI 256), der ohne weitere Reinigung für Folgereaktionen eingesetzt wird.
2. Eine Lösung von 2.00 g 2-Fluor-5-(5-trifluormethyl-1 H-imidazol-2-yl)-benzonitril (ca. 50%ig, ca. 3.9 mmol) in 20 ml 1-Butanol wird mit 1.17 ml (24 mmol) Hydraziniumhydroxid versetzt und 3 Stunden auf 100° C erhitzt. Das Reaktionsgemisch wird eingedampft und der Rückstand in Ethylacetat aufgenommen. Der entstandene Niederschläg wird abfiltriert und getrocknet: 5-(5-Methyl-1*H*-imidazol-2-yl)-1*H*-indazol-3-ylamin ("164") als gelber Feststoff, ESI 268.

### Beispiel 10

Die Herstellung von (3-Chlor-phenyl)-essigsäure-[1-(3-amino-1*H*-indazol-5-yl)-meth-(E)-yliden]-hydrazid ("165") erfolgt analog nachstehendem Schema: 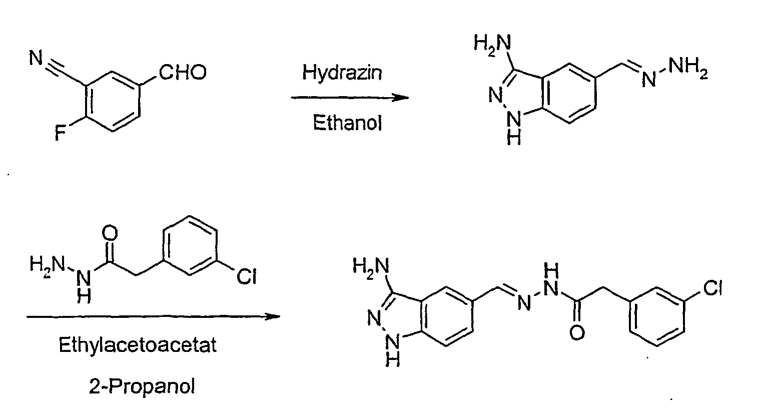
1. Eine Suspension von 2.00 g (13.4 mmol) 3-Cyan-4-fluorbenzaldehyd in 10 ml Ethanol wird mit 9.0 ml (185 mmol) Hydraziniumhydroxid versetzt und 5 Stunden auf 60° C erhitzt. Ethanol wird abdestilliert und der Rückstand 14 Stunden im Kühlschrank belassen. Die entstandenen Kristalle werden abfiltriert: 5-Hydrazonomethyl-1*H*-indazol-3-ylamin als gelbliche Kristalle; ESI 176.

Eine Lösung von 300 mg (1.71 mmol) Hydrazonomethyl-1*H*-indazol-3-ylamin, 318 mg (1.72 mmol) (3-Chlorphenyl)-essigsäurehydrazid und 224 mg (1.72 mmol) Ethylacetoacetat in 10 ml 2-Propanol wird 10 Stunden auf 80° C erhitzt. Das Reaktionsgemisch wird eingedampft und an einer Kieselgelsäule mit Dichlormethan/Methanol 8:2 als Laufmittel chromatographiert: (3-Chlor-phenyl)-essigsäure-[1-(3-amino-1*H*-indazol-5-yl)-meth-(E)-yliden]-hydrazid ("165") als gelblicher Feststoff; ESI 328.

### Beispiel 11

Die Herstellung von 5-(5-Methyl-1*H*-imidazol-2-yl)-1*H*-indazol-3-ylamin ("166") erfolgt analog nachstehendem Schema:

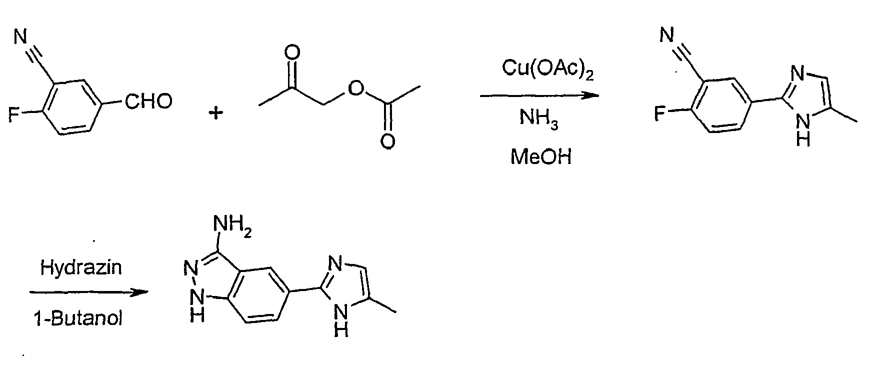

### Beispiel 12

Die Herstellung von 5-(3-Amino-1*H*-indazoi-5-yl)-furan-2-carbonsäure ("167") erfolgt analog nachstehendem Schema: 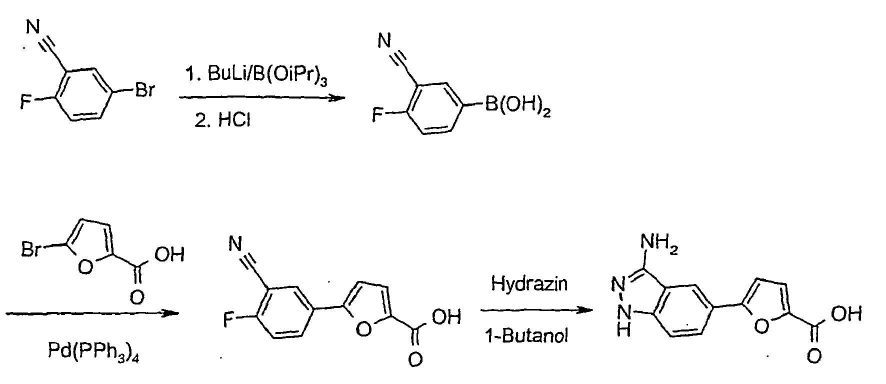
1. Eine Lösung von 5.00 g (25.0 mmol) 5-Brom-2-fluorbenzonitril und 5.64 g (30.0 mmol) Trüsopropylborat in einem Gemisch von 10 ml THF und 40 ml Toluol wird unter Stickstoff auf-70° C gekühlt. Bei dieser Temperatur werden 12 ml einer 15%igen Lösung von n-Butyllithium in Hexan (30 mmol) innerhalb von einer Stunde zugetropft. Man erwärmt das Reaktionsgemisch langsam auf-20° C und gibt dann 25 ml 1N HCl zu. Nach Erwärmen auf Raumtemperatur wird die organische Phase abgetrennt, über Natriumsulfat getrocknet und eingedampft. Der feste Rückstand wird in tert.-Butylmethylether aufgenommen und der Niederschlag filtriert und getrocknet: 3-Cyan-4-fluorbenzolboronsäure als farbloser Feststoff, ESI 166.
2. Eine Lösung von 1.30 g (7.88 mmol) 3-Cyan-4-fluorbenzolboronsäure und 1.62 g (8.50 mmol) 5-Brom-2-furansäure in einem Gemisch von 10 ml Toluol und 8 ml THF wird unter Stickstoff mit 260 mg (0.23 mol) Tetrakis(triphenylphosphin)palladium(0) versetzt und eine Lösung von 1.40 g (16.7 mmol) Natriumhydrogencarbonat in 10 ml Wasser zugegeben. Das Reaktionsgemisch wird 3 Stunden unter kräftigem Rühren zum Sieden erhitzt. Nach Abkühlen wird das Reaktionsgemisch zwischen Wasser und Ethylacetat verteilt. Die wässrige Phase wird mit konz. HCl angesäuert und mit Ethylacetat extrahiert. Die organische Phase wird über Natriumsulfat getrocknet und eingedampft: 5-(3-Cyan-4-fluorphenyl)-furan-2-carbonsäure als gelblicher Feststoff, ESI 232.
3. Eine Lösung von 870 mg (3.76 mmol) 5-(3-Cyan-4-fluorphenyl)-furan-2-carbonsäure in 20 ml 1-Butanol wird mit 1.85 g (37.0 mmol) Hydraziniumhydroxid versetzt und 12 Stunden auf 80° C erhitzt. Der entstandene Niederschlag wird abfiltriert und mit Methanol gewaschen: 5-(3-Amino-1*H-*indazol-5-yl)-furan-2-carbonsäure als farbloser Feststoff, ESI 244.

### Beispiel 13

Die Herstellung von 2-(3-Amino-1*H*-indazol-6-yl)-oxazol-4-carbonsäure ("168") erfolgt analog nachstehendem Schema:

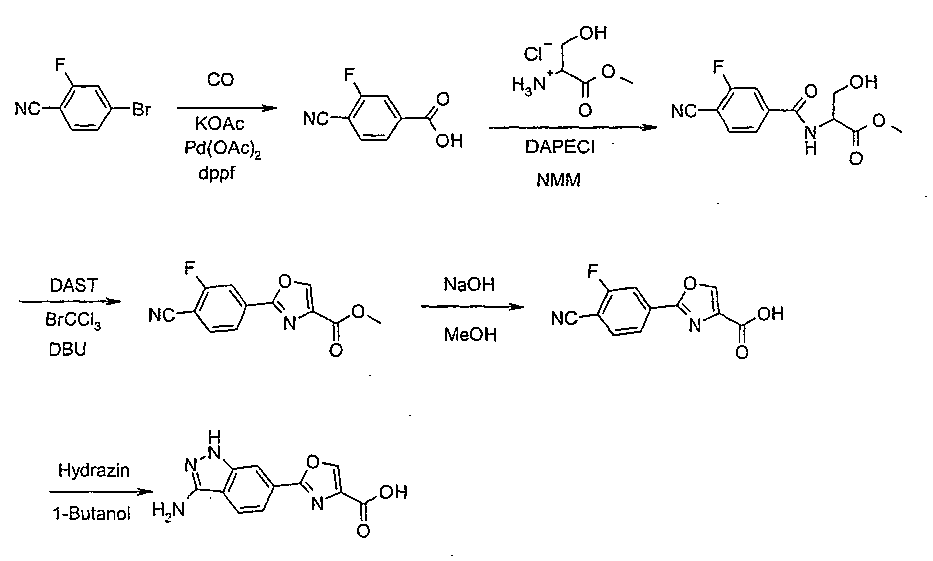

### Beispiel 14

Die Herstellung von [5-(3-Amino-1*H*-indazol-6-yl)-furan-2-yl]-methanol ("169") erfolgt analog nachstehendem Schema:

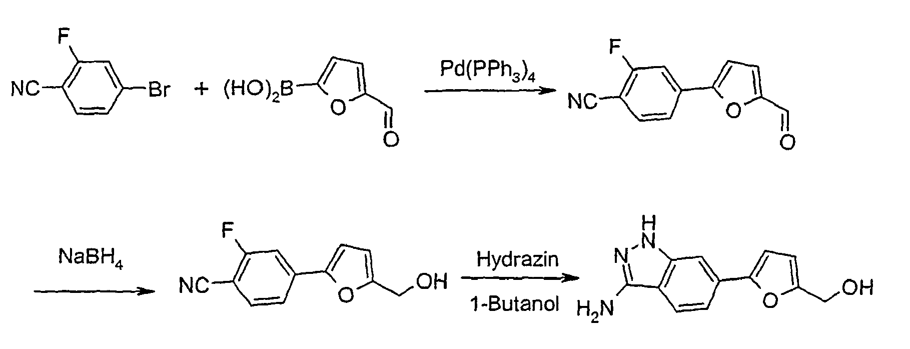

### Beispiel 15

Die Herstellung von 6-(5-Methyl-oxazol-2-yl)-1H-indazol-3-ylamin ("170") erfolgt analog nachstehendem Schema: 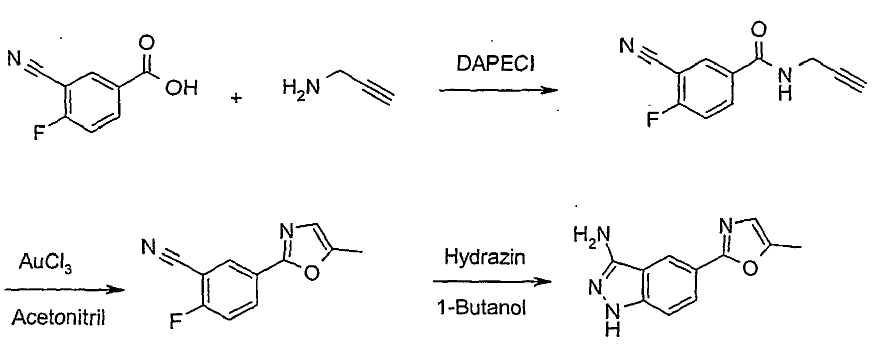
1. Eine Lösung von 3.00 g (18.2 mmol) 3-Cyan-4-fluorbenzoesäure und 1.01 g (18.2 mmol) Propargylamin in 10 ml DMF wird mit 4.53 g (23.6 mmol) *N-*(3-Dimethylaminopropyl)-*N'*-ethylcarbodiimidhydrochlorid (DAPECI) und 2.78 g (18.2 mmol) 1-Hydroxybenzotriazol-Hydrat versetzt und 18 Stunden bei Raumtemperatur gerührt. Das Reaktionsgemisch wird mit Wasser versetzt und der entstandene Niederschlag abfiltriert: 3-Cyan-4-fluor-N-prop-2-inyl-benzamid als bräunlicher Feststoff, ESI 203.
2. Eine Lösung von 2.10 g (10.4 mmol) 3-Cyan-4-fluor-N-prop-2-inyl-benzamid und 315 mg (1.04 mmol) Gold(III)chlorid in 20 ml Acetonitril wird 18 Stunden bei Raumtemperatur gerührt. Das Reaktionsgemisch wird filtriert und das Filtrat eingedampft: 2-Fluor-5-(5-methyloxazol-2-yl)-benzonitril als bräunlicher Feststoff, ESI 203.
3. Eine Lösung von 100 mg (0.495 mmol) 2-Fluor-5-(5-methyloxazol-2-yl)-benzonitril in 1 ml 1-Butanol wird mit 120 µl (2.4 mmol) Hydraziniumhydroxid versetzt und 12 Stunden auf 100° C erhitzt. Das Reaktionsgemisch wird eingedampft und der Rückstand durch präparative HPLC gereinigt: 5-(5-Methyl-oxazol-2-yl)-1 H-indazol-3-ylamin ("170") als farbloser Feststoff; ESI 215.

Analog erhält man die nachstehenden Verbindungen

| Nr. | Strukturformel | ESI | Herstellung analog Beispiel Nr. |
|---|---|---|---|
| 7 | 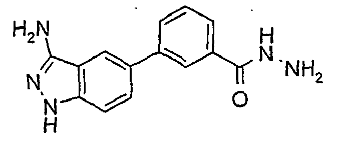 | 268 | 6 |
| 8 | 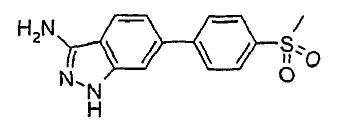 | 288 | 6 |
| 9 | 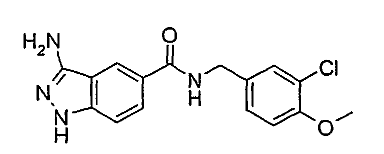 | 332 | 1 |
| 10 | 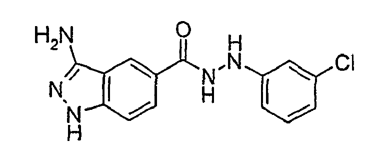 | 303 | 1 |
| 11 | 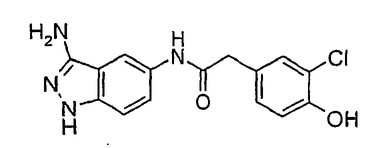 | 318 | 3 |
| 12 | 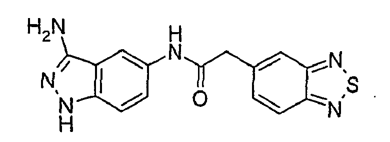 | 325 | 3 |
| 13 | 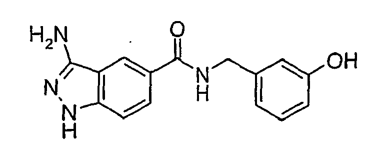 | 283 | 1 |
| 14 | 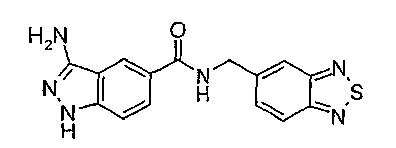 | | 1 |
| 15 | 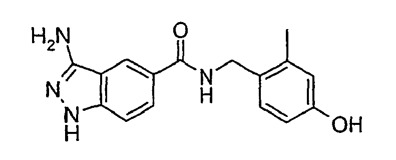 | | 1 |
| 16 | 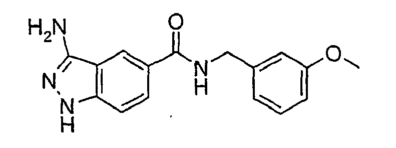 | | 1 |
| 17 | 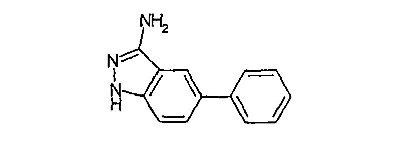 | 210 | 5 |
| 18 | 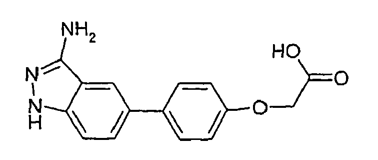 | 284 | 2 |
| 19 | 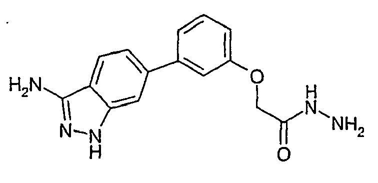 | 298 | 6 |
| 20 | 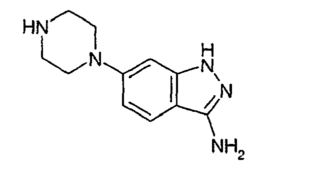 | 217 | 5,6 |
| 21 | 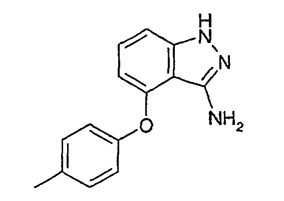 | 240 | 5, 6 |
| 22 | 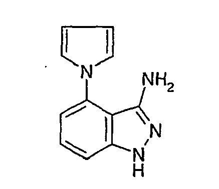 | 199 | 5, 6 |
| 23 | 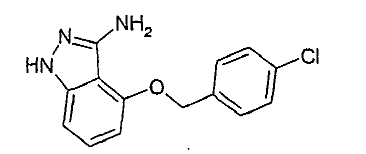 | 275 | 6.1 |
| 24 | 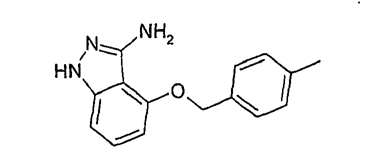 | 254 | 6.1 |
| 25 | 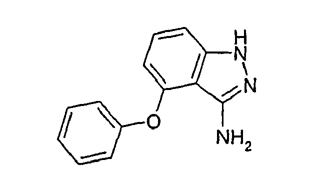 | 226 | 5 |
| 26 | 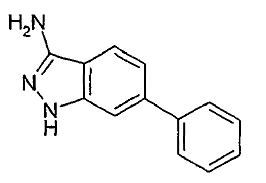 | 210 | 5 |
| 27 | 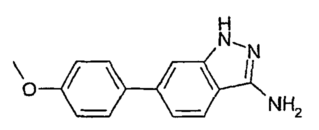 | 240 | 5 |
| 28 | 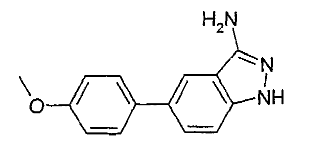 | 240 | 6 |
| 29 | 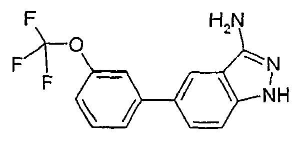 | 294 | 6 |
| 30 | 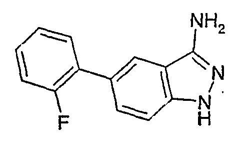 | 228 | 6 |
| 31 | 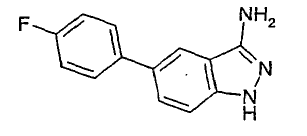 | 228 | 6 |
| 32 | 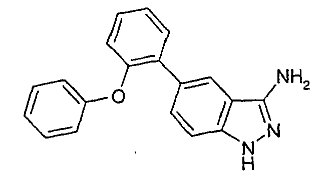 | 302 | 6 |
| 33 | 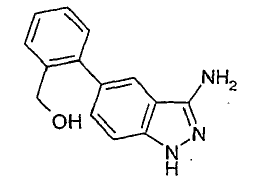 | 240 | 5 |
| 34 | 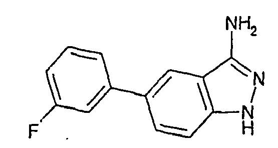 | 228 | 6 |
| 35 | 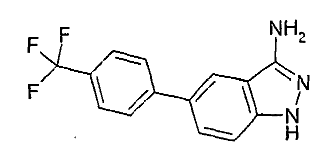 | 278 | 6 |
| 36 | 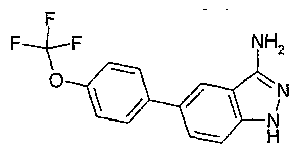 | 294 | 6 |
| 37 | 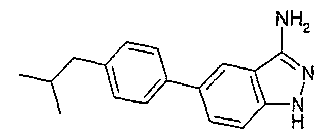 | 26 | 6 |
| 38 | 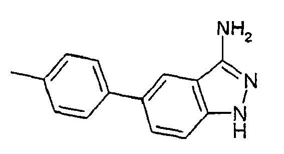 | 224 | 6 |
| 39 | 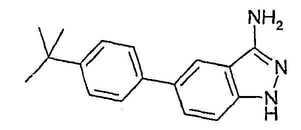 | 266 | 6 |
| 40 | 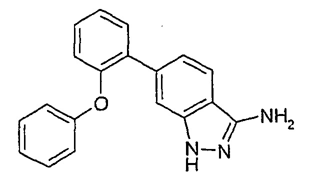 | 302 | 5 |
| 41 | 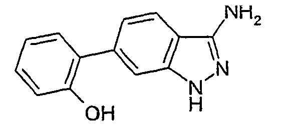 | 226 | 5 |
| 42 | 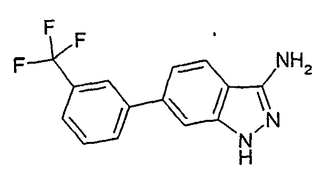 | 278 | 5 |
| 43 | 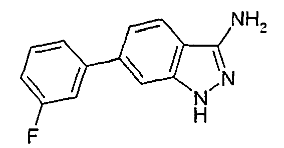 | 228 | 5 |
| 44 | 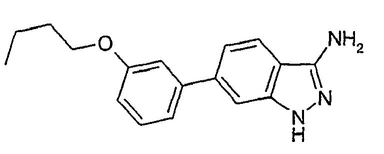 | 282 | 5 |
| 45 | 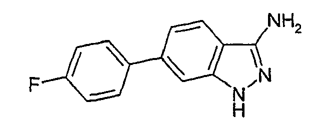 | 228 | 5 |
| 46 | 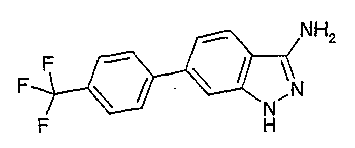 | 278 | 5 |
| 47 | 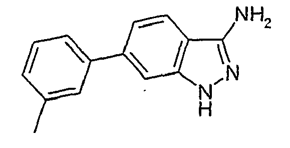 | 224 | 5 |
| 48 | 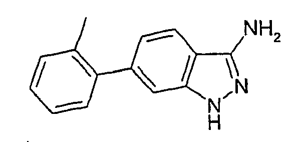 | 224 | 5 |
| 49 | 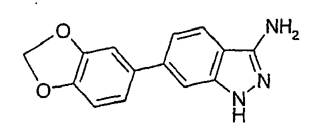 | 254 | 5 |
| 50 | 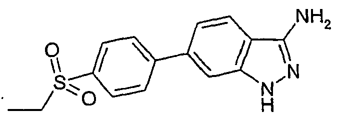 | 278 | 5 |
| 51 | 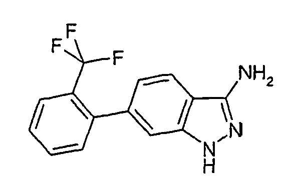 | 278 | 5 |
| 52 | 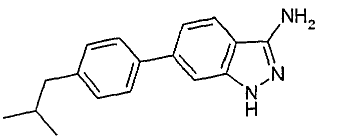 | 266 | 5 |
| 53 | 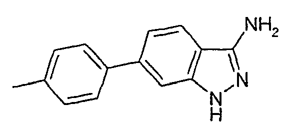 | 224 | 5 |
| 54 | 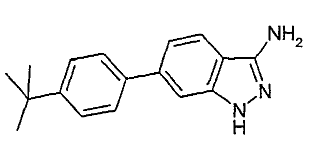 | 266 | 5 |
| 55 | 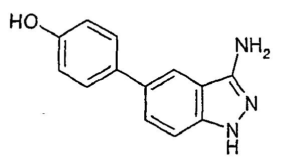 | 226 | 6 |
| 56 | 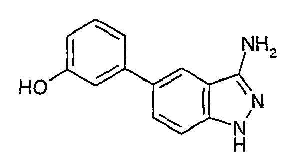 | 226 | 6 |
| 57 | 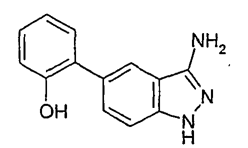 | 226 | 6 |
| 58 | 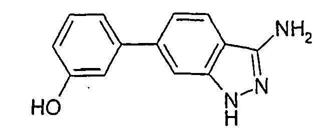 | 226 | 5 |
| 59 | 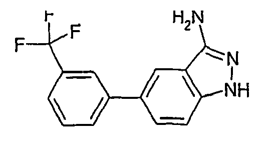 | 278 | 6 |
| 60 | 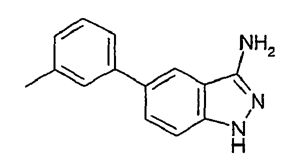 | 224 | 6 |
| 61 | 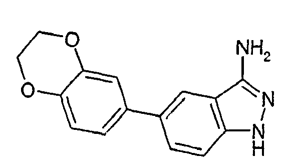 | 268 | 6 |
| 62 | 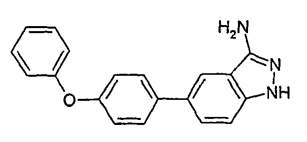 | 302 | 6 |
| 63 | 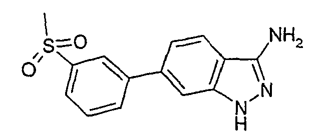 | 287 | 5 |
| 64 | 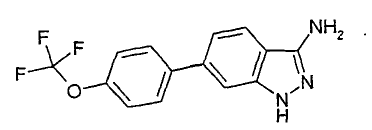 | 294 | 5 |
| 65 | 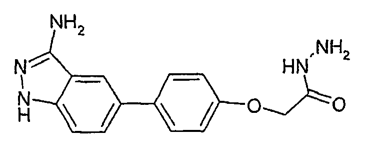 | 298 | 6 |
| 66 | 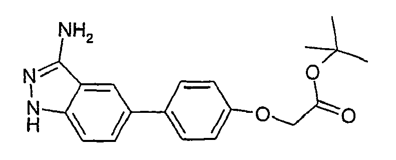 | 340 | 6 |
| 67 | 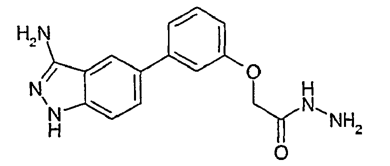 | 298 | 6 |
| 68 | 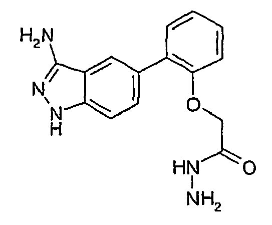 | 298 | 6 |
| 69 | 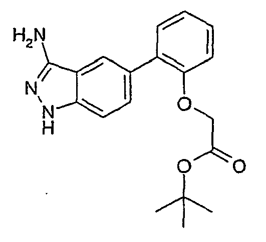 | 340 | 6 |
| 70 | 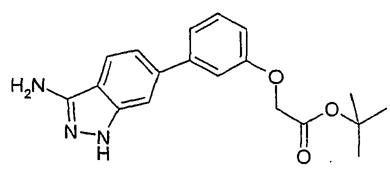 | 340 | 5 |
| 71 | 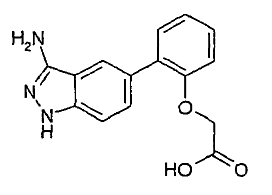 | 284 | 2 |
| 72 | 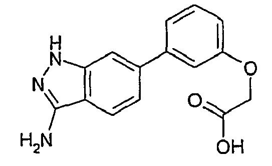 | 284 | 5 |
| 73 | 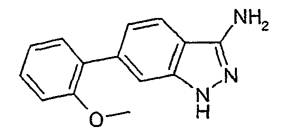 | 240 | 5 |
| 74 | 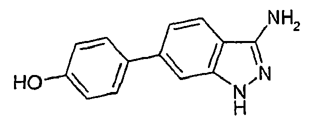 | 226 | 5 |
| 75 | 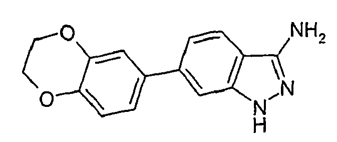 | 268 | 5 |
| 76 | 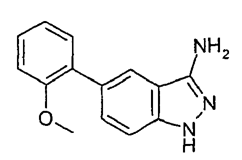 | 240 | 6 |
| 77 | 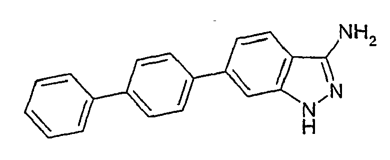 | 286 | 5 |
| 78 | 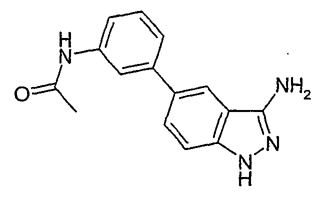 | 267 | 6 |
| 79 | 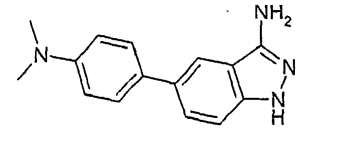 | 253 | 6 |
| 80 | 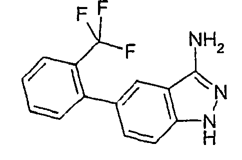 | 278 | 6 |
| 81 | 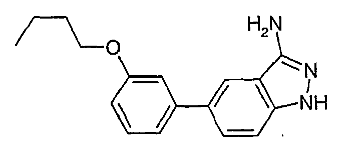 | 282 | 6 |
| 82 | 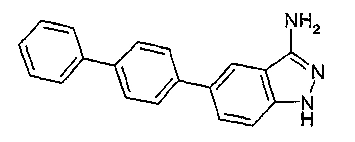 | 286 | 6 |
| 83 | 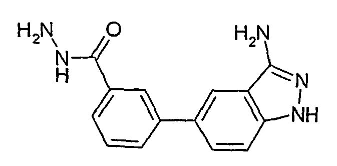 | 268 | 6 |
| 84 | 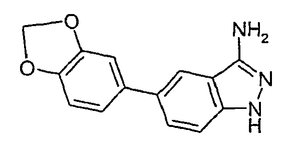 | 254 | 6 |
| 85 | 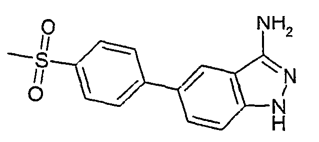 | 288 | 6 |
| 86 | 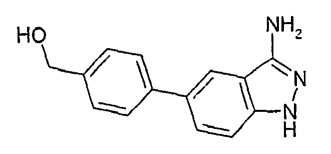 | 240 | 6 |
| 87 | 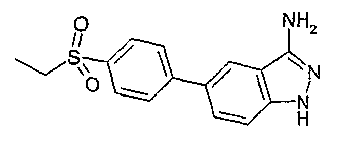 | 302 | 6 |
| 88 | 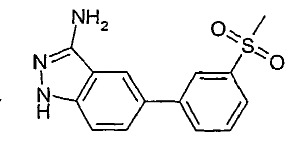 | 288 | 6 |
| | 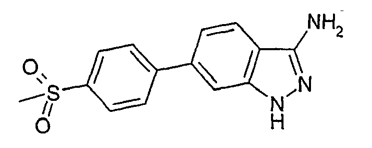 | 288 | 5 |
| 90 | 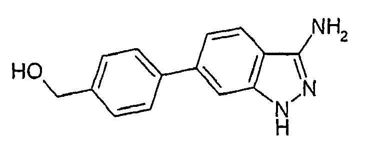 | 240 | 5 |
| 91 | 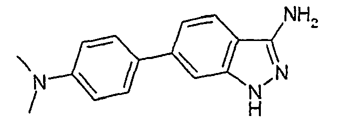 | 253 | 5 |
| 92 | 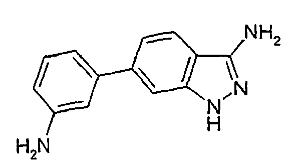 | 225 | 5 |
| 93 | 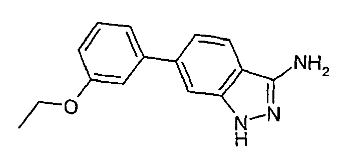 | 254 | 5 |
| 94 | 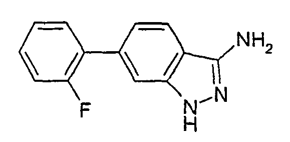 | 228 | 5 |
| 95 | 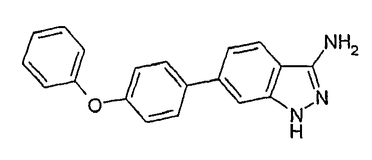 | 302 | 5 |
| 96 | 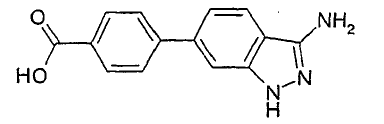 | 254 | 5 |
| 97 | 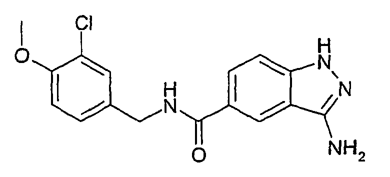 | 331 | 1 |
| 98 | 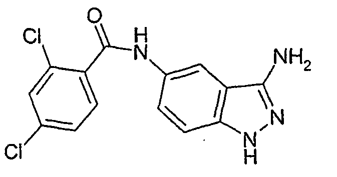 | 321 | 3 |
| 99 | 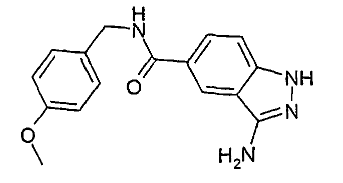 | 297 | 1 |
| 100 | 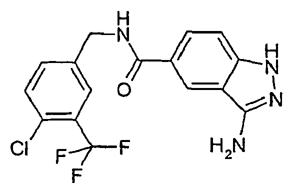 | 369 | 1 |
| 101 | 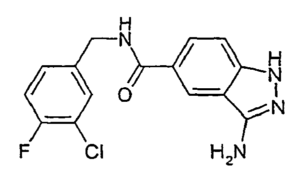 | 319 | 1 |
| 102 | 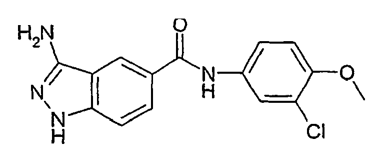 | 317 | 1 |
| 103 | 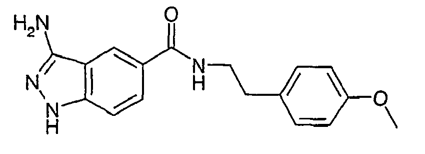 | 311 | 1 |
| 104 | 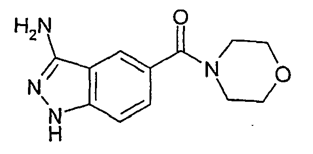 | 247 | 1 |
| 105 | 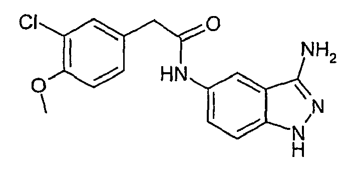 | 331 | 3 |
| 106 | 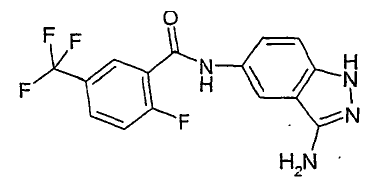 | 339 | 3 |
| 107 | 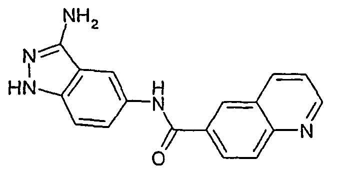 | 304 | 3 |
| 108 | 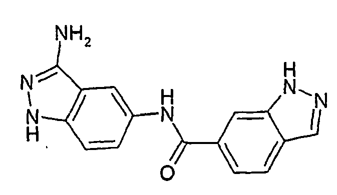 | 293 | 3 |
| 109 | 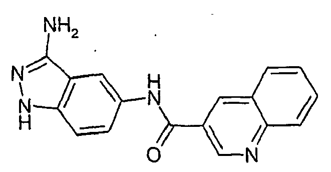 | 304 | 3 |
| 110 | 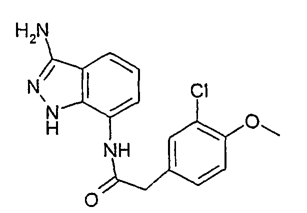 | 331 | 3 |
| 111 | 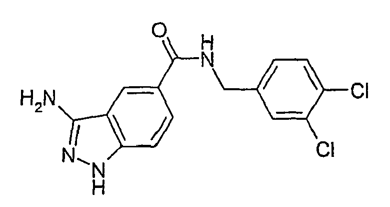 | 335 | 1 |
| 112 | 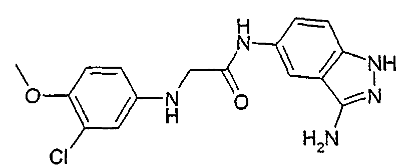 | 346 | 3 |
| 113 | 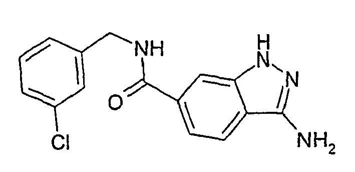 | 301 | 4.1 |
| 114 | 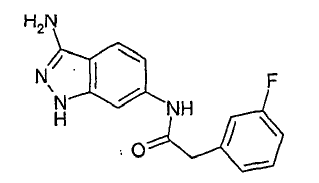 | 285 | 3 |
| 115 | 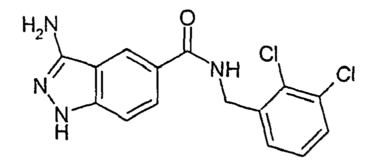 | 336 | 1 |
| 116 | 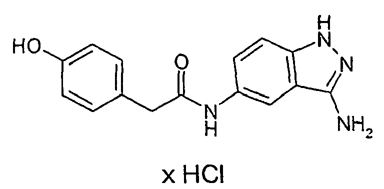 | 283 | 3 |
| 117 | 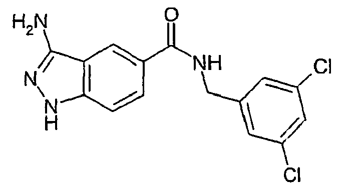 | 335 | 1 |
| 118 | 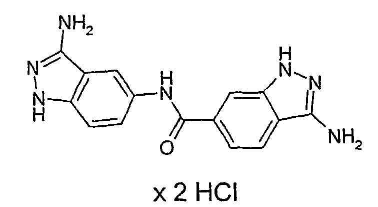 | 308 | 1 |
| 119 | 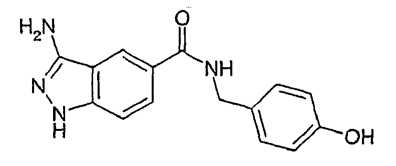 | 283 | 1 |
| 120 | 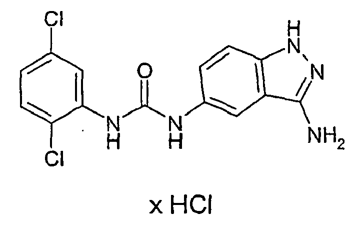 | 336 | 6.3 |
| 121 | 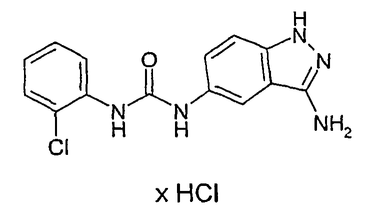 | 302 | 6.3 |
| 122 | 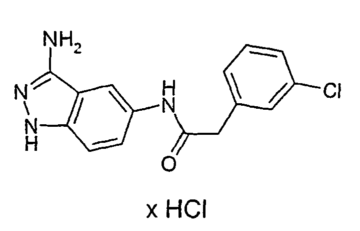 | 301 | 3 |
| 123 | 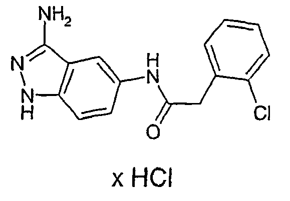 | 301 | 3 |
| 124 | 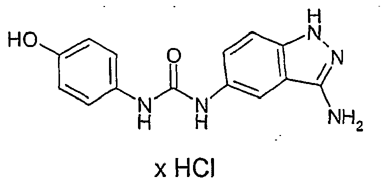 | 284 | 6.3 anschließend H₂/Pd |
| 125 | 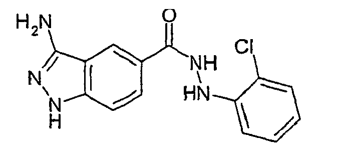 | 302 | 1 |
| 126 | 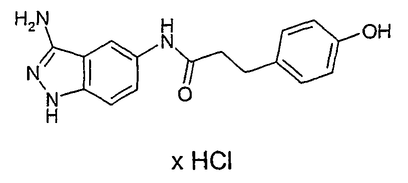 | 297 | 3 |
| 127 | 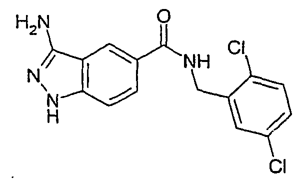 | 335 | 1 |
| 128 | 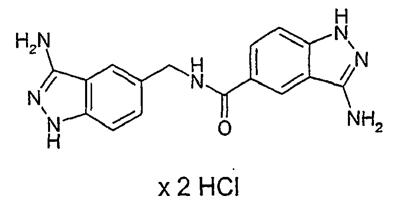 | 322 | 3.1 |
| 129 | 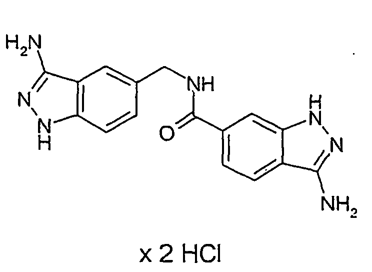 | 322 | 3.1 |
| 130 | 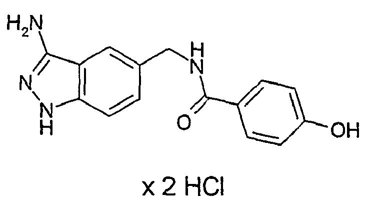 | 283 | 3.1 |
| 131 | 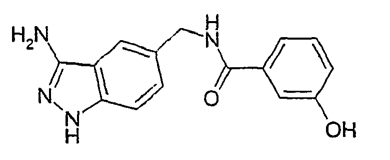 | 283 | 3.1 |
| 132 | 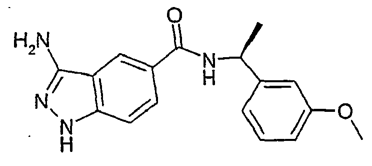 | 311 | 1 |
| 133 | 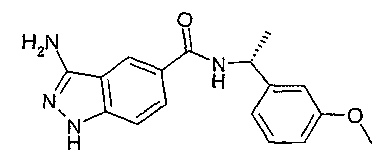 | 311 | 1 |
| 134 | 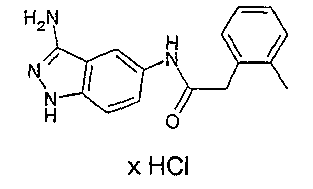 | 281 | 3 |
| 135 | 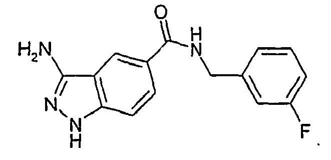 | 285 | 1 |
| 136 | 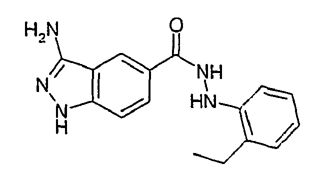 | 296 | 1 |
| 137 | 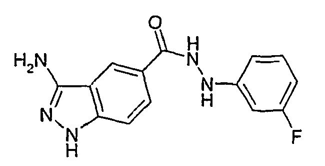 | 286 | 1 |
| 138 | 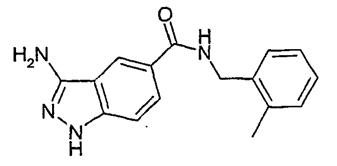 | 281 | 1 |
| 139 | 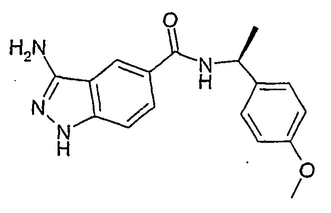 | 311 | 1 |
| 140 | 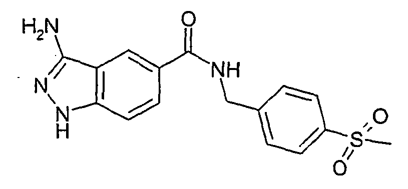 | 345 | 1 |
| 141 | 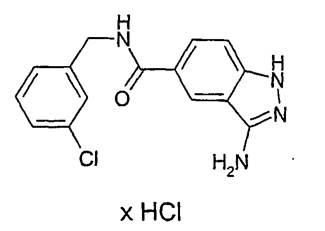 | 301 | 1 |
| 142 | 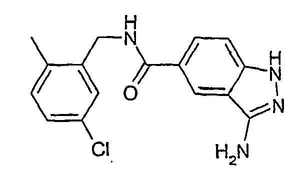 | 315 | 1 |
| 143 | 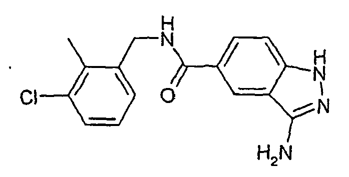 | 315 | 1 |
| 144 | 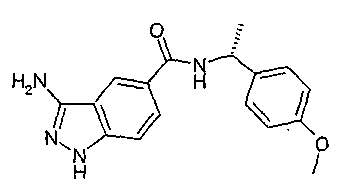 | 311 | 1 |
| 145 | 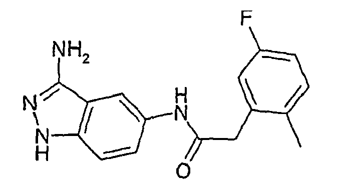 | 299 | 3 |
| 146 | 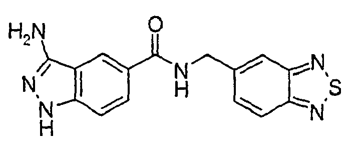 | 325 | 1 |
| 147 | 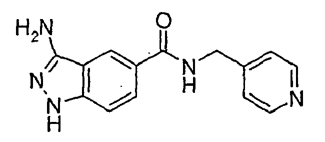 | 268 | 1 |
| 148 | 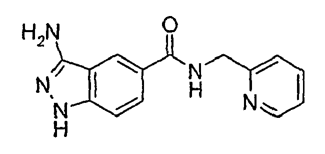 | 268 | 1 |
| 149 | 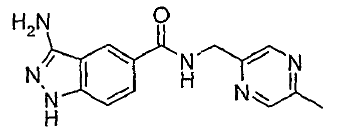 | 283 | 1 |
| 150 | 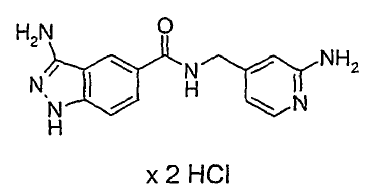 | 283 | Aminkomponente BOC-geschützt; 1; dann HCI/Dioxan |
| 151 | 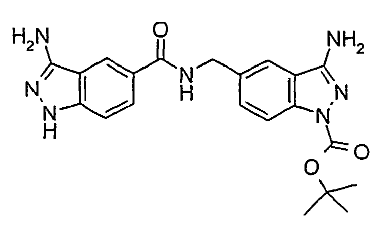 | 422 | 1 |
| 152 | 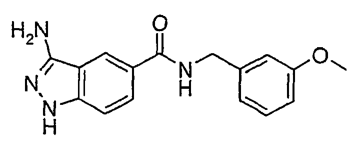 | 297 | 1 |
| 153 | 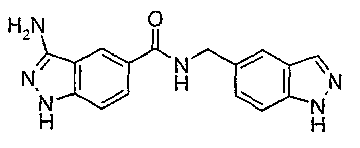 | | |
| 154 | 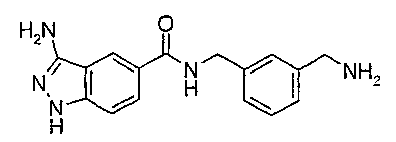 | | |
| 155 | 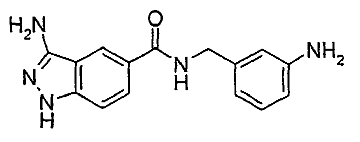 | | |
| 156 | 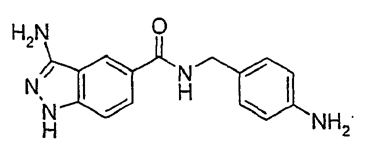 | | |
| 157 | 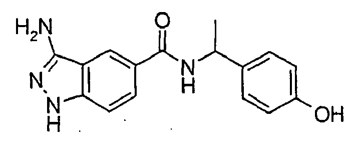 | | |
| 158 | 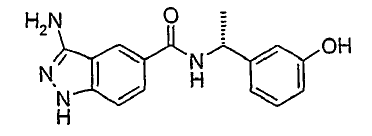 | | aus "133" mit BBr₃ |
| 159 | 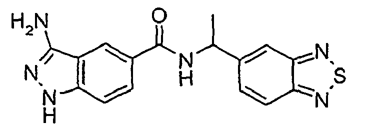 | | |
| 160 | 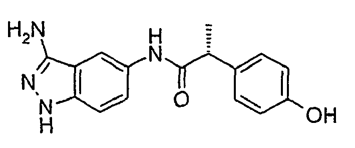 | | aus "144" mit BBr₃ |
| 161 | 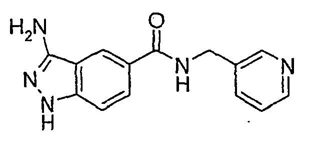 | | |
| 171 | 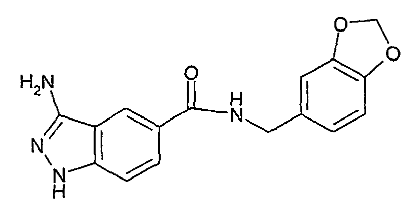 | 311 | 1 |
| 172 | 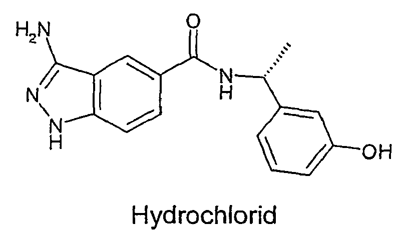 | 297 | 1 |
| 173 | 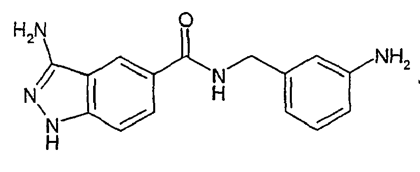 | 282 | 1 |
| 174 | 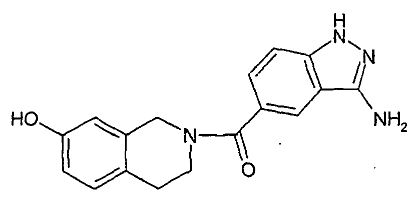 | 309 | 1 |
| 175 | 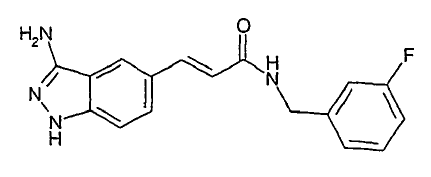 | 311 | 7 |
| 176 | 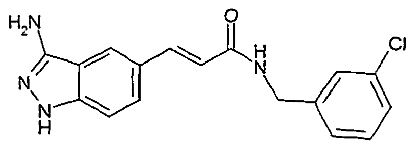 | 327 | 7 |
| 177 | 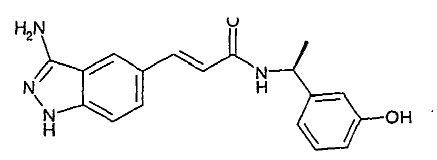 | 323 | 7 |
| 178 | 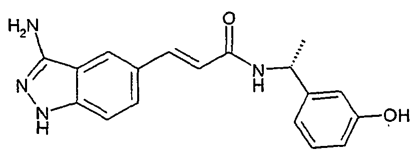 | 323 | 7 |
| 179 | 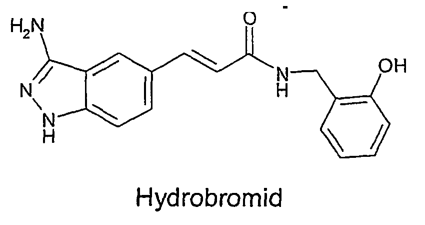 | 309 | Aus "181" durch Umsetzung mit BBr₃ |
| 180 | 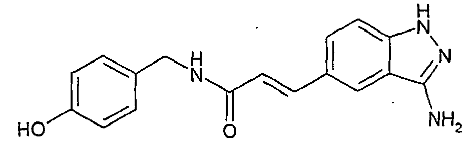 | 309 | 7 |
| 181 | 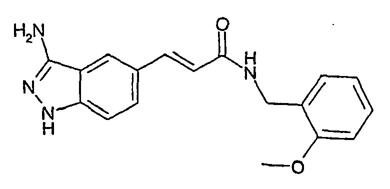 | 323 | 7 |
| 182 | 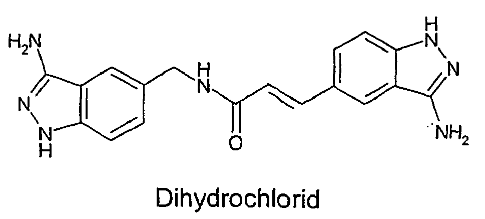 | 348 | 1. analog Bsp. 7 (Amin-komponente BOC-geschützt) 2. HCl/Dioxan |
| 183 | 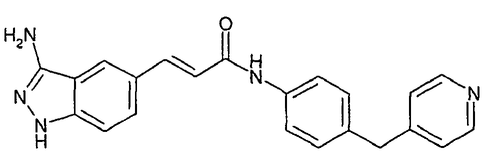 | 370 | 7 |
| 184 | 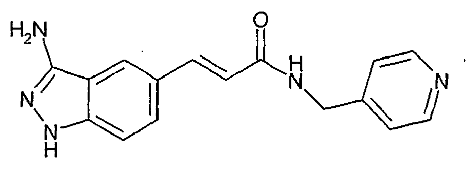 | 294 | 7 |
| 185 | 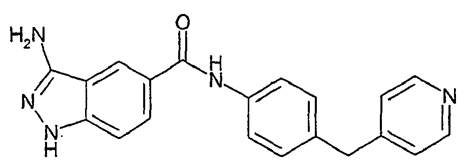 | 344 | 1 |
| 186 | 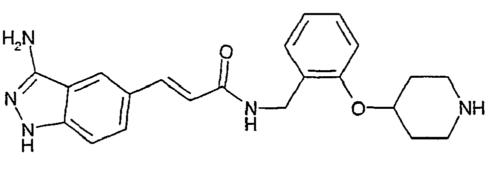 | 392 | 1. analog Bsp. 7 (Amin-komponente BOC-geschützt) 2. HCl/Dioxan |
| 187 | 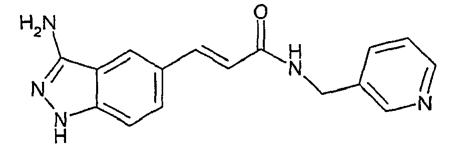 | 294 | 7 |
| 188 | 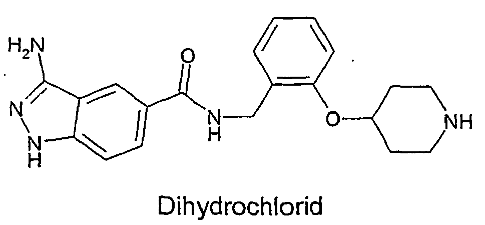 | 366 | 1. analog Bsp. 1 (Amin-komponente BOC-geschützt) 2. HCl/Dioxan |
| 189 | 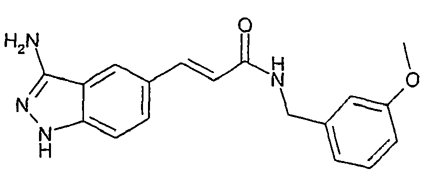 | 323 | 7 |
| 190 | 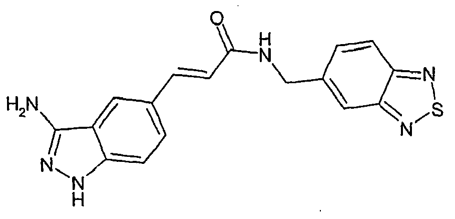 | 351 | 7 |
| 191 | 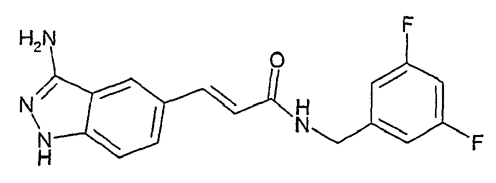 | 329 | 7 |
| 192 | 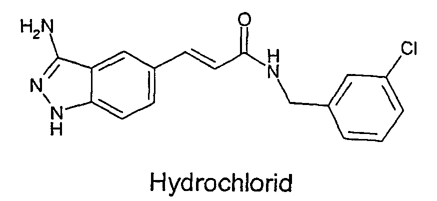 | 327 | 7 |
| 193 | 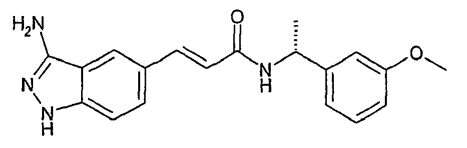 | 337 | 7 |
| 194 | 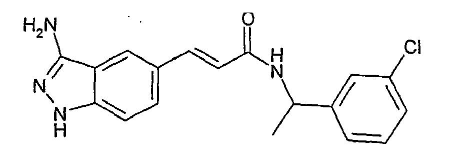 | 341 | 7 |
| 195 | 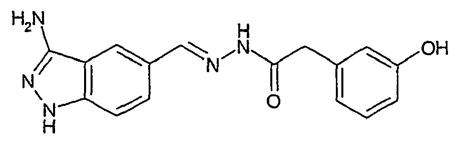 | 310 | 10 |
| 196 | 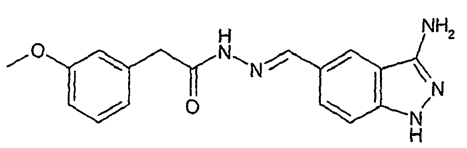 | 324 | 10 |
| 197 | 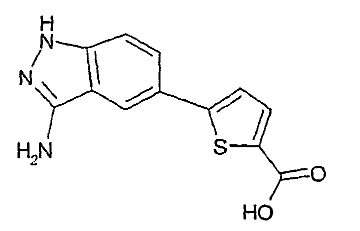 | 260 | 12 |
| 198 | 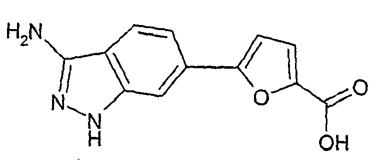 | 244 | 12 |
| 199 | 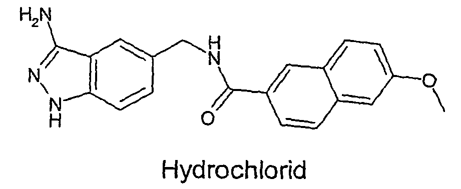 | 347 | 3.1 |
| 200 | 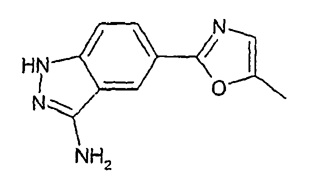 | 215 | 15 |
| 201 | 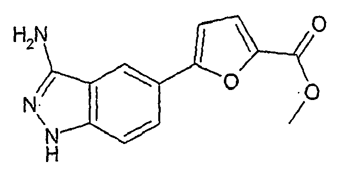 | 258 | Aus "167" mit Methanol /Chlortrimethylsilan |

Die nachfolgenden Beispiele betreffen pharmazeutische Zubereitungen:

### Beispiel A: Injektionsgläser

Eine Lösung von 100 g eines erfindungsgemäßen Wirkstoffes und 5 g Dinatriumhydrogenphosphat wird in 3 l zweifach destilliertem Wasser mit 2 n Salzsäure auf pH 6,5 eingestellt, steril filtriert, in Injektionsgläser abgefüllt, unter sterilen Bedingungen lyophilisiert und steril verschlossen. Jedes Injektionsglas enthält 5 mg Wirkstoff.

### Beispiel B: Suppositorien

Man schmilzt ein Gemisch von 20 g eines erfindungsgemäßen Wirkstoffes mit 100 g Sojalecithin und 1400 g Kakaobutter, gießt in Formen und läßt erkalten. Jedes Suppositorium enthält 20 mg Wirkstoff.

### Beispiel C: Lösung

Man bereitet eine Lösung aus 1 g eines erfindungsgemäßen Wirkstoffes, 9,38 g NaH₂PO₄ · 2 H₂O, 28,48 g Na₂HPO₄ · 12 H₂O und 0,1 g Benzalkoniumchlorid in 940 ml zweifach destilliertem Wasser. Man stellt auf pH 6,8 ein, füllt auf 1 l auf und sterilisiert durch Bestrahlung. Diese Lösung kann in Form von Augentropfen verwendet werden.

### Beispiel D: Salbe

Man mischt 500 mg eines erfindungsgemäßen Wirkstoffes mit 99,5 g Vaseline unter aseptischen Bedingungen.

### Beispiel E: Tabletten

Ein Gemisch von 1 kg Wirkstoff, 4 kg Lactose, 1,2 kg Kartoffelstärke, 0,2 kg Talk und 0,1 kg Magnesiumstearat wird in üblicher Weise zu Tabletten verpreßt, derart, daß jede Tablette 10 mg Wirkstoff enthält.

### Beispiel F: Dragees

Analog Beispiel E werden Tabletten gepreßt, die anschließend in üblicher Weise mit einem Überzug aus Saccharose, Kartoffelstärke, Talk, Tragant und Farbstoff überzogen werden.

### Beispiel G: Kapseln

2 kg Wirkstoff werden in üblicher Weise in Hartgelatinekapseln gefüllt, so daß jede Kapsel 20 mg des Wirkstoffs enthält.

### Beispiel H: Ampullen

Eine Lösung von 1 kg eines erfindungsgemäßen Wirkstoffes in 60 I zweifach destilliertem Wasser wird steril filtriert, in Ampullen abgefüllt, unter sterilen Bedingungen lyophilisiert und steril verschlossen. Jede Ampulle enthält 10 mg Wirkstoff.

## Patentansprüche

1. Verbindungen der Formel I 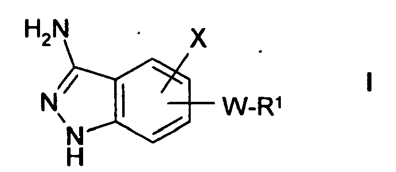 worin
W -[C(R³)₂]ₙ-, -(C(R³)₂]ₙCONH(C(R³)₂]ₙ-, -[C(R³)₂]ₙNR³CO[C(R³)₂]ₙNR³-, -[C(R³)₂]ₙO[C(R³)₂]ₙ-, -[C(R³)₂]ₙNR³[C(R³)₂]ₙ-, -[C(R³)₂]ₙN[C(R³)₂R¹][C(R³)₂]ₙ-, -[C(R³)₂]ₙNR³C(R³)₂]ₙCONR³[C(R³)₂]ₙ-,-(C(R³)₂]ₙCO[C(R³)₂]ₙ-, -[C(R³)₂]ₙCONHNH[C(R³)₂]ₙ-, -(C(R³)₂]ₙNHCO(C(R³)₂)ₙ]-, -C(R³)=C(R³)CONR³(C(R³)₂]ₙ oder -C(R³)=N-NR³CO[C(R³)₂]ₙ,
X H,
R¹ Phenyl oder Naphthyl, das ein-, zwei- oder dreifach durch Hal, A, -(CH₂)ₙOH, OA, Phenyl, Phenoxy, Benzyloxy, Pyridin-4-ylmethyl-, Piperidin-4-oxy-, NO₂, CN, -(CH₂)ₙCOOR³, O-(CH₂)ₙCOOR³, SO₂A, NHCOA, (CH₂)ₙNH₂, (CH₂)ₙNHA, (CH₂)ₙNA₂, -(C(R³)₂]ₙCONH₂, (CH₂)ₙCONHNH₂ oder O-(CH₂)ₙCONHNH₂ substituiert sein kann,
oder
einen unsubstituierten oder ein- oder zweifach durch NH₂, A, OH, CH₂OH, COOH und/oder COOA substituierten ein- oder zweikernigen gesättigten oder ungesättigten Heterocyclus mit 1 bis 4 N-, O- und/oder S-Atomen,
R³ H oder A,
A unverzweigtes oder verzweigtes Alkyl mit 1-6 C-Atomen, worin 1-7 H-Atome durch F ersetzt sein können,
Hal F, Cl, Br oder I,
n 0, 1 oder 2,
bedeuten,
sowie ihre pharmazeutisch verwendbaren Salze, E,Z-Isomere, Diastereomere, Enantiomere, Mono-, Dihydrate und Alkoholate, einschließlich deren Mischungen in allen Verhältnissen.

2. Verbindungen nach Anspruch 1 ausgewählt aus der Gruppe
3-Amino-1*H*-indazol-5-carbonsäure-3-chlorbenzylamid ("1"),
[3-(3-Amino-1*H*-indazol-5-yl)-phenoxy]-essigsäure ("2"),
N-(3-Amino-1*H*-indazol-5-yl)-2-(3-hydroxy-phenyl)-acetamid ("3"),
N-(3-Amino-1*H*-indazol-5-ylmethyl)-3-chlorbenzamid ("3.1"),
N-(3-Amino-1*H*-indazo)-6-yl)-2-(3-chlor-4-methoxyphenyl)-acetamid ("4"),
3-Amino-1*H*-indazol-6-carbonsäure-3-chlorbenzylamid ("4.1"),
6-(4-Phenoxy-phenyl)-1*H*-indazol-3-ylamin ("5"),
5-(3-Methansulfonyl-phenyl)-1*H*-indazol-3-ylamin ("6"),
6-(3-Chlor-4-methoxy-benzyloxy)-1*H*-indazol-3-ylamin ("6.1 "),
N5-(3-Chlor-4-methoxy-benzyl)-1*H*-indazol-3,5-diamin("6.2"),
N5,N5-Bis-(3-chlor-4-methoxy-benzyl)-1*H*-indazol-3,5-diamin ("6.2a"),
1-(3-Amino-1*H*-indazol-5-yl)-3-(4-benzyloxy-phenyl)-harnstoff ("6.3"),
1-(3-Amino-1*H*-indazol-5-yl)-3-(3-chlor-phenyl)-harnstoff ("6.3a"),
| | |
|---|---|
| 7 | 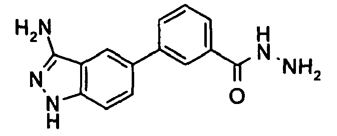 |
| 8 | 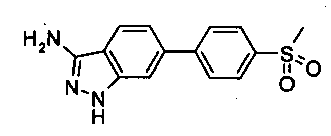 |
| 9 | 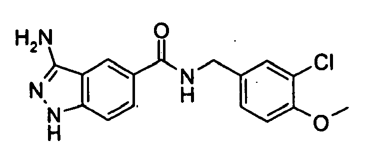 |
| 10 | 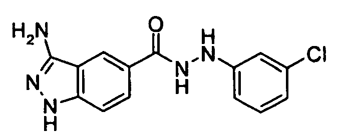 |
| 11 | 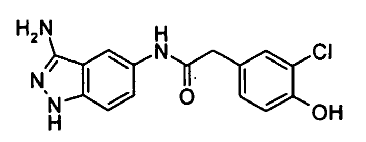 |
| 12 | 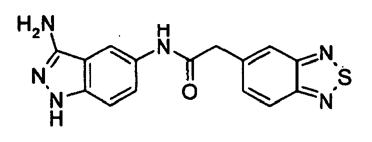 |
| 13 | 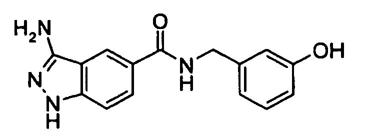 |
| 14 | 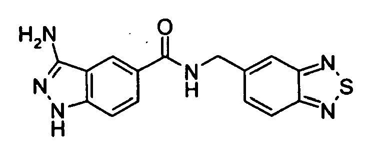 |
| 15 | 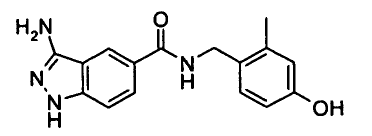 |
| 16 | 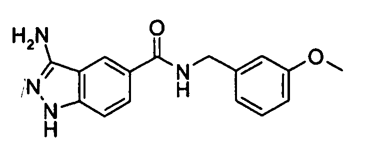 |
| 17 | 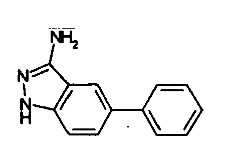 |
| 18 | 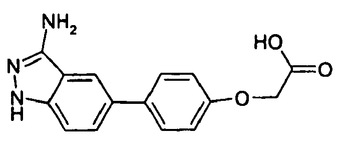 |
| 19 | 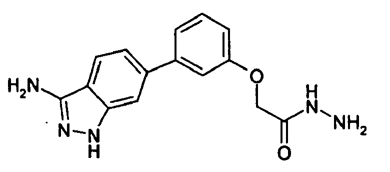 |
| 20 | 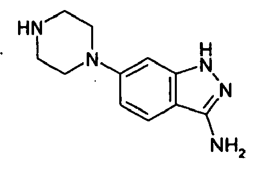 |
| 21 | 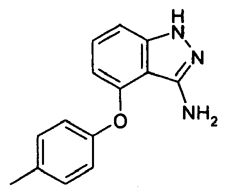 |
| 22 | 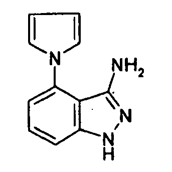 |
| 23 | 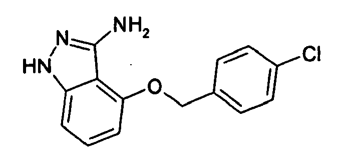 |
| 24 | 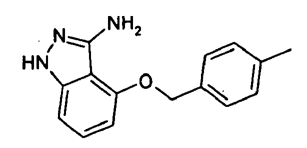 |
| 25 | 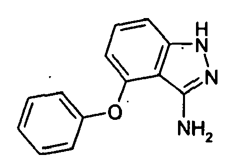 |
| 26 | 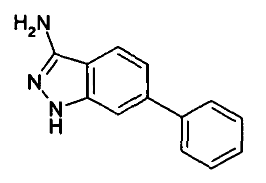 |
| 27 | 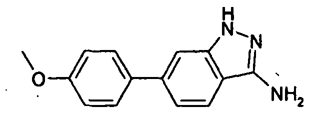 |
| 28 | 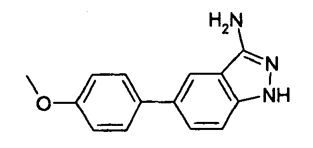 |
| 29 | 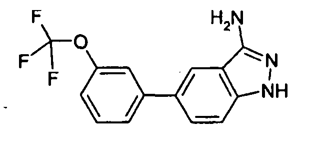 |
| 30 | 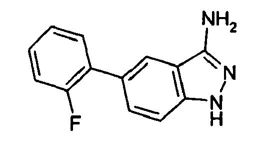 |
| 31 | 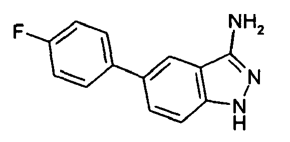 |
| 32 | 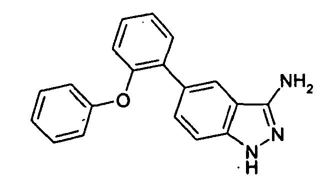 |
| 33 | 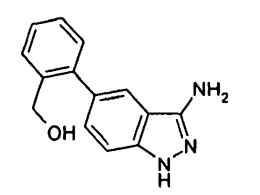 |
| 34 | 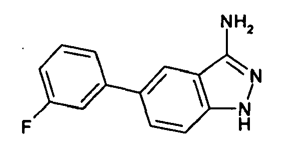 |
| 35 | 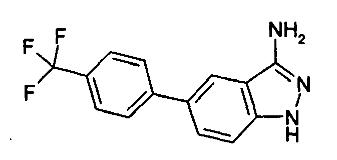 |
| 36 | 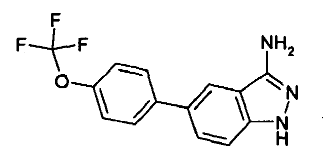 |
| 37 | 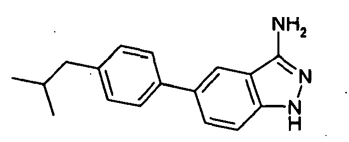 |
| 38 | 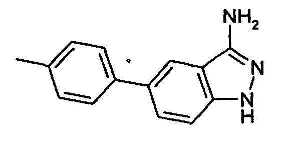 |
| 39 | 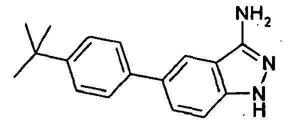 |
| 40 | 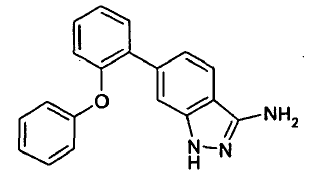 |
| 41 | 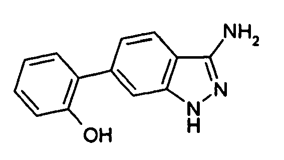 |
| 42 | 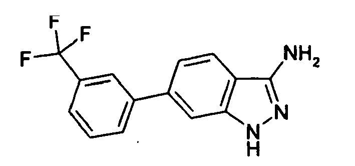 |
| 43 | 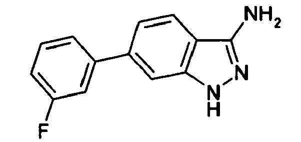 |
| 44 | 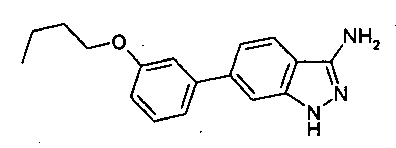 |
| 45 | 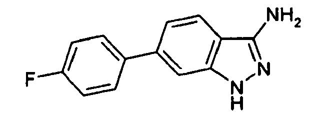 |
| 46 | 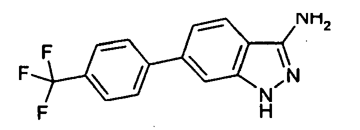 |
| 47 | 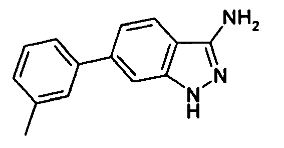 |
| 48 | 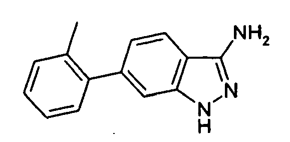 |
| 49 | 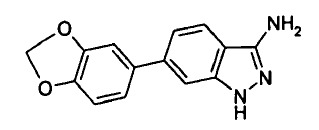 |
| 50 | 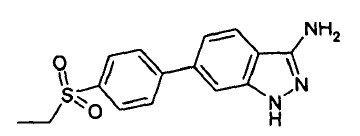 |
| 51 | 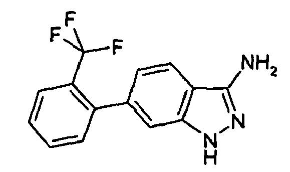 |
| 52 | 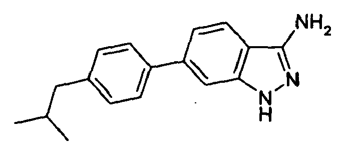 |
| 53 | 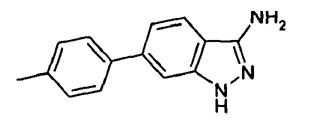 |
| 54 | 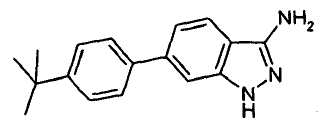 |
| 55 | 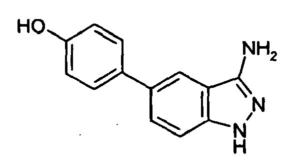 |
| 56 | 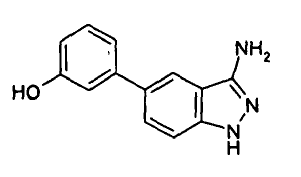 |
| 57 | 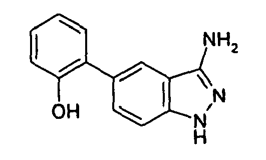 |
| 58 | 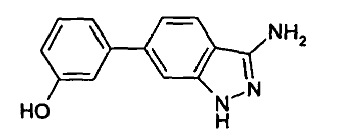 |
| 59 | 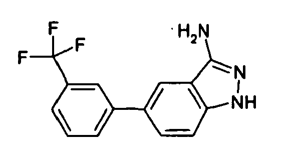 |
| 60 | 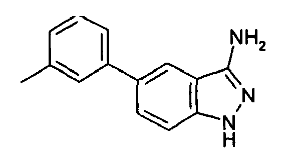 |
| 61 | 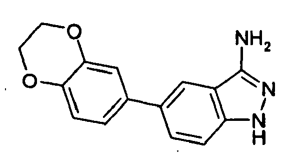 |
| 62 | 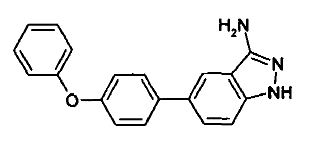 |
| 63 | 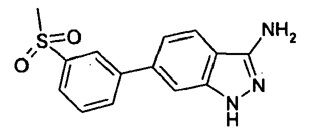 |
| 64 | 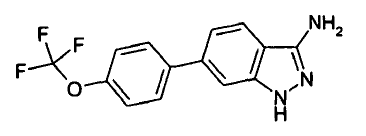 |
| 65 | 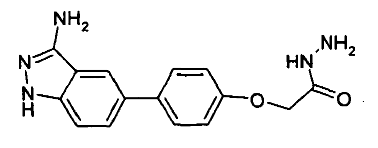 |
| 66 | 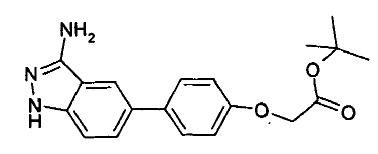 |
| 67 | 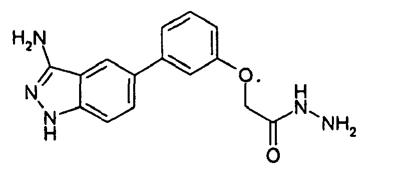 |
| 68 | 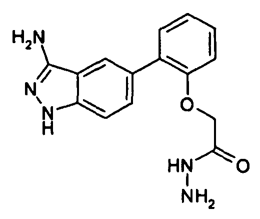 |
| 69 | 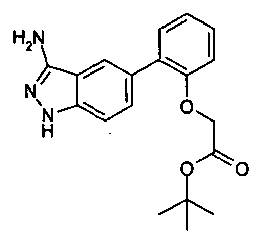 |
| 70 | 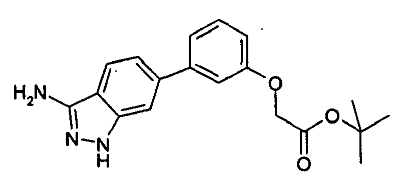 |
| 71 | 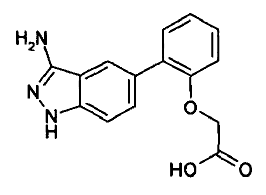 |
| 72 | 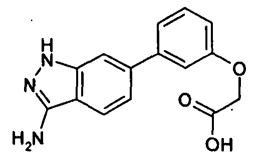 |
| 73 | 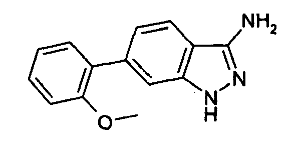 |
| 74 | 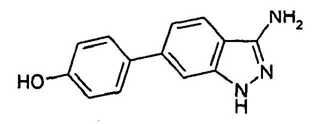 |
| 75 | 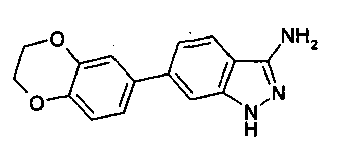 |
| 76 | 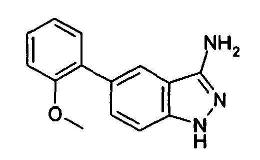 |
| 77 | 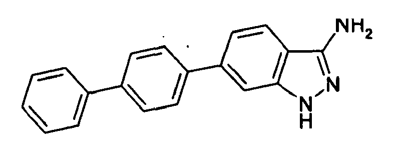 |
| 78 | 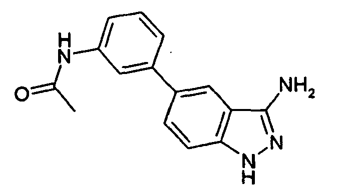 |
| 79 | 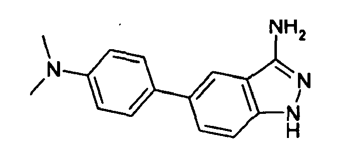 |
| 80 | 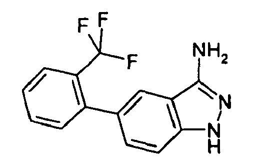 |
| 81 | 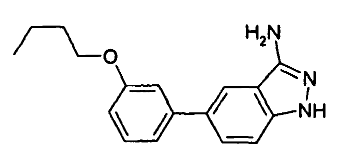 |
| 82 | 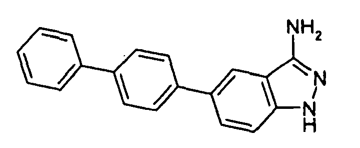 |
| 83 | 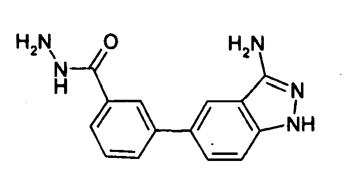 |
| 84 | 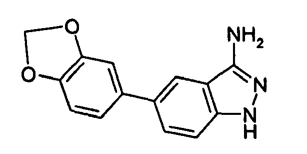 |
| 85 | 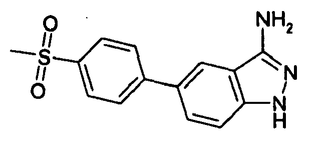 |
| 86 | 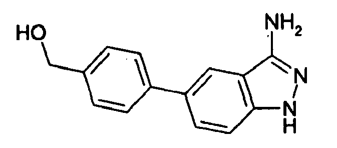 |
| 87 | 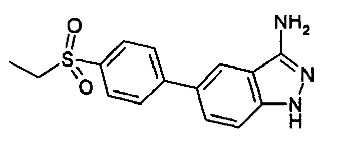 |
| 88 | 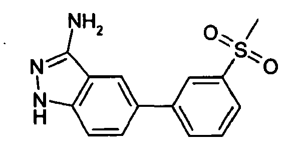 |
| 89 | 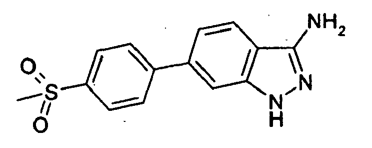 |
| 90 | 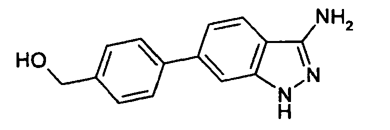 |
| 91 | 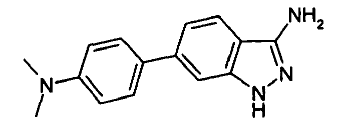 |
| 92 | 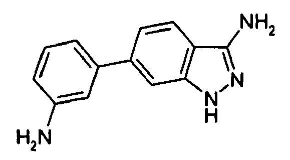 |
| 93 | 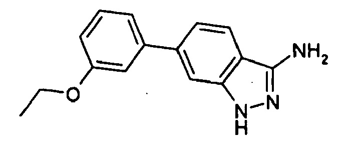 |
| 94 | 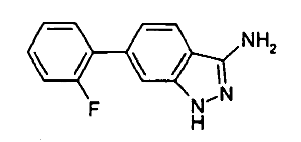 |
| 95 | 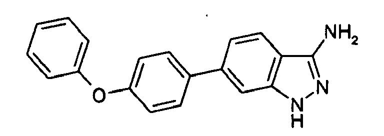 |
| 96 | 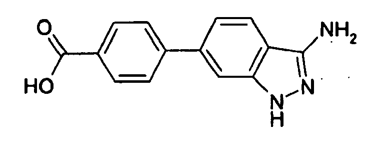 |
| 97 | 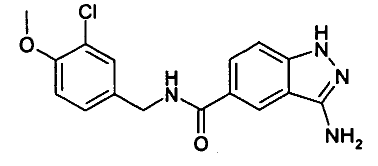 |
| 98 | 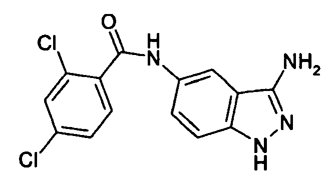 |
| 99 | 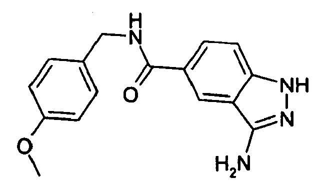 |
| 100 | 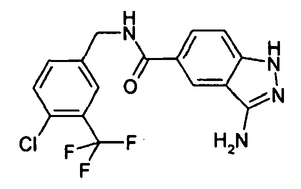 |
| 101 | 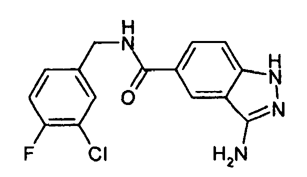 |
| 102 | 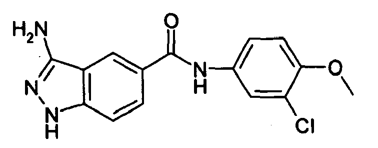 |
| 103 | 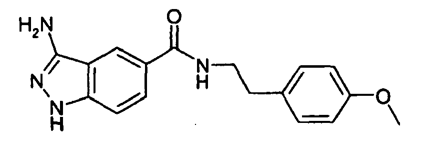 |
| 104 | 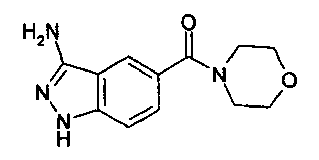 |
| 105 | 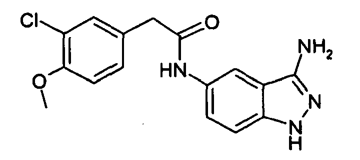 |
| 106 | 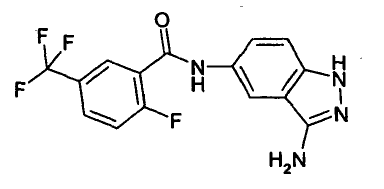 |
| 107 | 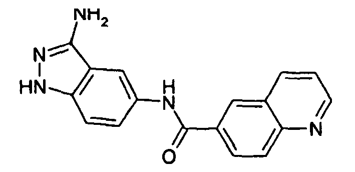 |
| 108 | 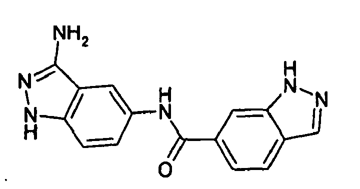 |
| 109 | 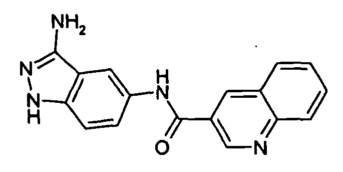 |
| 110 | 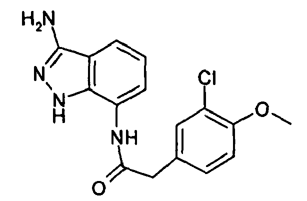 |
| 111 | 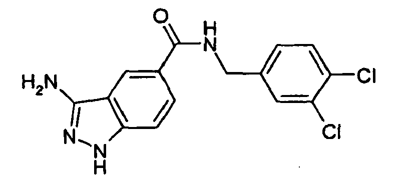 |
| 112 | 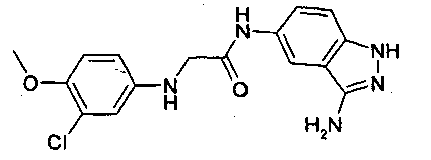 |
| 113 | 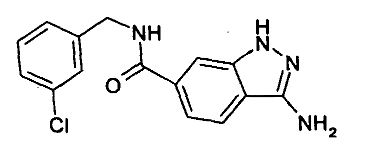 |
| 114 | 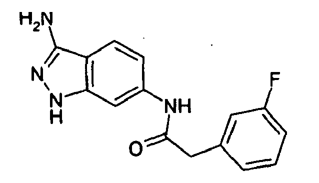 |
| 115 | 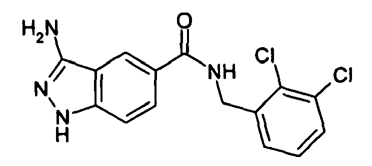 |
| 116 | 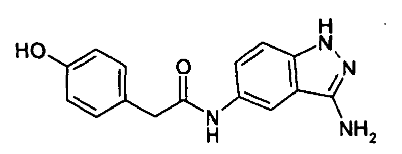 |
| 117 | 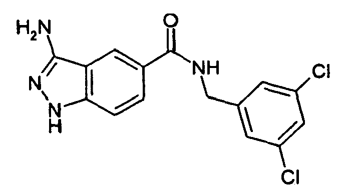 |
| 118 | 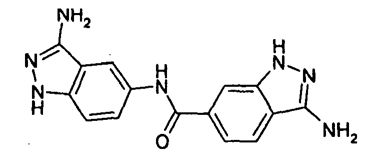 |
| 119 | 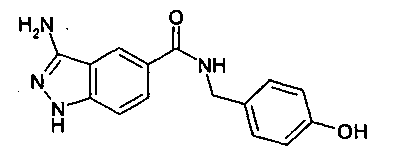 |
| 120 | 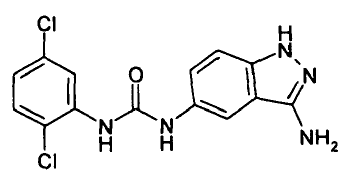 |
| 121 | 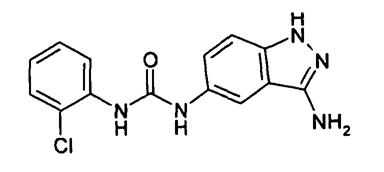 |
| 122 | 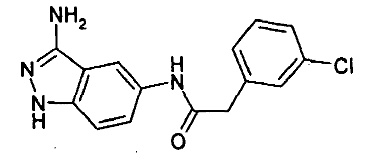 |
| 123 | 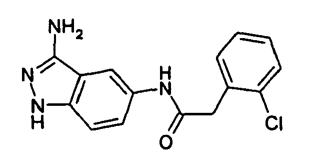 |
| 124 | 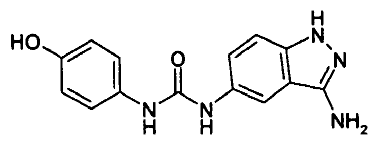 |
| 125 | 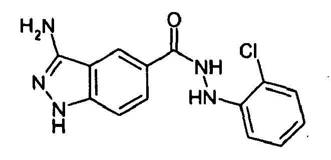 |
| 126 | 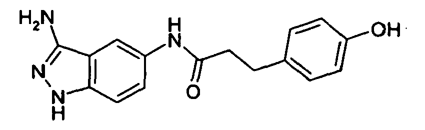 |
| 127 | 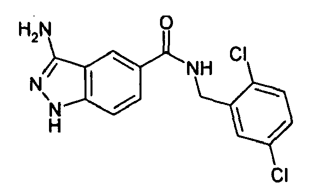 |
| 128 | 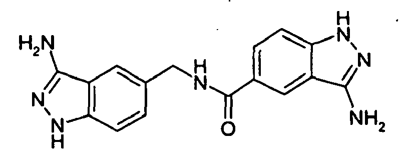 |
| 129 | 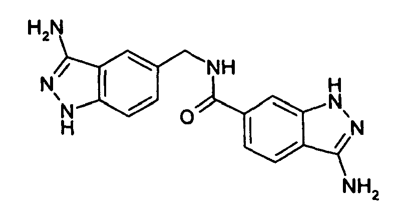 |
| 130 | 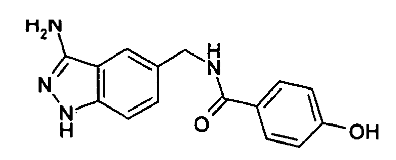 |
| 131 | 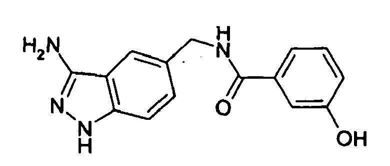 |
| 132 | 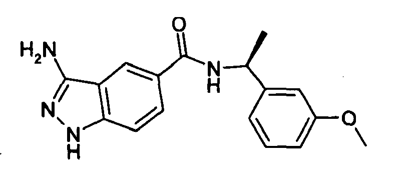 |
| 133 | 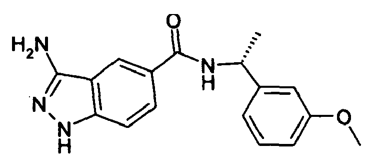 |
| 134 | 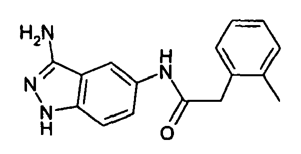 |
| 135 | 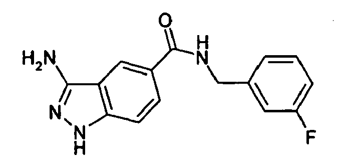 |
| 136 | 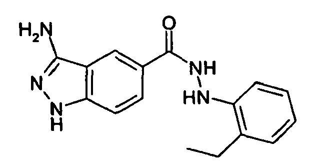 |
| 137 | 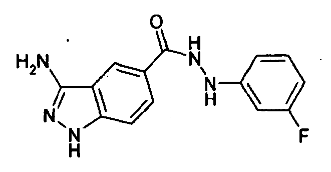 |
| 138 | 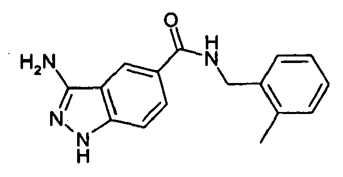 |
| 139 | 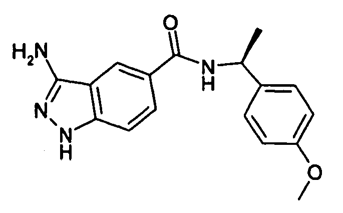 |
| 140 | 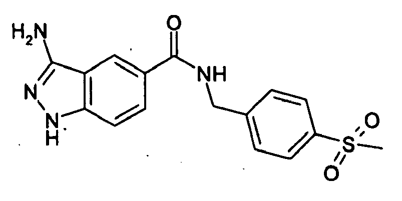 |
| 141 | 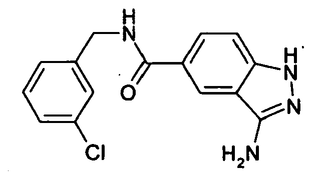 |
| 142 | 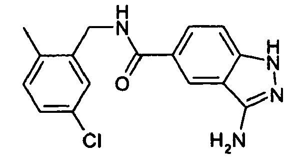 |
| 143 | 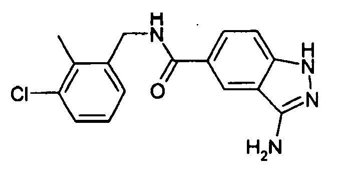 |
| 144 | 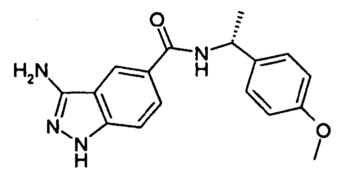 |
| 145 | 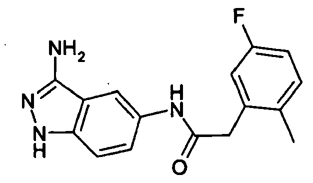 |
| 146 | 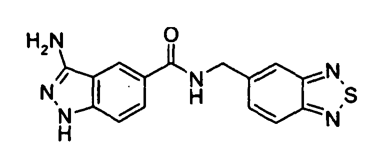 |
| 147 | 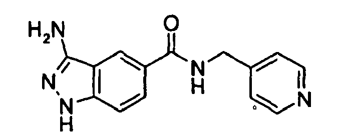 |
| 148 | 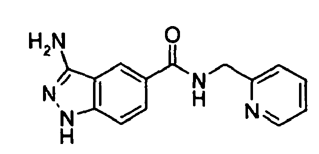 |
| 149 | 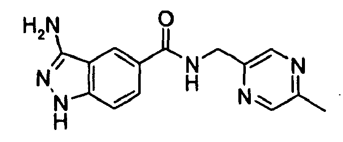 |
| 150 | 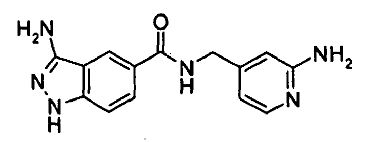 |
| 151 | 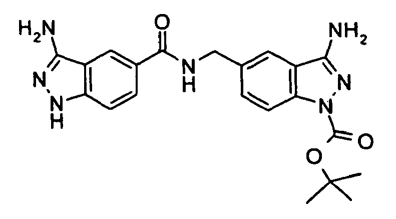 |
| 152 | 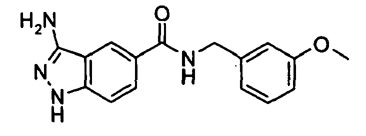 |
| 153 | 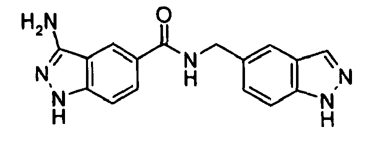 |
| 154 | 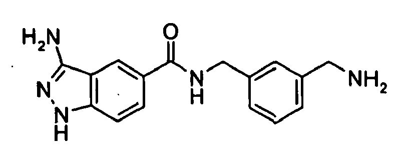 |
| 155 | 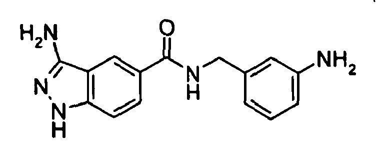 |
| 156 | 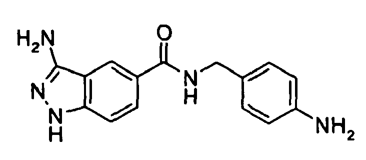 |
| 157 | 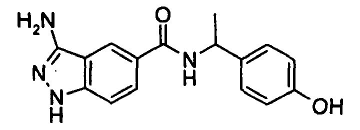 |
| 158 | 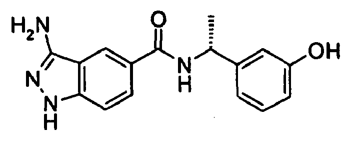 |
| 159 | 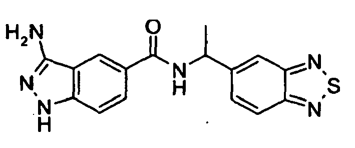 |
| 160 | 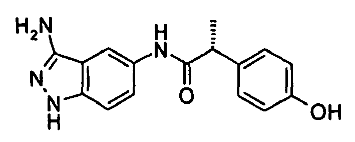 |
| 161 | 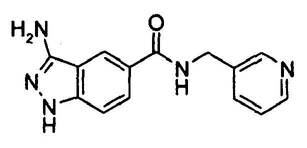 |
| 162 | 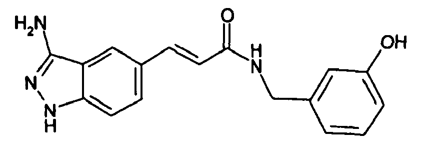 |
| 163 | 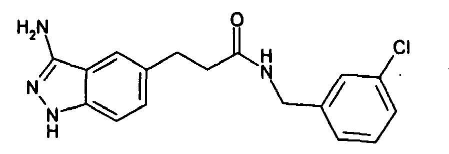 |
| 164 | 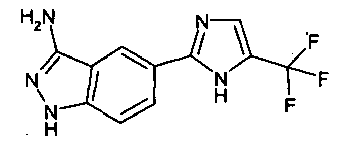 |
| 165 | 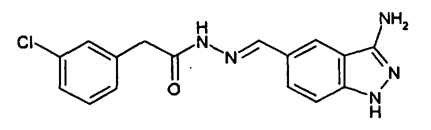 |
| 166 | 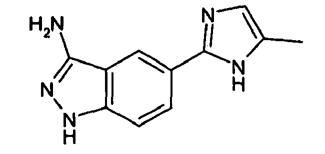 |
| 167 | 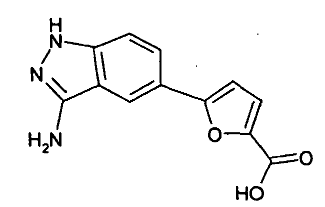 |
| 168 | 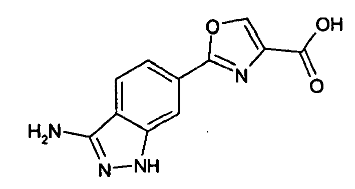 |
| 169 | 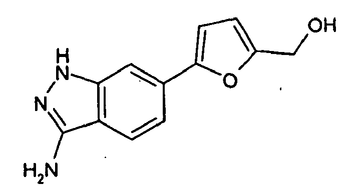 |
| 170 | 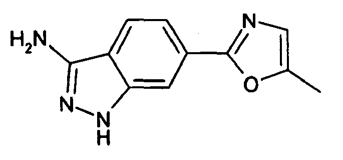 |
| 171 | 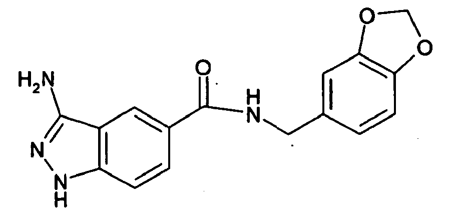 |
| 172 | 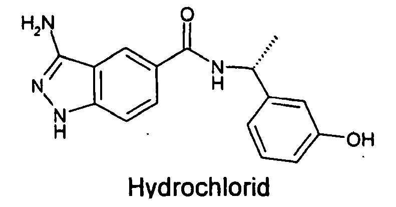 |
| 173 | 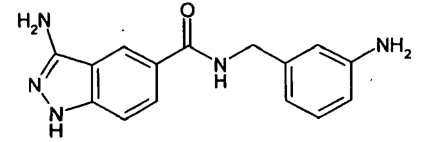 |
| 174 | 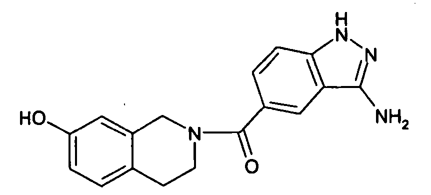 |
| 175 | 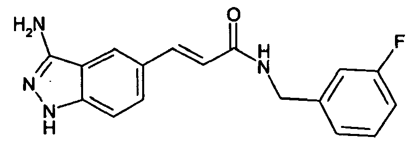 |
| 176 | 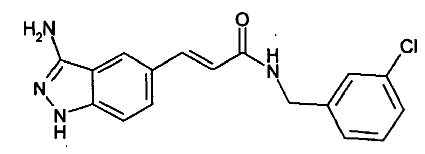 |
| 177 | 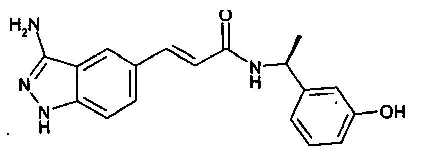 |
| 178 | 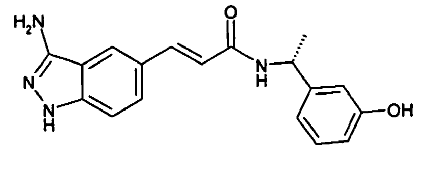 |
| 179 | 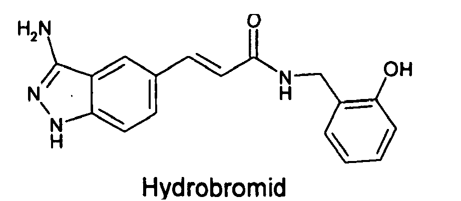 |
| 180 | 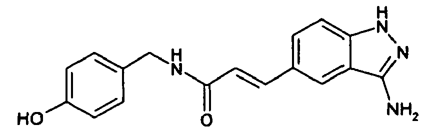 |
| 181 | 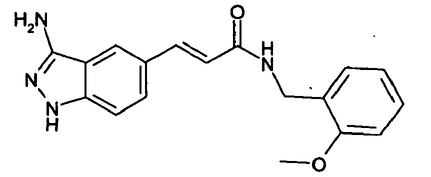 |
| 182 | 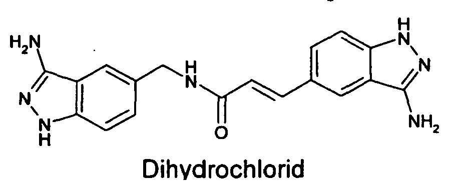 |
| 183 | 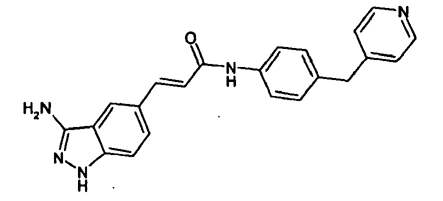 |
| 184 | 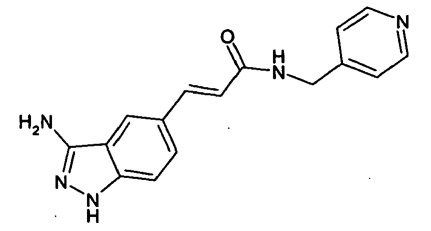 |
| 185 | 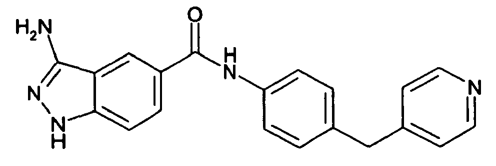 |
| 186 | 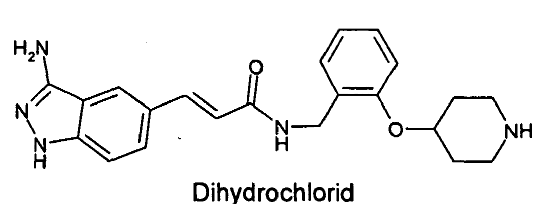 |
| 187 | 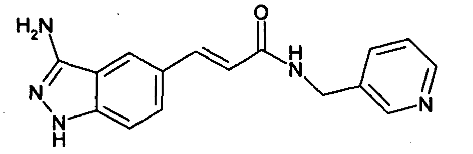 |
| 188 | 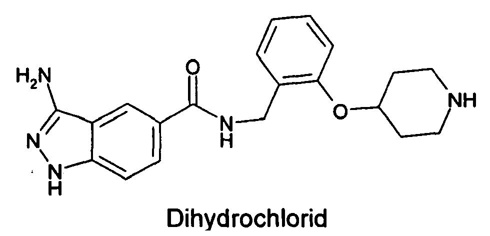 |
| 189 | 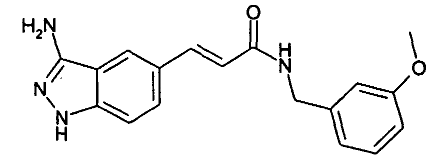 |
| 190 | 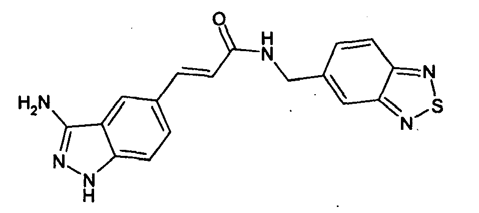 |
| 191 | 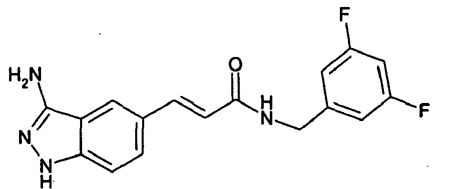 |
| 192 | 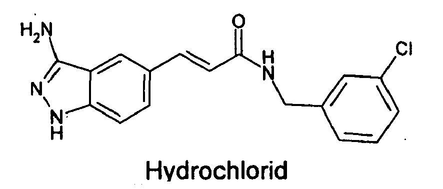 |
| 193 | 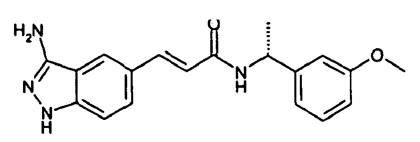 |
| 194 | 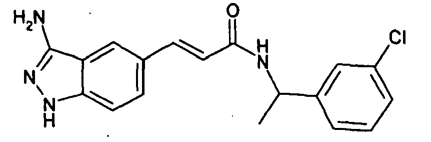 |
| 195 | 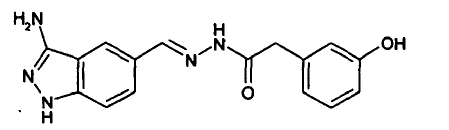 |
| 196 | 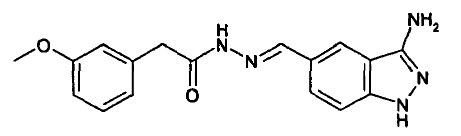 |
| 197 | 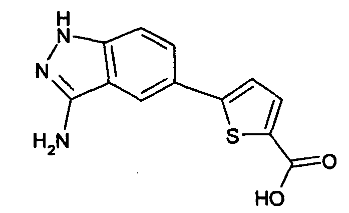 |
| 198 | 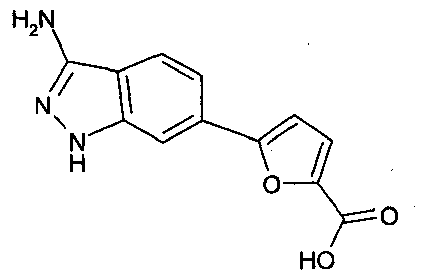 |
| 199 | 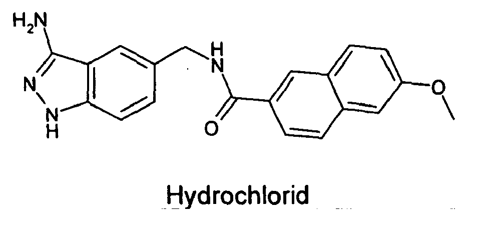 |
| 200 | 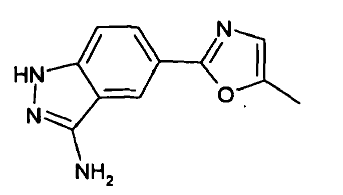 |
| 201 | 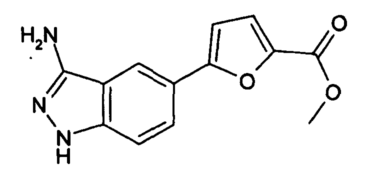 |
sowie ihre pharmazeutisch verwendbaren Salze, E,Z-Isomere, Diastereomere, Enantiomere, Mono-, Dihydrate und Alkoholate, einschließlich deren Mischungen in allen Verhältnissen.

3. Verfahren zur Herstellung von Verbindungen der Formel I nach den Ansprüchen 1-2 sowie ihrer pharmazeutisch verwendbaren Salze, E,Z-Isomere, Diastereomere, Enantiomere, Mono-, Dihydrate und Alkoholate, **dadurch gekennzeichnet, daß** man
a) zur Herstellung von Verbindungen der Formel I, worin
W
i) -[C(R³)₂]ₙCONH[C(R³)₂]ₙ- oder
ii) -[C(R³)₂]ₙCONHNH[C(R³)₂]ₙ-
bedeutet,
eine Verbindung der Formel II 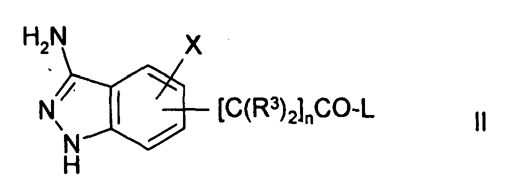 worin
X, R³ und n die in Anspruch 1 angegebene Bedeutung haben, und L Cl, Br, I oder eine freie oder reaktionsfähig funktionell abgewandelte OH-Gruppe bedeutet,
mit
i) einer Verbindung der Formel IIIa
H₂N[C(R³)₂]ₙ-R¹ IIIa
worin
R¹, R³ und n die in Anspruch 1 angegebenen Bedeutungen haben,
oder
ii) einer Verbindung der Formel IIIb
H₂N-NH[C(R³)₂]ₙ-R¹ IIIb
worin
R¹, R³ und n die in Anspruch 1 angegebenen Bedeutungen haben,
umsetzt,
oder
b) zur Herstellung von Verbindungen der Formel I, worin
W -[C(R³)₂]ₙNHCO[C(R³)₂]ₙ- bedeutet,
eine Verbindung der Formel IV 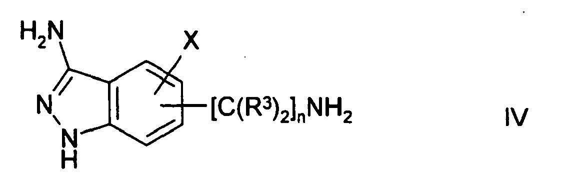 worin X, R³ und n die in Anspruch 1 angegebenen Bedeutungen
haben,
mit einer Verbindung der Formel V
L-CO-[C(R³)₂]ₙ-R¹ V
worin
L Cl, Br, I oder eine freie oder reaktionsfähig funktionell abgewandelte OH-Gruppe bedeutet und
R¹, R³ und n die in Anspruch 1 angegebenen Bedeutungen
haben,
umsetzt,
oder
c) zur Herstellung von Verbindungen der Formel I, worin
W -[C(R³)₂]ₙ-,-[C(R³)₂]ₙO[C(R³)₂]ₙ-, -[C(R³)₂]ₙNR³[C(R³)₂]ₙ- oder -[C(R³)₂]ₙNR¹[C(R³)₂]ₙ-
bedeutet,
eine Verbindung der Formel VI 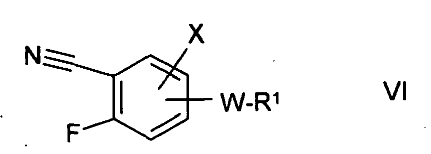 worin
R¹, W und X die in Anspruch 1 angegebenen Bedeutungen haben,
mit Hydrazin umsetzt,
oder
d) zur Herstellung von Verbindungen der Formel 1, worin
W -(C(R³)₂]ₙNHCONH(C(R³)₂]ₙ- bedeutet,
eine Verbindung der Formel IV mit einer Verbindung der Formel VII
O=C=N-[C(R³)₂]ₙ-R¹ VII
worin R¹, R³ und n die in Anspruch 1 angegebenen Bedeutungen
haben,
umsetzt,
oder
e) daß man sie aus einem ihrer funktionellen Derivate durch Behandeln mit einem solvolysierenden oder hydrogenolysierenden Mittel in Freiheit setzt,
und/oder
eine Base oder Säure der Formel I in eines ihrer Salze umwandelt.

4. Arzneimittel, enthaltend mindestens eine Verbindung nach Anspruch 1-2 und/oder ihre pharmazeutisch verwendbaren Salze, E,Z-Isomere, Diastereomere, Enantiomere, Mono-, Dihydrate und Alkoholate, einschließlich deren Mischungen in allen Verhältnissen, sowie gegebenenfalls Träger- und/oder Hilfsstoffe.

5. Verwendung von Verbindungen gemäß Anspruch 1-2, sowie ihrer pharmazeutisch verwendbaren Salze, E,Z-Isomere, Diastereomere, Enantiomere, Mono-, Dihydrate und Alkoholate, einschließlich deren Mischungen in allen Verhältnissen, zur Herstellung eines Arzneimittels zur Behandlung und/oder Prophylaxe von Krankheiten, bei denen die Hemmung, Regulierung und/oder Modulation der Signaltransduktion von Kinasen eine Rolle spielt.

6. Verwendung nach Anspruch 5, wobei es sich bei der Kinase um SGK handelt.

7. Verwendung nach Anspruch 6 von Verbindungen gemäß Anspruch 1-2, sowie ihrer pharmazeutisch verwendbaren Salze, E,Z-Isomere, Diastereomere, Enantiomere, Mono-, Dihydrate und Alkoholate, einschließlich deren Mischungen in allen Verhältnissen, zur Herstellung eines Arzneimittels zur Behandlung von Krankheiten, die durch Inhibierung der SGK durch die Verbindungen nach Anspruch 1-2 beeinflußt werden.

8. Verwendung nach Anspruch 7 von Verbindungen gemäß Anspruch 1-2, sowie ihrer pharmazeutisch verwendbaren Salze, E,Z-Isomere, Diastereomere, Enantiomere, Mono-, Dihydrate und Alkoholate, einschließlich deren Mischungen in allen Verhältnissen, zur Herstellung eines Arzneimittels zur Behandlung oder Vorbeugung von Diabetes, Fettsucht, metabolischem Syndrom (Dyslipidämie), systemischer und pulmonaler Hypertonie, Herzkreislauferkrankungen und Nierenerkrankungen, allgemein bei jeglicher Art von Fibrosen und entzündlichen Prozessen, Krebs, Tumorzellen, Tumormetastasen, Koagulopathien, neuronaler Erregbarkeit, Glaukom, Katarakt, bakteriellen Infektionen sowie in einer antiinfektiösen Therapie, zur Steigerung der Lernfähigkeit und Aufmerksamkeit und zur Behandlung von Tinitus.

9. Verwendung nach Anspruch 8, wobei es sich bei Diabetes um Diabetes mellitus, diabetische Nephropathie, diabetische Neuropathie, diabetische Angiopathie und Mikroangiopathie handelt.

10. Verwendung nach Anspruch 8, wobei es sich bei Herzkreislauferkrankungen um kardiale Fibrosen nach Myokardinfarkt, Herzhypertrophie, Herzinsuffizienz und Arteriosklerose handelt.

11. Verwendung nach Anspruch 8, wobei es sich bei Nierenerkrankungen um Glomerulosklerose, Nephrosklerose, Nephritis, Nephropathie und Störung der Elektrolytausscheidung handelt.

12. Verwendung nach Anspruch 8, wobei es sich bei Fibrosen und entzündlichen Prozessen um Leberzirrhose, Lungenfibrose, fibrosierende Pankreatitis, Rheumatismus und Arthrosen, Morbus Crohn, chronische Bronchitis, Strahlenfibrose, Sklerodermitis, zystische Fibrose, Narbenbildung und Morbus Alzheimer handelt.

13. Arzneimittel enthaltend mindestens eine Verbindung gemäß Anspruch 1-2 und/oder ihre pharmazeutisch verwendbaren Salze, E,Z-Isomere, Diastereomere, Enantiomere, Mono-, Dihydrate und Alkoholate, einschließlich deren Mischungen in allen Verhältnissen, und mindestens einen weiteren Arzneimittelwirkstoff.

14. Set (Kit), bestehend aus getrennten Packungen von
(a) einer wirksamen Menge an einer Verbindung gemäß Anspruch 1-2 und/oder ihrer pharmazeutisch verwendbaren Salze, E,Z-Isomere, Diastereomere, Enantiomere, Mono-, Dihydrate und Alkoholate, einschließlich deren Mischungen in allen Verhältnissen, und
(b) einer wirksamen Menge eines weiteren Arzneimittelswirkstoffs.

15. Zwischenverbindungen ausgewählt aus der Gruppe 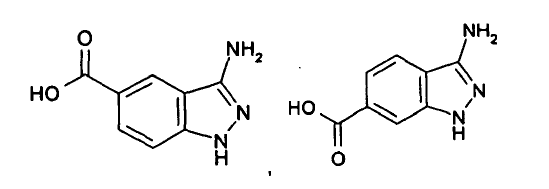 sowie deren Salze.

## Claims

1. Compounds of the formula I 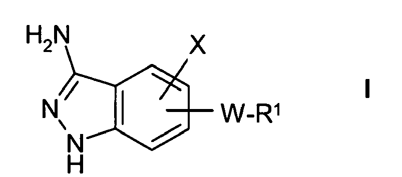 in which
W denotes -[C(R³)₂]ₙ-, -[C(R³)₂]ₙCONH[C(R³)₂]n-_{,} -[C(R³)₂]ₙNR³CO[C(R³)₂]ₙNR³-, -[C(R³)₂]ₙO[C(R³)₂]ₙ-, -[C(R³)₂]ₙNR³[C(R³)₂]ₙ-, -[C(R³)₂]ₙN[C(R³)₂R¹][C(R³)₂]ₙ-, -[C(R³)₂]ₙNR³C(R³)₂]ₙCONR³[C(R³)₂]ₙ-, -[C(R³)₂]ₙCO[C(R³)₂]ₙ-, -[C(R³)₂]ₙCONHNH[C(R³)₂]ₙ-, -[C(R³)₂]ₙNHCO[C(R³)₂]ₙ-, -C(R³)=C(R³)CONR³[C(R³)₂]ₙ or -C(R³)=N-NR³CO[C(R³)₂]ₙ,
X denotes H,
R¹ denotes phenyl or naphthyl, each of which may be mono-, di- or trisubstituted by Hal, A, -(CH₂)ₙOH, OA, phenyl, phenoxy, benzyloxy, pyridin-4-ylmethyl-, piperidin-4-oxy-, NO₂, CN, -(CH₂)ₙCOOR³, O-(CH₂)ₙCOOR³, SO₂A, NHCOA, (CH₂)ₙNH₂, (CH₂)ₙNHA, (CH₂)ₙNA₂, -[C(R³)₂]ₙCONH₂, (CH₂)ₙCONHNH₂ or O-(CH₂)ₙCONHNH₂,
or
a mono- or bicyclic saturated or unsaturated heterocycle having 1 to 4 N, O and/or S atoms, which is unsubstituted or mono- or disubstituted by NH₂, A, OH, CH₂OH, COOH and/or COOA,
R³ denotes H or A,
A denotes unbranched or branched alkyl having 1-6 C atoms, in which 1-7 H atoms may be replaced by F,
Hal denotes F, Cl, Br or I,
n denotes 0, 1 or 2,
and pharmaceutically usable salts, E,Z isomers, diastereomers, enantiomers, mono-, dihydrates and alcoholates thereof, including mixtures thereof in all ratios.

2. Compounds according to Claim 1 selected from the group
N-(3-chlorobenzyl)-3-amino-1*H*-indazole-5-carboxamide ("1 "),
[3-(3-amino-1*H*-indazol-5-yl)phenoxy]acetic acid ("2"),
N-(3-amino-1*H*-indazol-5-yl)-2-(3-hydroxyphenyl)acetamide ("3"),
N-(3-amino-1*H*-indazol-5-ylmethyl)-3-chlorobenzamide ("3.1 "),
N-(3-amino-1*H*-indazol-6-yl)-2-(3-chloro-4-methoxyphenyl)-acetamide ("4"),
N-3-chlorobenzyl-3-amino-1*H*-indazole-6-carboxamide ("4.1"),
6-(4-phenoxyphenyl)-1*H*-indazol-3-ylamine ("5"),
5-(3-methanesulfonylphenyl)-1*H*-indazol-3-ylamine ("6"),
6-(3-chloro-4-methoxybenzyloxy)-1*H*-indazol-3-ylamine ("6.1 "),
N5-(3-chloro-4-methoxybenzyl)-1*H*-indazole-3,5-diamine ("6.2"),
N5,N5-bis(3-chloro-4-methoxybenzyl)-1*H*-indazole-3,5-diamine ("6.2a"),
1-(3-amino-1*H*-indazol-5-yl)-3-(4-benzyloxyphenyl)urea ("6.3"),
1-(3-amino-1*H*-indazol-5-yl)-3-(3-chlorophenyl)urea ("6.3a"),
| | |
|---|---|
| 7 | 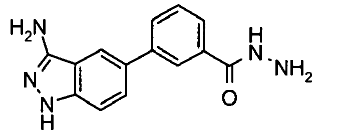 |
| 8 | 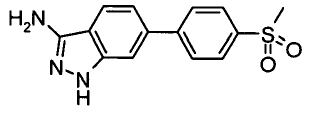 |
| 9 | 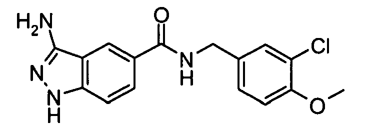 |
| 10 | 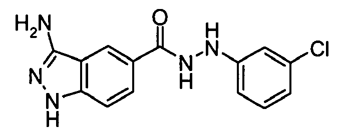 |
| 11 | 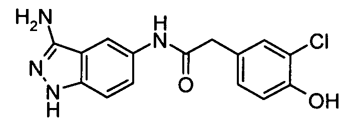 |
| 12 | 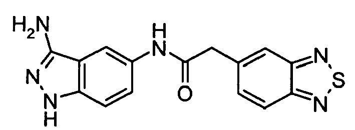 |
| 13 | 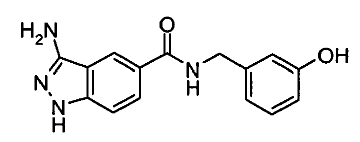 |
| 14 | 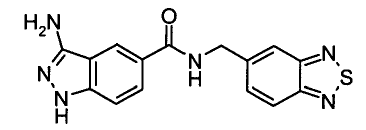 |
| 15 | 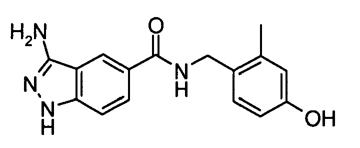 |
| 16 | 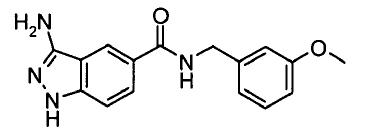 |
| 17 | 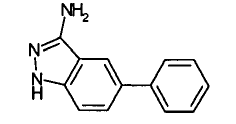 |
| 18 | 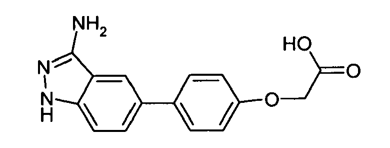 |
| 19 | 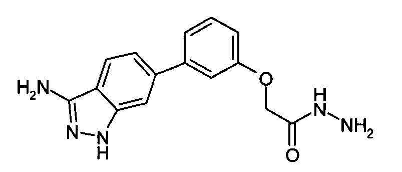 |
| 20 | 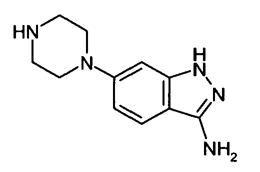 |
| 21 | 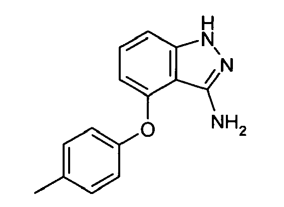 |
| 22 | 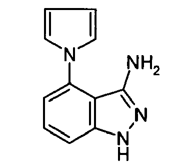 |
| 23 | 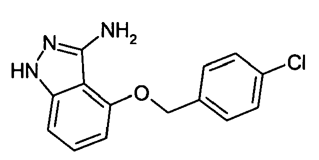 |
| 24 | 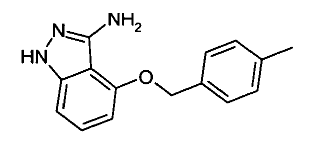 |
| 25 | 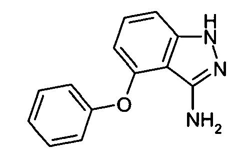 |
| 26 | 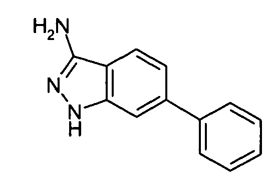 |
| 27 | 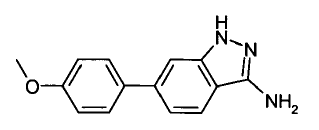 |
| 28 | 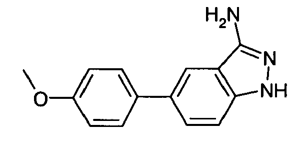 |
| 29 | 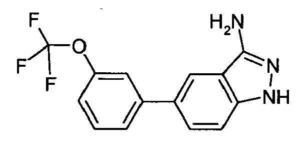 |
| 30 | 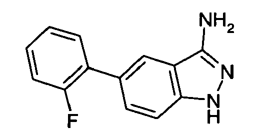 |
| 31 | 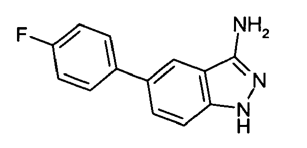 |
| 32 | 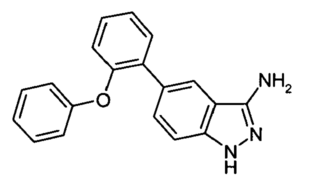 |
| 33 | 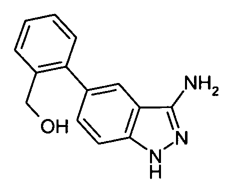 |
| 34 | 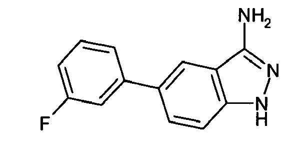 |
| 35 | 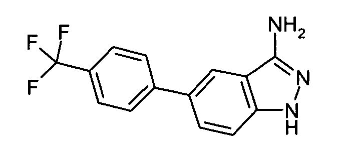 |
| 36 | 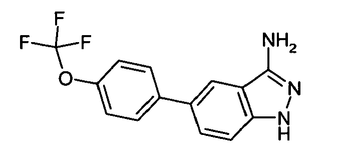 |
| 37 | 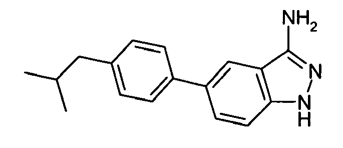 |
| 38 | 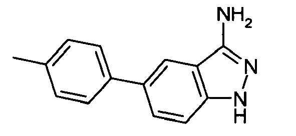 |
| 39 | 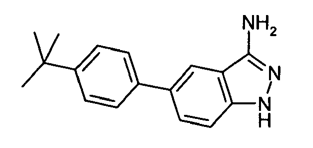 |
| 40 | 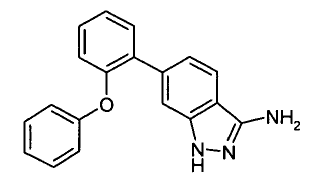 |
| 41 | 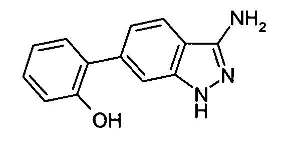 |
| 42 | 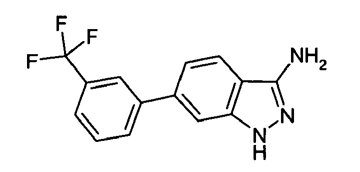 |
| 43 | 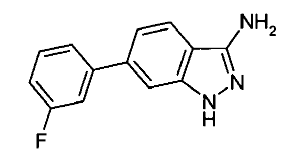 |
| 44 | 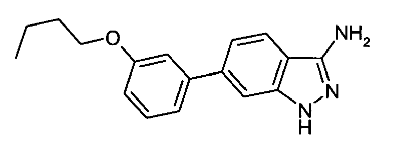 |
| 45 | 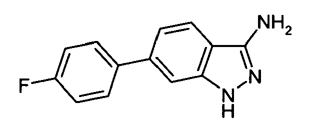 |
| 46 | 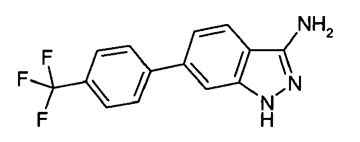 |
| 47 | 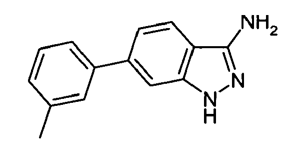 |
| 48 | 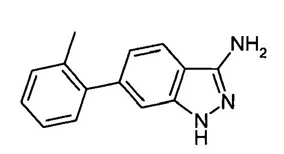 |
| 49 | 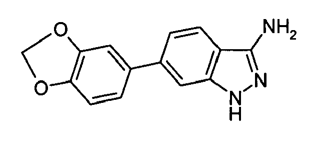 |
| 50 | 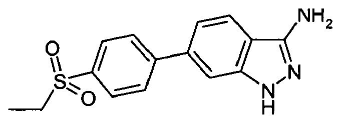 |
| 51 | 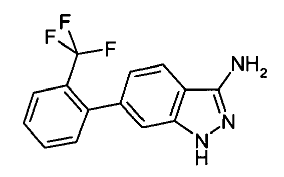 |
| 52 | 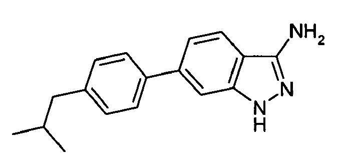 |
| 53 | 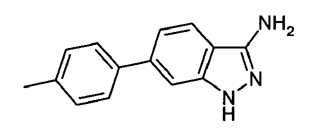 |
| 54 | 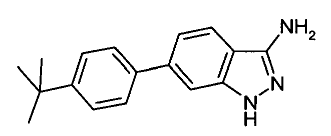 |
| 55 | 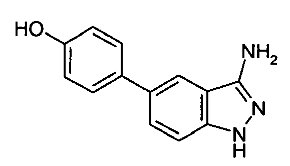 |
| 56 | 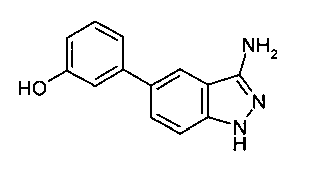 |
| 57 | 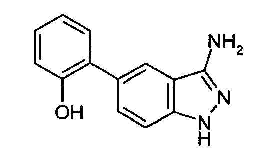 |
| 58 | 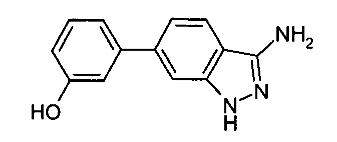 |
| 59 | 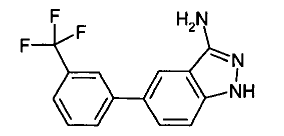 |
| 60 | 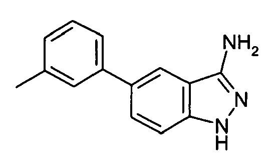 |
| 61 | 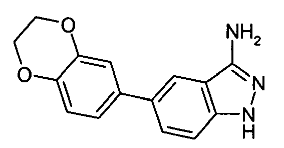 |
| 62 | 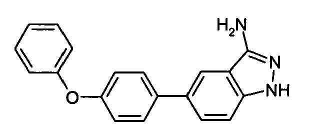 |
| 63 | 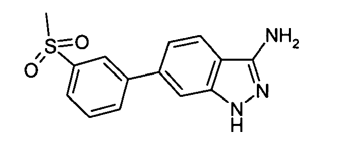 |
| 64 | 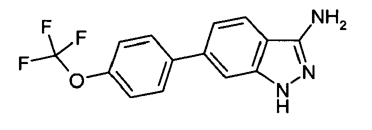 |
| 65 | 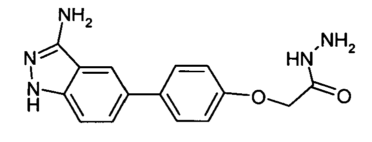 |
| 66 | 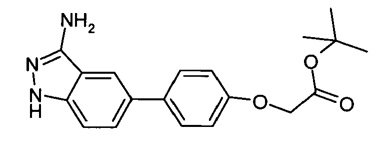 |
| 67 | 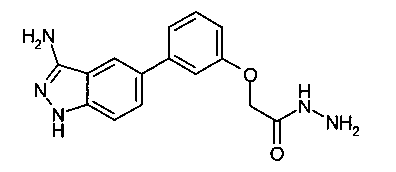 |
| 68 | 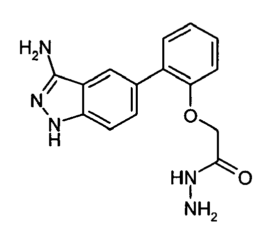 |
| 69 | 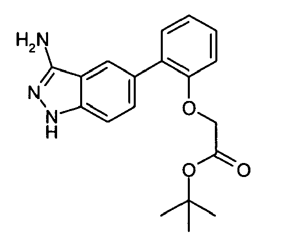 |
| 70 | 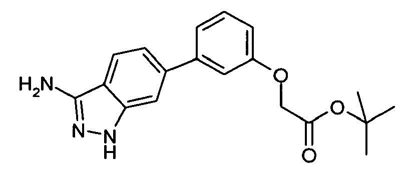 |
| 71 | 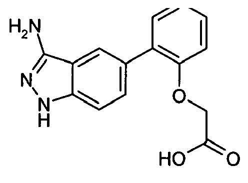 |
| 72 | 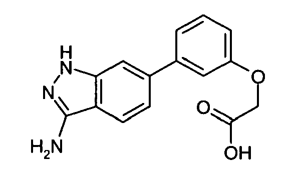 |
| 73 | 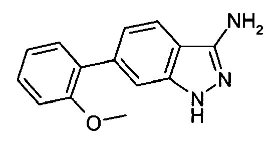 |
| 74 | 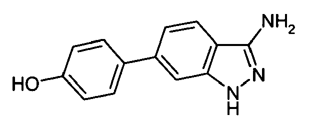 |
| 75 | 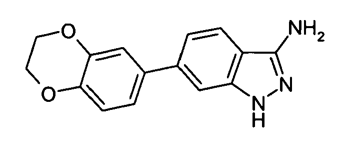 |
| 76 | 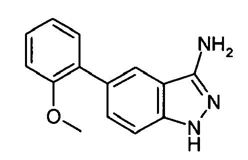 |
| 77 | 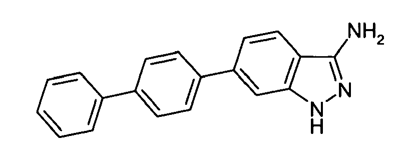 |
| 78 | 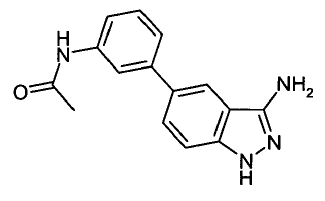 |
| 79 | 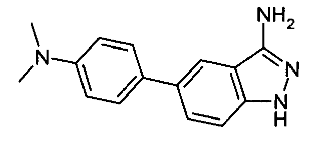 |
| 80 | 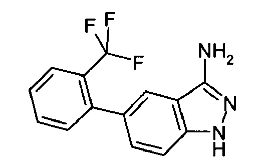 |
| 81 | 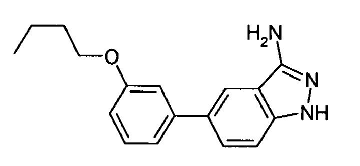 |
| 82 | 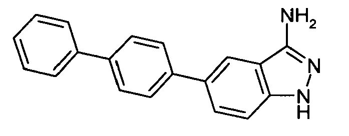 |
| 83 | 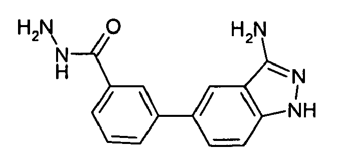 |
| 84 | 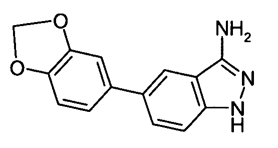 |
| 85 | 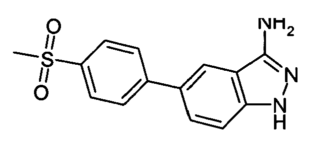 |
| 86 | 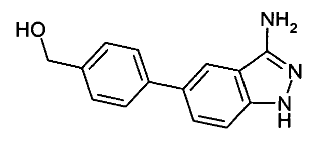 |
| 87 | 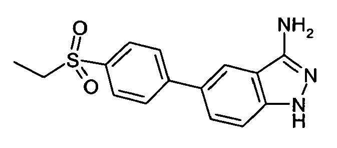 |
| 88 | 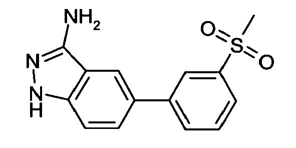 |
| 89 | 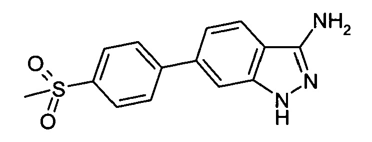 |
| 90 | 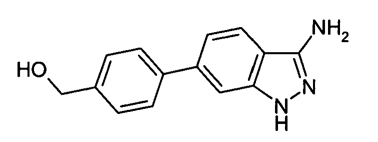 |
| 91 | 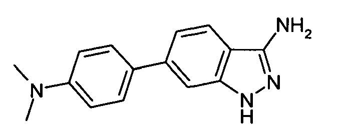 |
| 92 | 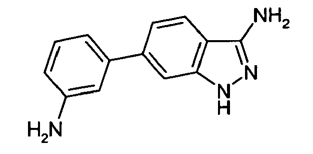 |
| 93 | 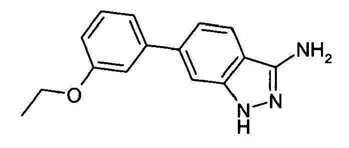 |
| 94 | 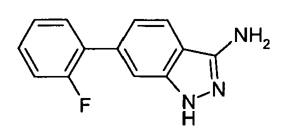 |
| 95 | 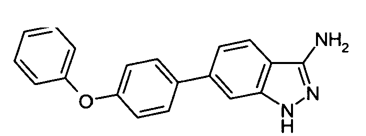 |
| 96 | 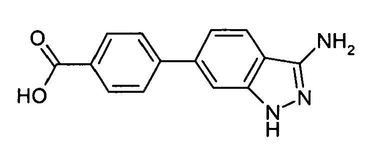 |
| 97 | 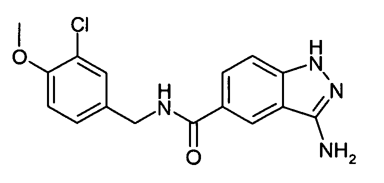 |
| 98 | 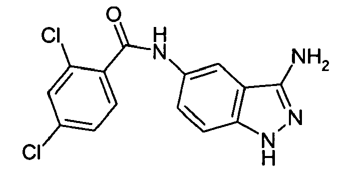 |
| 99 | 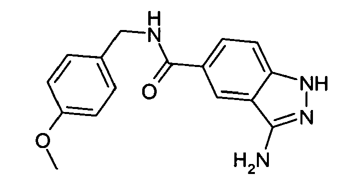 |
| 100 | 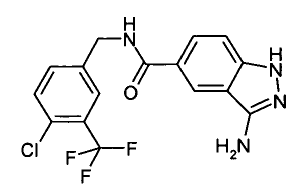 |
| 101 | 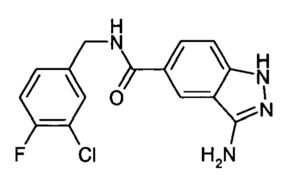 |
| 102 | 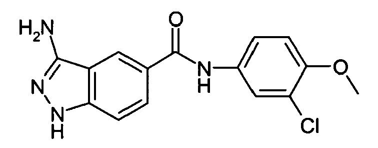 |
| 103 | 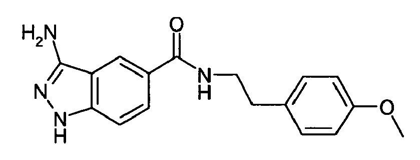 |
| 104 | 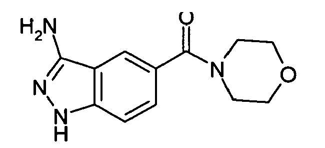 |
| 105 | 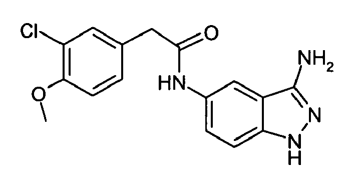 |
| 106 | 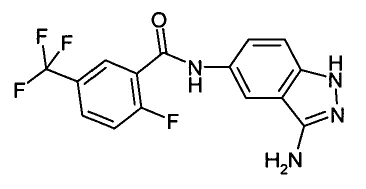 |
| 107 | 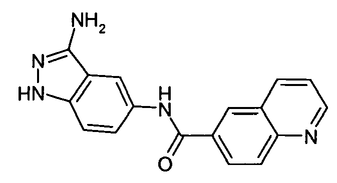 |
| 108 | 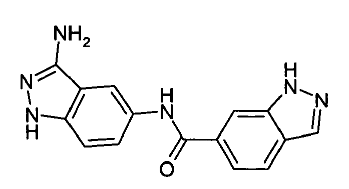 |
| 109 | 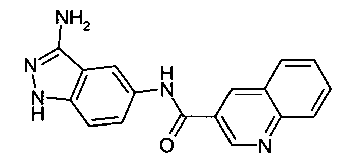 |
| 110 | 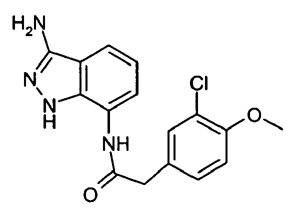 |
| 111 | 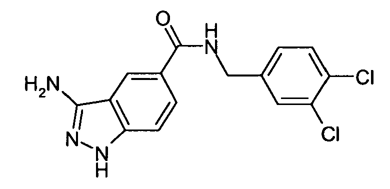 |
| 112 | 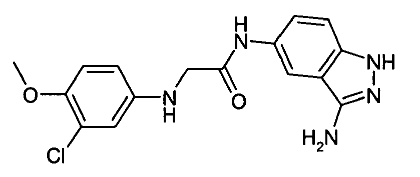 |
| 113 | 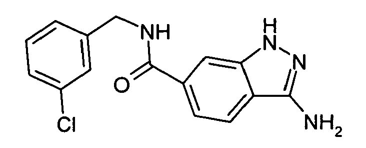 |
| 114 | 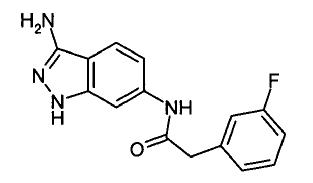 |
| 115 | 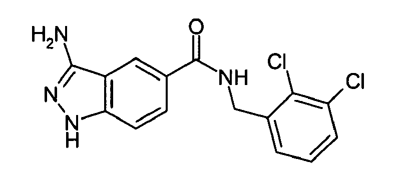 |
| 116 | 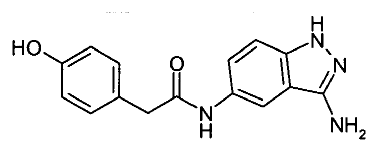 |
| 117 | 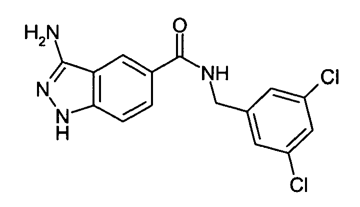 |
| 118 | 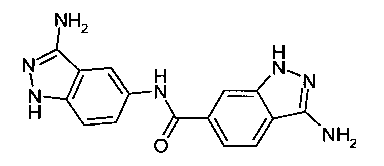 |
| 119 | 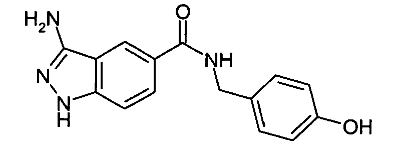 |
| 120 | 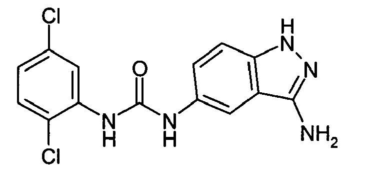 |
| 121 | 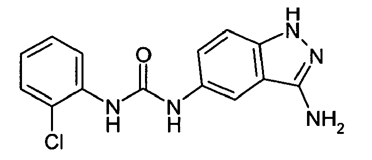 |
| 122 | 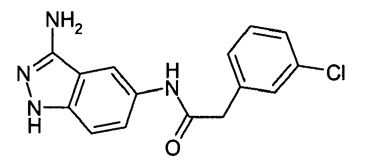 |
| 123 | 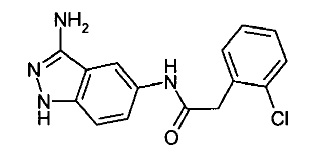 |
| 124 | 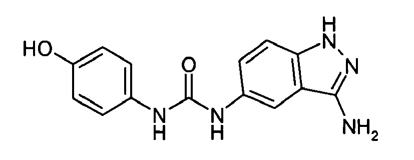 |
| 125 | 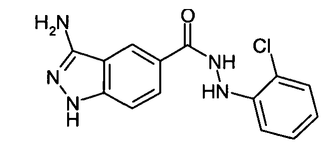 |
| 126 | 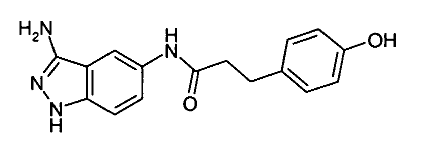 |
| 127 | 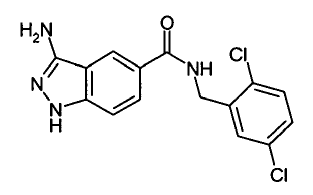 |
| 128 | 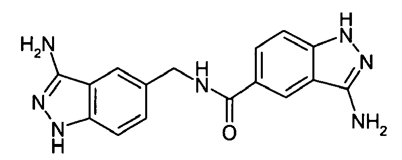 |
| 129 | 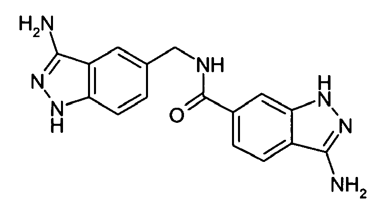 |
| 130 | 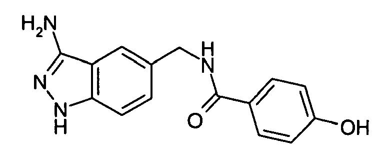 |
| 131 | 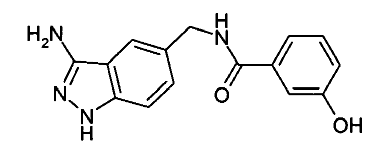 |
| 132 | 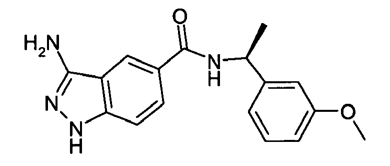 |
| 133 | 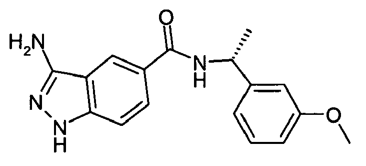 |
| 134 | 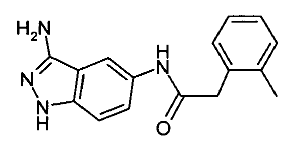 |
| 135 | 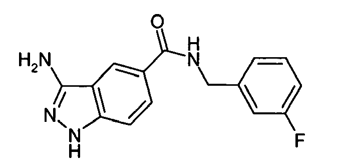 |
| 136 | 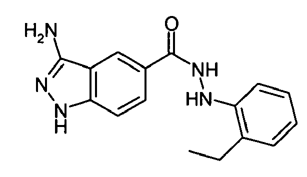 |
| 137 | 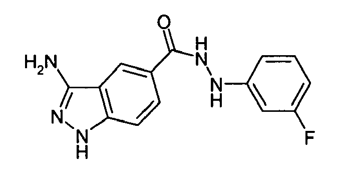 |
| 138 | 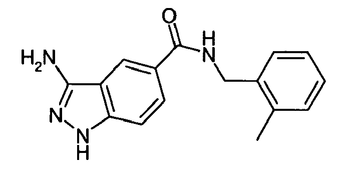 |
| 139 | 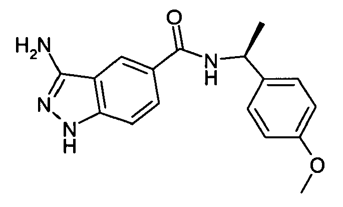 |
| 140 | 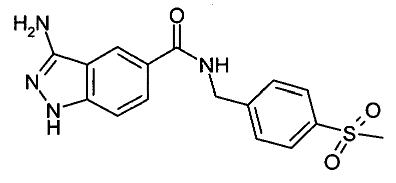 |
| 141 | 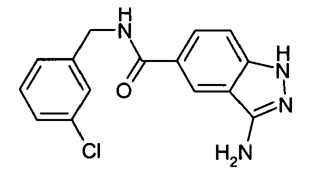 |
| 142 | 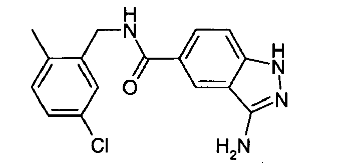 |
| 143 | 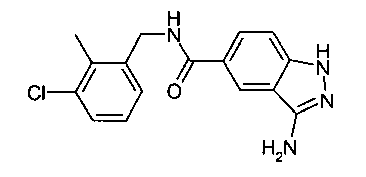 |
| 144 | 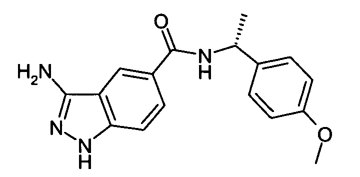 |
| 145 | 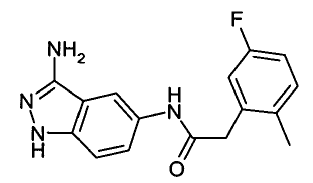 |
| 146 | 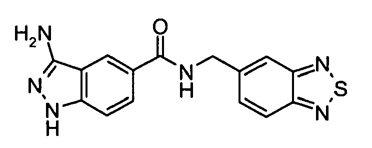 |
| 147 | 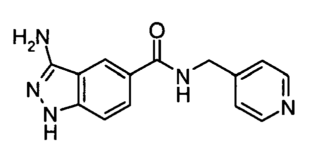 |
| 148 | 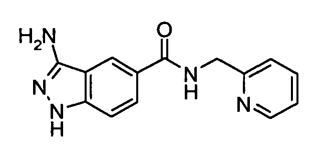 |
| 149 | 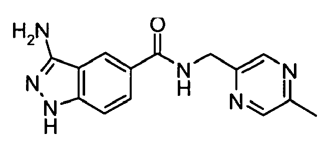 |
| 150 | 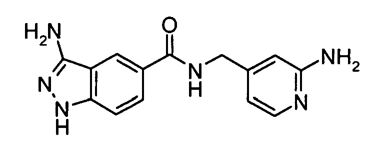 |
| 151 | 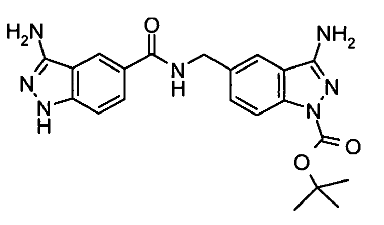 |
| 152 | 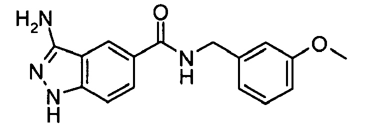 |
| 153 | 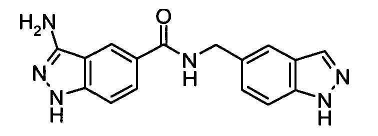 |
| 154 | 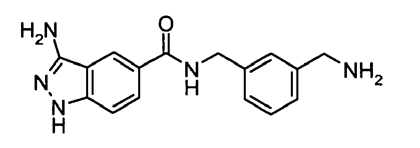 |
| 155 | 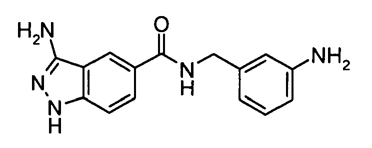 |
| 156 | 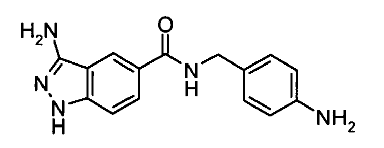 |
| 157 | 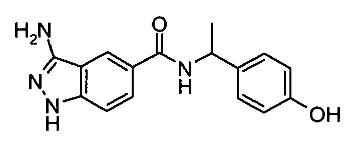 |
| 158 | 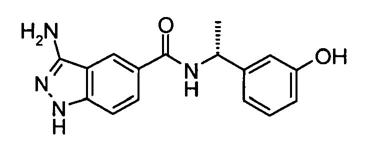 |
| 159 | 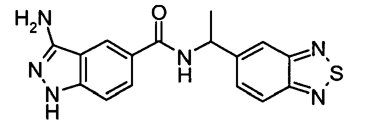 |
| 160 | 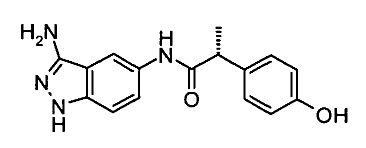 |
| 161 | 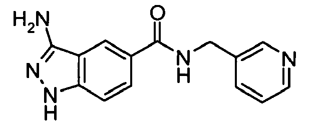 |
| 162 | 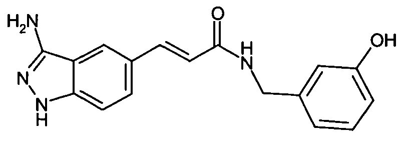 |
| 163 | 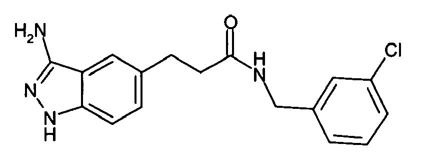 |
| 164 | 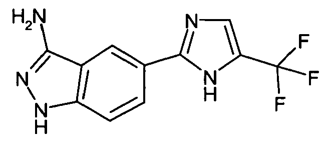 |
| 165 | 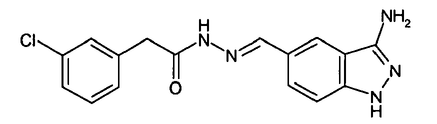 |
| 166 | 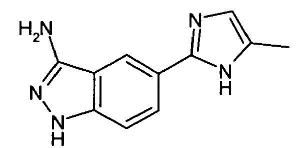 |
| 167 | 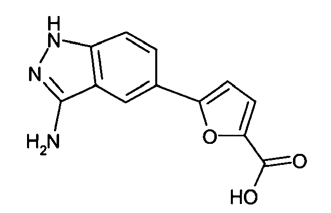 |
| 168 | 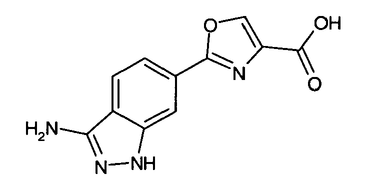 |
| 169 | 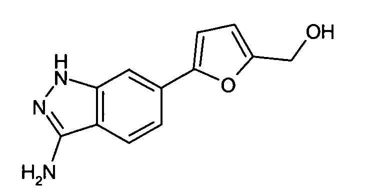 |
| 170 | 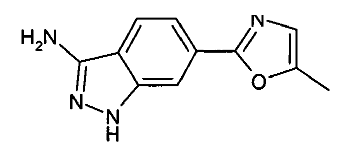 |
| 171 | 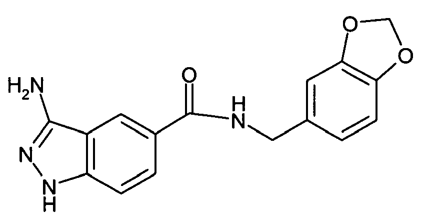 |
| 172 | 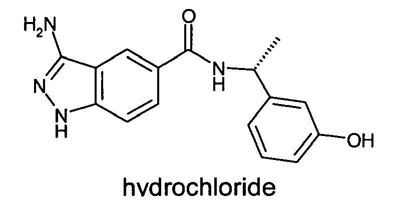 |
| 173 | 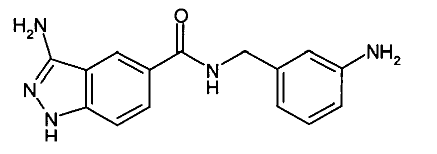 |
| 174 | 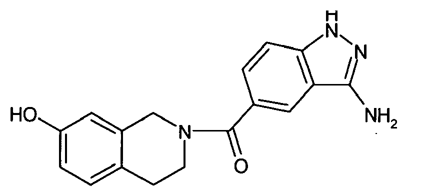 |
| 175 | 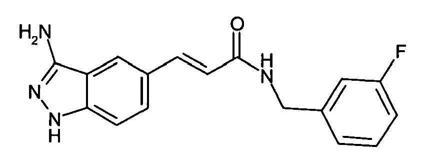 |
| 176 | 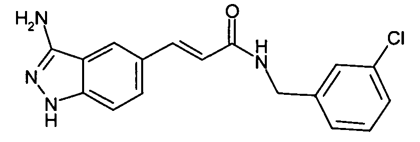 |
| 177 | 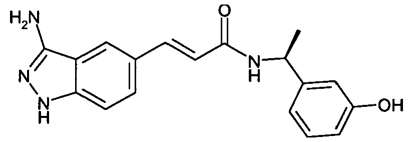 |
| 178 | 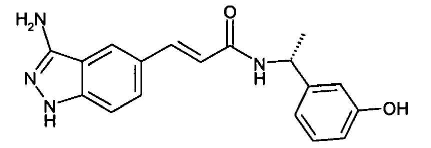 |
| 179 | 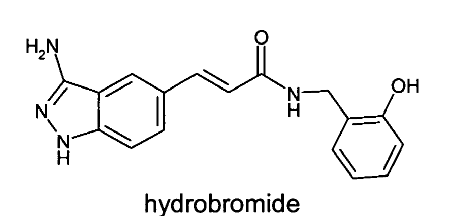 |
| 180 | 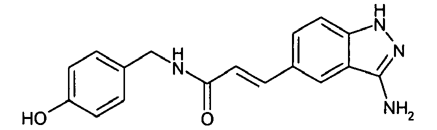 |
| 181 | 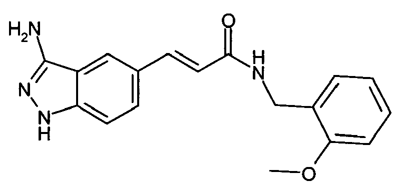 |
| 182 | 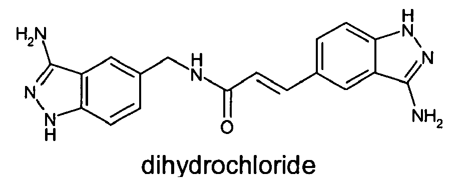 |
| 183 | 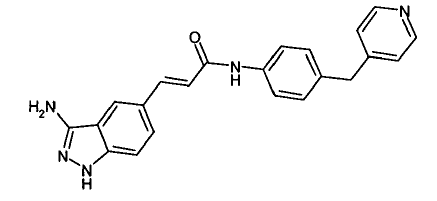 |
| 184 | 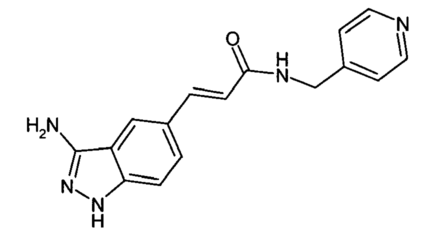 |
| 185 | 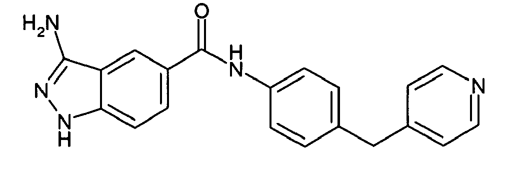 |
| 186 | 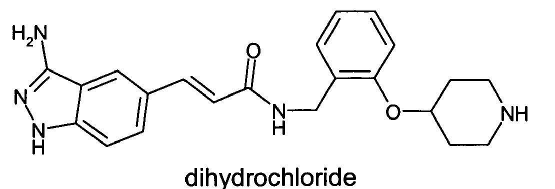 |
| 187 | 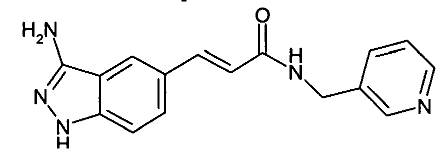 |
| 188 | 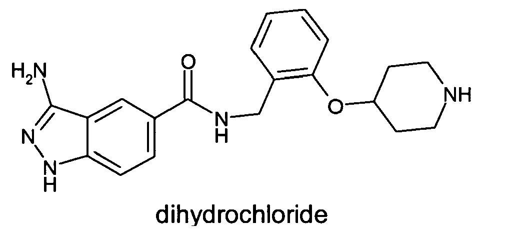 |
| 189 | 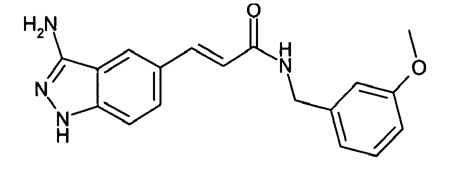 |
| 190 | 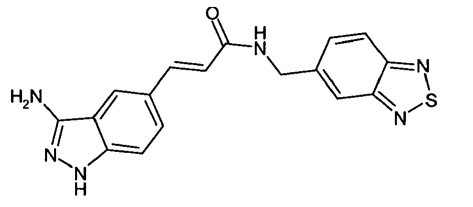 |
| 191 | 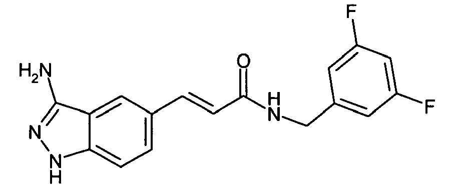 |
| 192 | 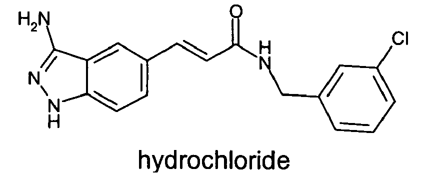 |
| 193 | 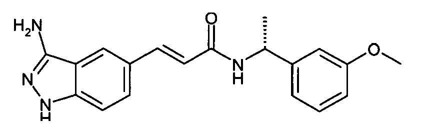 |
| 194 | 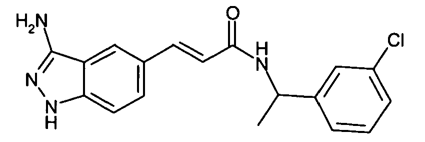 |
| 195 | 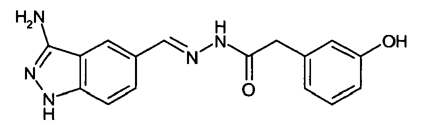 |
| 196 | 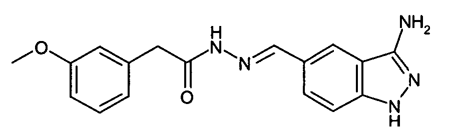 |
| 197 | 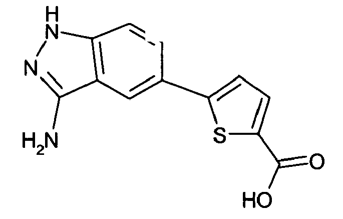 |
| 198 | 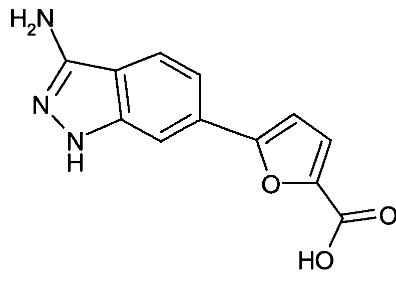 |
| 199 | 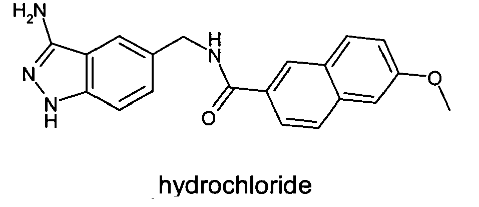 |
| 200 | 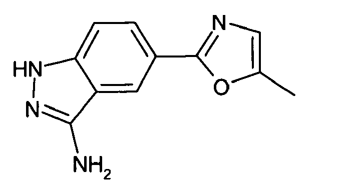 |
| 201 | 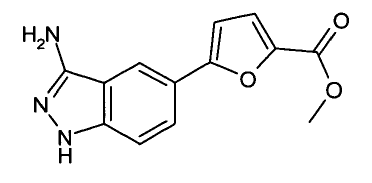 |
and pharmaceutically usable salts, E,Z isomers, diastereomers, enantiomers, mono-, dihydrates and alcoholates thereof, including mixtures thereof in all ratios.

3. Process for the preparation of compounds of the formula I according to Claims 1-13 and pharmaceutically usable salts, E,Z isomers, diastereomers, enantiomers, mono-, dihydrates and alcoholates thereof, including mixtures thereof in all ratios, **characterised in that**
a) for the preparation of compounds of the formula I in which
W denotes
i) -[C(R³)₂]ₙCONH[C(R³)₂]ₙ- or
ii) -[C(R³)₂]ₙCONHNH[C(R³)₂]ₙ-,
a compound of the formula II 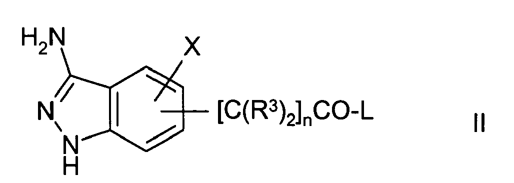 in which
X, R³ and n have the meaning indicated in Claim 1,
and L denotes Cl, Br, I or a free or reactively functionally
modified OH group,
is reacted with
i) a compound of the formula IIIa
H₂N[C(R³)₂]ₙ-R¹ IIIa
in which
R¹, R³ and n have the meanings indicated in Claim 1,
or
ii) a compound of the formula IIIb
H₂N-NH[C(R³)₂]ₙ-R¹ IIIb
in which
R¹, R³ and n have the meanings indicated in Claim 1,
or
b) for the preparation of compounds of the formula I in which
W denotes -[C(R³)₂]ₙNHCO[C(R³)₂]ₙ-,
a compound of the formula IV 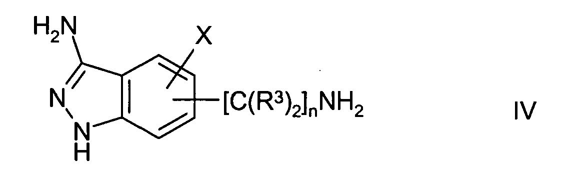 in which X, R³ and n have the meanings indicated in Claim 1, is reacted with a compound of the formula V
L-CO-[C(R³)₂]ₙ-R¹ V
in which
L denotes Cl, Br, I or a free or reactively functionally modified OH group and
R¹, R³ and n have the meanings indicated in Claim 1,
or
c) for the preparation of compounds of the formula I in which
W denotes -[C(R₃)₂]ₙ-, -[C(R³)₂]ₙO[C(R³)₂]ₙ-, -[C(R³)₂]ₙNR³[C(R³)₂]ₙ- or -[C(R³)₂]ₙNR¹[C(R³)₂]ₙ-,
a compound of the formula VI 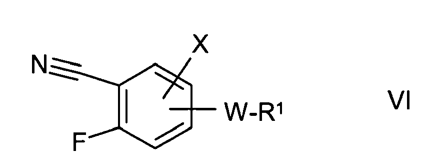 in which
R¹, W and X have the meanings indicated in Claim 1,
is reacted with hydrazine,
or
d) for the preparation of compounds of the formula I in which
W denotes -[C(R³)₂]ₙNHCONH[C(R³)₂]ₙ-,
a compound of the formula IV is reacted with a compound of the formula VII
O=C=N-[C(R³)₂]ₙ-R¹ VII
in which R¹, R³ and n have the meanings indicated in Claim 1,
or
e) **in that** they are liberated from one of their functional derivatives by treatment with a solvolysing or hydrogenolysing agent,
and/or
a base or acid of the formula I is converted into one of its salts.

4. Medicaments comprising at least one compound according to Claims 1-2 and/or pharmaceutically usable salts, E,Z isomers, diastereomers, enantiomers, mono-, dihydrates and alcoholates thereof, including mixtures thereof in all ratios, and optionally excipients and/or adjuvants.

5. Use of compounds according to Claims 1-2, and pharmaceutically usable salts, E,Z isomers, diastereomers, enantiomers, mono-, dihydrates and alcoholates thereof, including mixtures thereof in all ratios, for the preparation of a medicament for the treatment and/or prophylaxis of diseases in which the inhibition, regulation and/or modulation of kinase signal transduction plays a role.

6. Use according to Claim 5, where the kinase is SGK.

7. Use according to Claim 6 of compounds according to Claims 1-2, and pharmaceutically usable salts, E,Z isomers, diastereomers, enantiomers, mono-, dihydrates and alcoholates thereof, including mixtures thereof in all ratios, for the preparation of a medicament for the treatment of diseases which are influenced by inhibition of SGKs by the compounds according to Claims 1-2.

8. Use according to Claim 7 of compounds according to Claims 1-2, and pharmaceutically usable salts, E,Z isomers, diastereomers, enantiomers, mono-, dihydrates and alcoholates thereof, including mixtures thereof in all ratios, for the preparation of a medicament for the treatment or prevention of diabetes, obesity, metabolic syndrome (dyslipidaemia), systemic and pulmonary hypertonia, cardiovascular diseases and renal diseases, generally in fibrosis and inflammatory processes of any type, cancer, tumour cells, tumour metastases, coagulopathies, neuronal excitability, glaucoma, cataracts, bacterial infections and in antiinfection therapy, for increasing learning ability and attention and for the treatment of tinnitus.

9. Use according to Claim 8, where diabetes is diabetes mellitus, diabetic nephropathy, diabetic neuropathy, diabetic angiopathy and microangiopathy.

10. Use according to Claim 8, where cardiovascular diseases are cardiac fibroses after myocardial infarction, cardiac hypertrophy, cardiac insufficiency and arteriosclerosis.

11. Use according to Claim 8, where renal diseases are glomerulosclerosis, nephrosclerosis, nephritis, nephropathy and electrolyte excretion disorder.

12. Use according to Claim 8, where fibroses and inflammatory processes are liver cirrhosis, lung fibrosis, fibrosing pancreatitis, rheumatism and arthritis, Crohn's disease, chronic bronchitis, radiation fibrosis, sclerodermatitis, cystic fibrosis, scarring and Alzheimer's disease.

13. Medicaments comprising at least one compound according to Claims 1-2 and/or pharmaceutically usable salts, E,Z isomers, diastereomers, enantiomers, mono-, dihydrates and alcoholates thereof, including mixtures thereof in all ratios, and at least one further medicament active compound.

14. Set (kit) consisting of separate packs of
(a) an effective amount of a compound according to Claims 1-2 and/or pharmaceutically usable salts, E,Z isomers, diastereomers, enantiomers, mono-, dihydrates and alcoholates thereof, including mixtures thereof in all ratios, and
(b) an effective amount of a further medicament active compound.

15. Intermediate compounds selected from the group 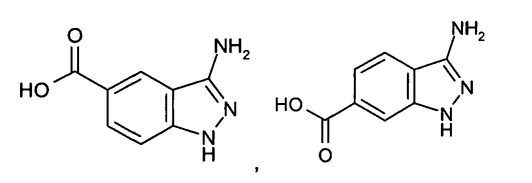 and salts thereof.

## Revendications

1. Composés de formule I 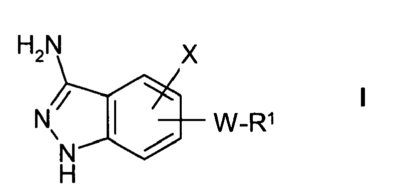 dans laquelle
W désigne -[C(R³)₂]ₙ-, -[C(R³)₂]ₙCONH[C(R³)₂]ₙ-, -[C(R³)₂]ₙNR³CO[C(R³)₂]ₙNR³-, -[C(R³)₂]ₙO[C(R³)₂]ₙ-, -[C(R³)₂]ₙNR³[C(R³)₂]ₙ-, -[C(R³)₂]ₙN[C(R³)₂R¹][C(R³)₂]ₙ-, -[C(R³)₂]ₙNR³C(R³)₂]ₙCONR³[C(R³)₂]ₙ-, -[C(R³)₂]ₙCO[C(R³)₂]ₙ-, -[C(R³)₂]ₙCONHNH[C(R³)₂]ₙ-, -[C(R³)₂]ₙNHCO[C(R³)₂]ₙ-, -C(R³)=C(R³)CONR³[C(R³)₂]ₙ ou -C(R³)=N-NR³CO[C(R³)₂]ₙ,
X désigne H,
R¹ désigne phényle ou naphtyle, chacun d'entre eux pouvant être mono-, di- ou trisubstitué par Hal, A, -(CH₂)ₙOH, OA, phényle, phénoxy, benzyloxy, pyridin-4-ylméthyl-, pipéridin-4-oxy-, NO₂, CN, -(CH₂)ₙCOOR³, O-(CH₂)ₙCOOR³, SO₂A, NHCOA, (CH₂)ₙNH₂, (CH₂)ₙNHA, (CH₂)ₙNA₂, -[C(R³)₂]ₙCONH₂, (CH₂)ₙCONHNH₂ ou O-(CH₂)ₙCONHNH₂,
ou
un hétérocycle saturé ou insaturé mono- ou bicyclique ayant de 1 à 4 atomes de N, O et/ou S, qui est non substitué ou mono- ou disubstitué par NH₂, A, OH, CH₂OH, COOH et/ou COOA,
R³ désigne H ou A,
A désigne alkyle non ramifié ou ramifié ayant 1-6 atomes de C, dans lequel 1-7 atomes de H peuvent être remplacés par F,
Hal désigne F, Cl, Br ou I,
n désigne 0, 1 ou 2,
et les sels, isomères E,Z, diastéréoisomères, énantiomères, mono-, dihydrates et alcoolates pharmaceutiquement utilisables de ceux-ci, y compris des mélanges de ceux-ci en toutes proportions.

2. Composés selon la revendication 1, choisis parmi le groupe constitué par
le N-(3-chlorobenzyl)-3-amino-1*H*-indazole-5-carboxamide (« 1 »),
l'acide [3-(3-amino-1*H*-indazol-5-yl)phénoxy]acétique (« 2 »),
le N-(3-amino-1*H*-indazol-5-yl)-2-(3-hydroxyphényl)acétamide (« 3 »),
le N-(3-amino-1*H*-indazol-5-ylméthyl)-3-chlorobenzamide (« 3.1 »),
le N-(3-amino-1*H*-indazol-6-yl)-2-(3-chloro-4-méthoxyphényl)-acétamide (« 4 »),
le N-3-chlorobenzyl-3-amino-1*H*-indazole-6-carboxamide (« 4.1 »),
la 6-(4-phénoxyphényl)-1*H*-indazol-3-ylamine (« 5 »),
la 5-(3-méthanesulfonylphényl)-1*H*-indazol-3-ylamine (« 6 »),
la 6-(3-chloro-4-méthoxybenzyloxy)-1*H*-indazol-3-ylamine (« 6.1 »),
la N5-(3-chloro-4-méthoxybenzyl)-1*H*-indazole-3,5-diamine (« 6.2 »),
la N5,N5-bis(3-chloro-4-méthoxybenzyl)-1*H*-indazole-3,5-diamine (« 6.2a »),
la 1-(3-amino-1*H*-indazol-5-yl)-3-(4-benzyloxyphényl)urée (« 6.3 »),
la 1-(3-amino-1*H*-indazol-5-yl)-3-(3-chlorophényl)urée (« 6.3a »),
| | |
|---|---|
| 7 | 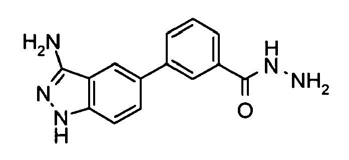 |
| 8 | 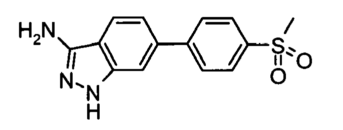 |
| 9 | 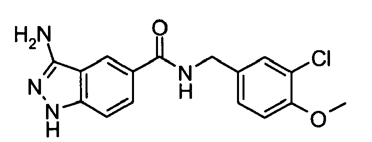 |
| 10 | 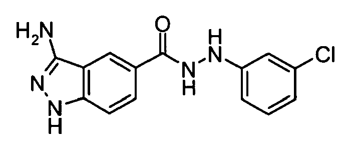 |
| 11 | 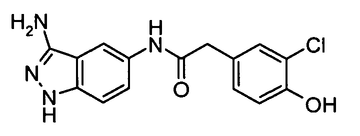 |
| 12 | 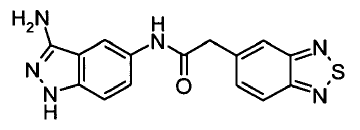 |
| 13 | 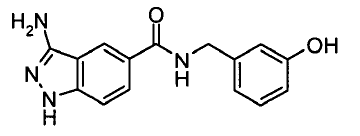 |
| 14 | 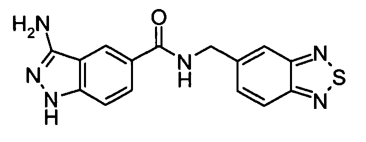 |
| 15 | 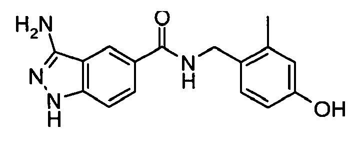 |
| 16 | 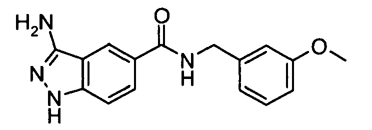 |
| 17 | 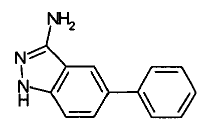 |
| 18 | 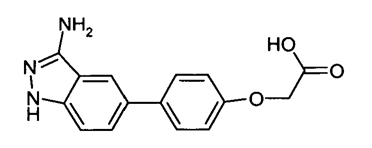 |
| 19 | 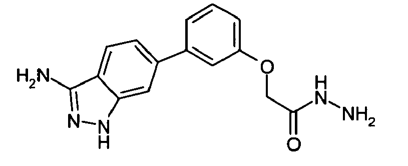 |
| 20 | 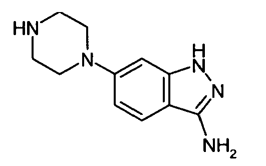 |
| 21 | 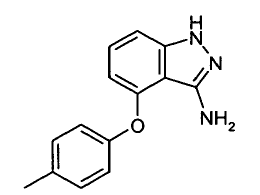 |
| 22 | 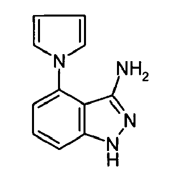 |
| 23 | 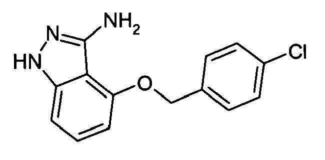 |
| 24 | 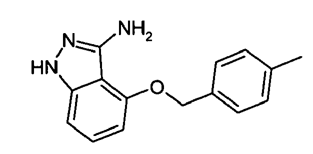 |
| 25 | 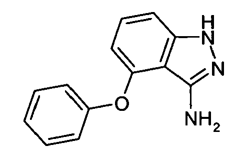 |
| 26 | 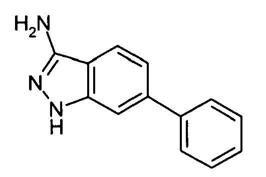 |
| 27 | 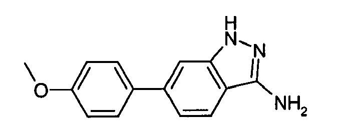 |
| 28 | 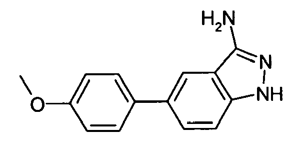 |
| 29 | 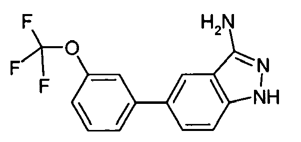 |
| 30 | 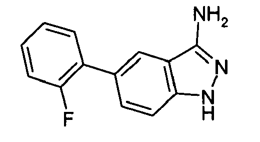 |
| 31 | 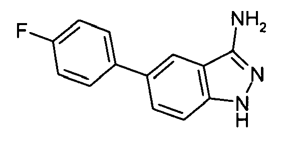 |
| 32 | 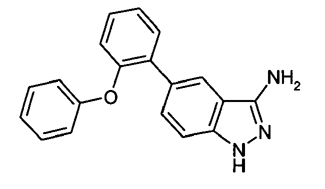 |
| 33 | 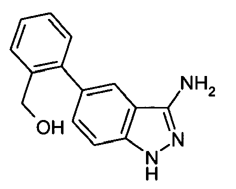 |
| 34 | 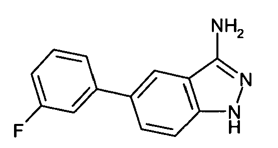 |
| 35 | 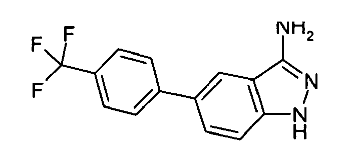 |
| 36 | 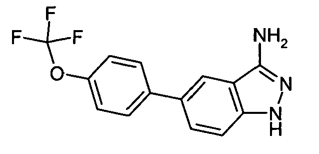 |
| 37 | 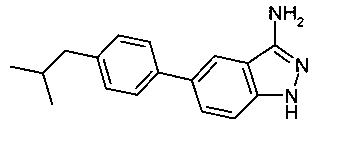 |
| 38 | 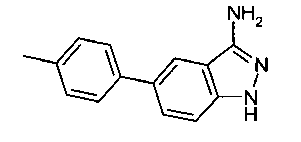 |
| 39 | 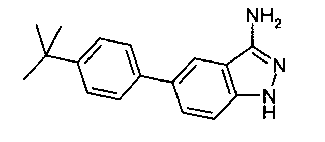 |
| 40 | 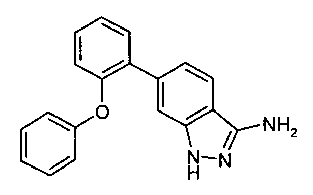 |
| 41 | 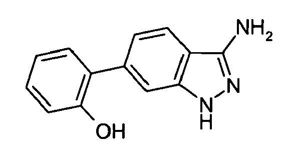 |
| 42 | 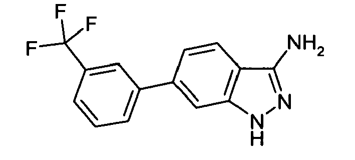 |
| 43 | 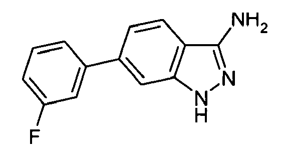 |
| 44 | 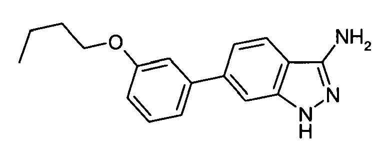 |
| 45 | 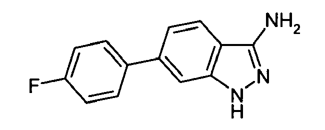 |
| 46 | 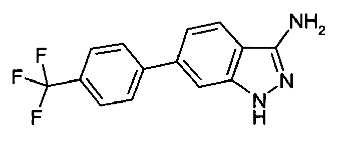 |
| 47 | 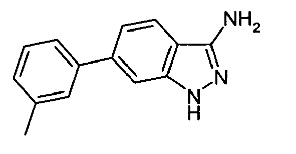 |
| 48 | 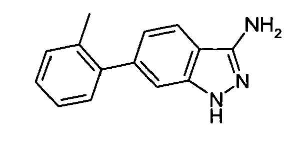 |
| 49 | 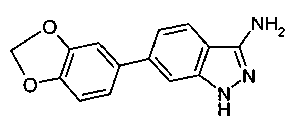 |
| 50 | 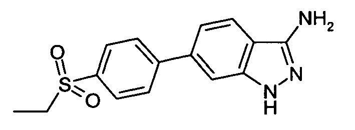 |
| 51 | 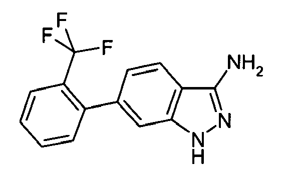 |
| 52 | 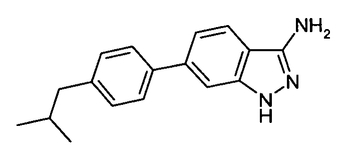 |
| 53 | 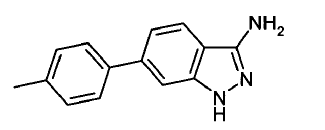 |
| 54 | 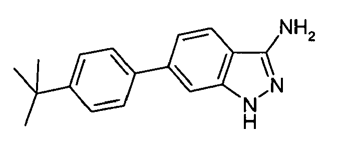 |
| 55 | 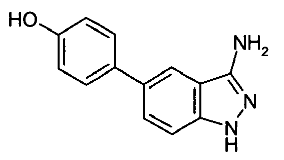 |
| 56 | 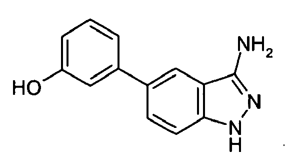 |
| 57 | 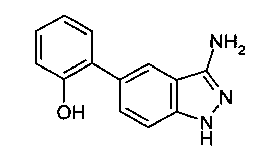 |
| 58 | 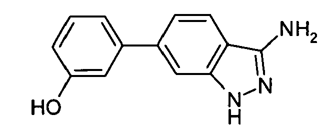 |
| 59 | 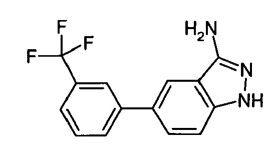 |
| 60 | 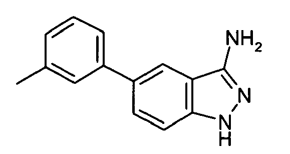 |
| 61 | 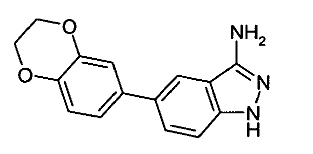 |
| 62 | 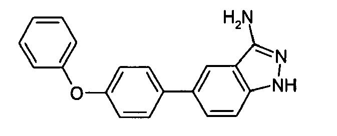 |
| 63 | 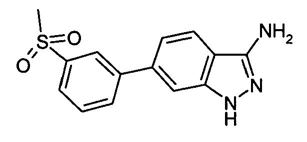 |
| 64 | 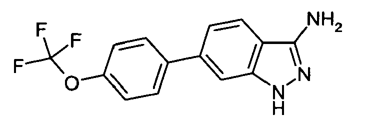 |
| 65 | 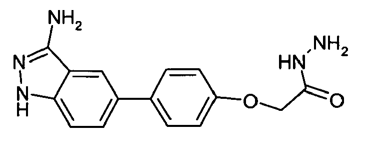 |
| 66 | 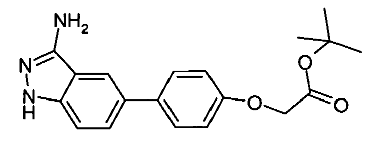 |
| 67 | 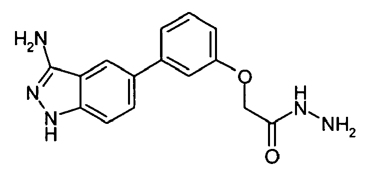 |
| 68 | 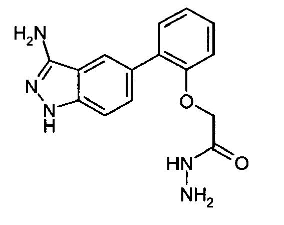 |
| 69 | 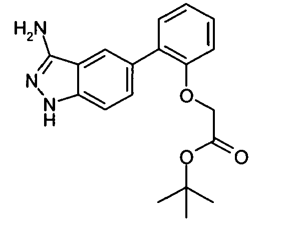 |
| 70 | 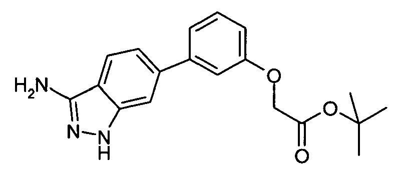 |
| 71 | 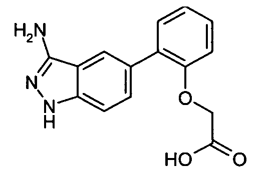 |
| 72 | 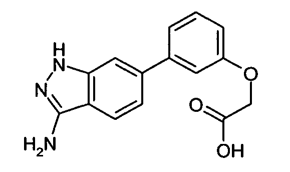 |
| 73 | 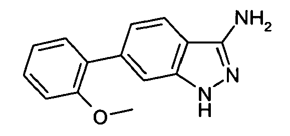 |
| 74 | 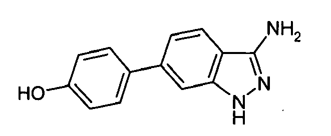 |
| 75 | 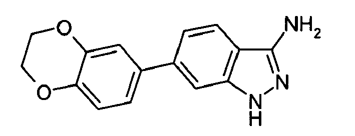 |
| 76 | 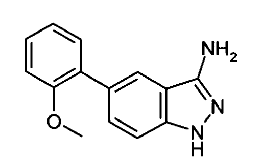 |
| 77 | 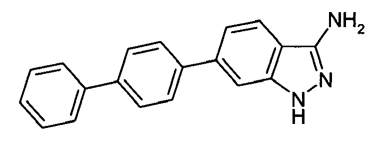 |
| 78 | 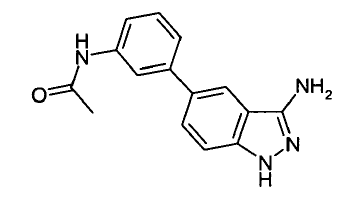 |
| 79 | 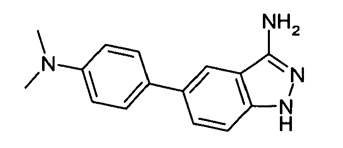 |
| 80 | 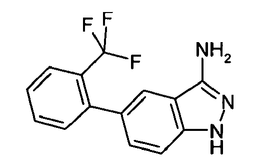 |
| 81 | 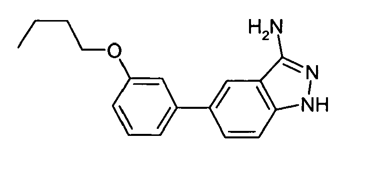 |
| 82 | 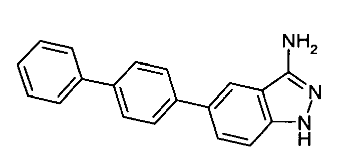 |
| 83 | 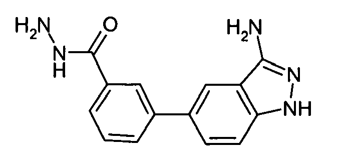 |
| 84 | 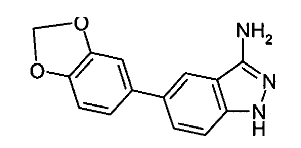 |
| 85 | 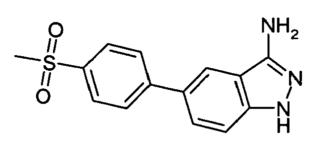 |
| 86 | 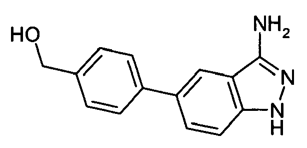 |
| 87 | 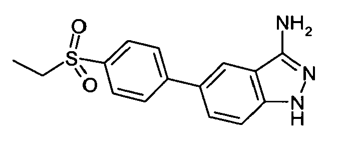 |
| 88 | 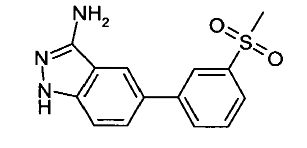 |
| 89 | 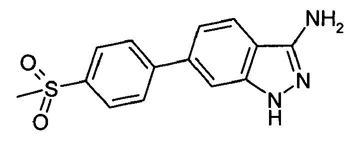 |
| 90 | 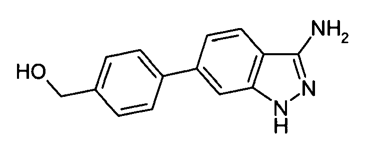 |
| 91 | 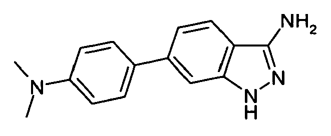 |
| 92 | 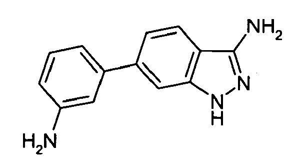 |
| 93 | 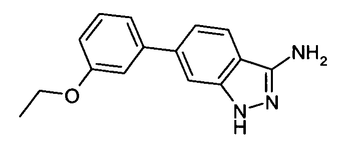 |
| 94 | 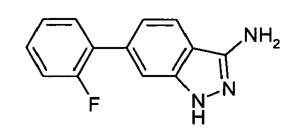 |
| 95 | 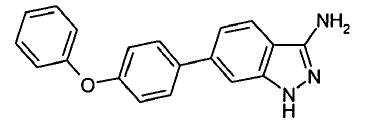 |
| 96 | 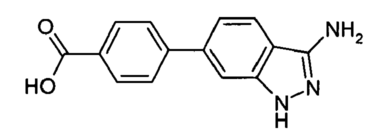 |
| 97 | 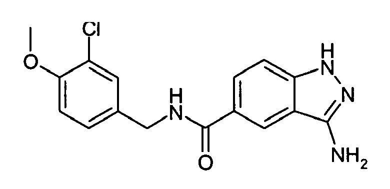 |
| 98 | 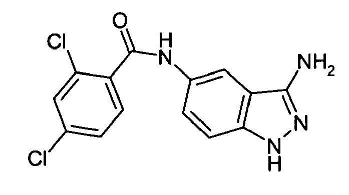 |
| 99 | 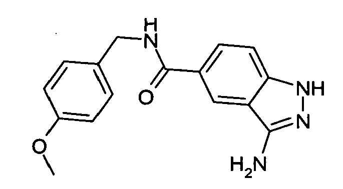 |
| 100 | 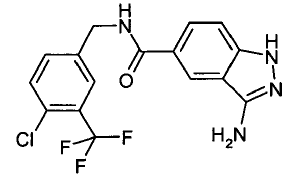 |
| 101 | 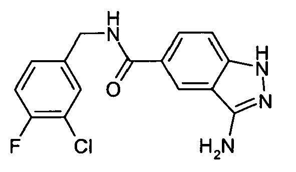 |
| 102 | 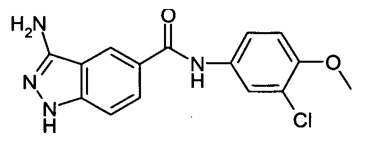 |
| 103 | 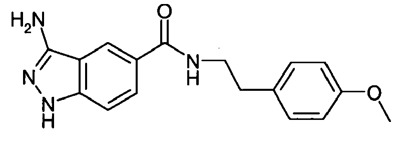 |
| 104 | 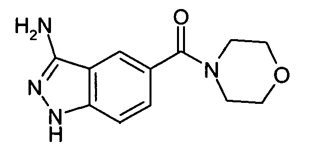 |
| 105 | 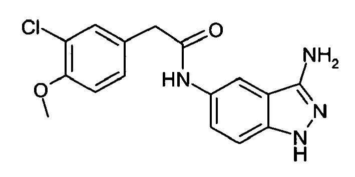 |
| 106 | 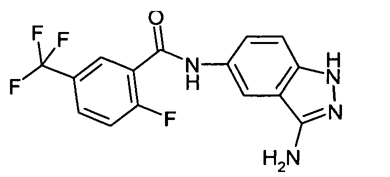 |
| 107 | 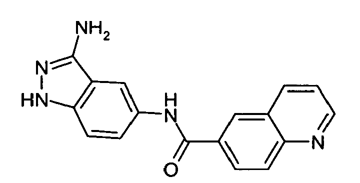 |
| 108 | 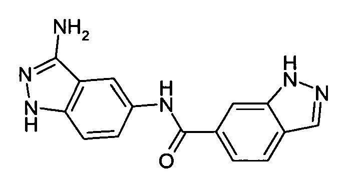 |
| 109 | 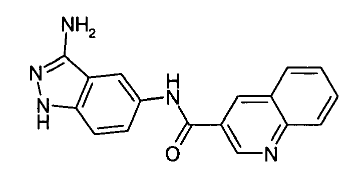 |
| 110 | 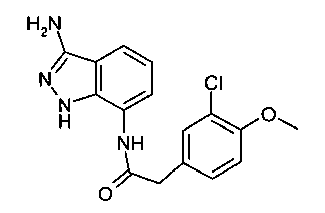 |
| 111 | 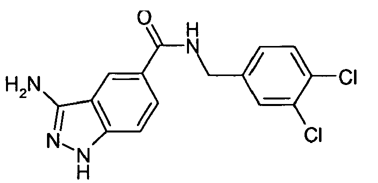 |
| 112 | 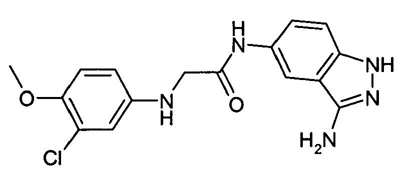 |
| 113 | 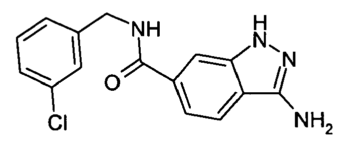 |
| 114 | 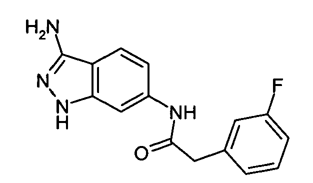 |
| 115 | 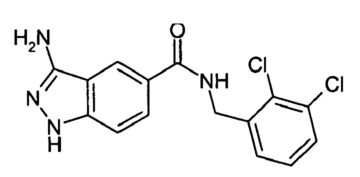 |
| 116 | 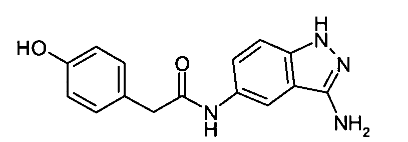 |
| 117 | 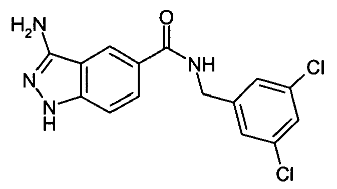 |
| 118 | 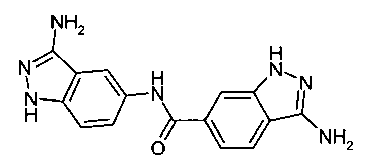 |
| 119 | 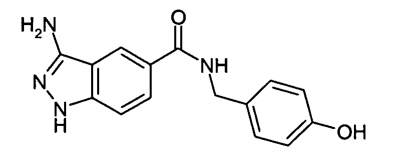 |
| 120 | 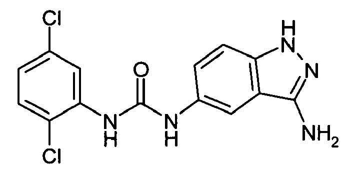 |
| 121 | 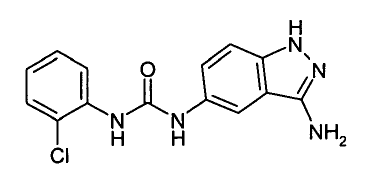 |
| 122 | 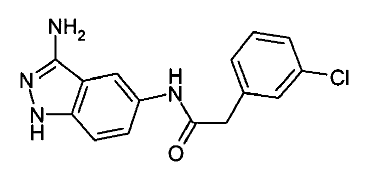 |
| 123 | 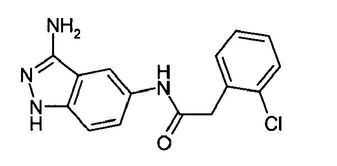 |
| 124 | 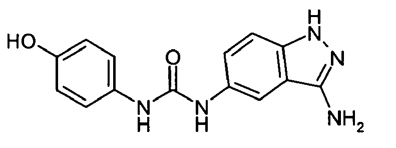 |
| 125 | 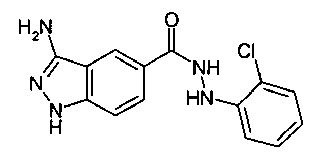 |
| 126 | 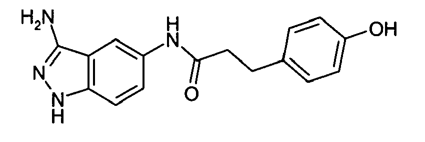 |
| 127 | 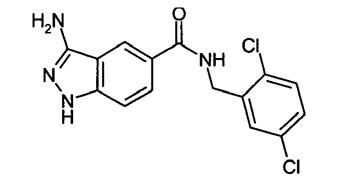 |
| 128 | 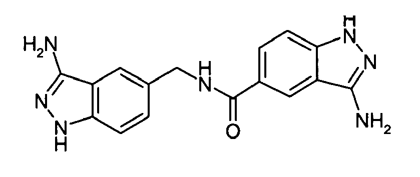 |
| 129 | 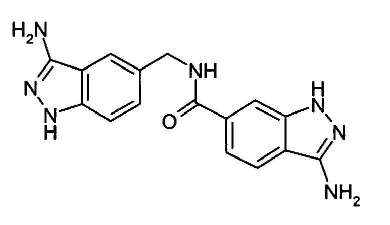 |
| 130 | 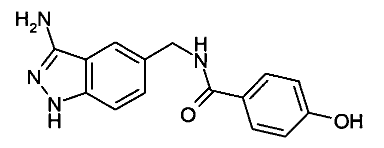 |
| 131 | 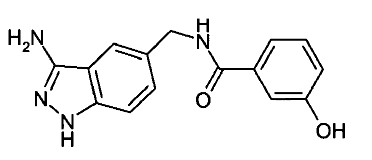 |
| 132 | 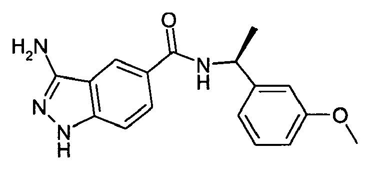 |
| 133 | 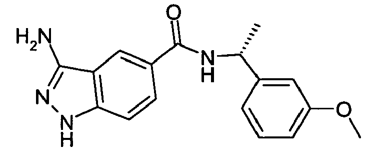 |
| 134 | 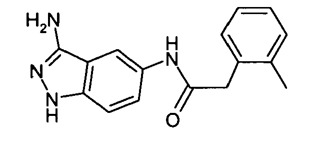 |
| 135 | 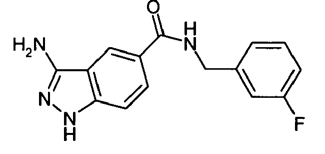 |
| 136 | 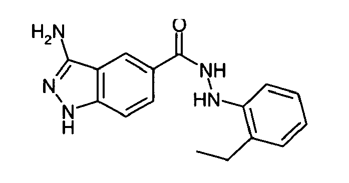 |
| 137 | 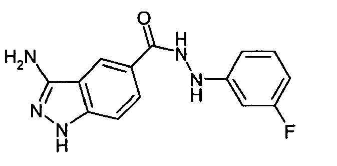 |
| 138 | 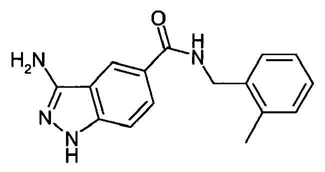 |
| 139 | 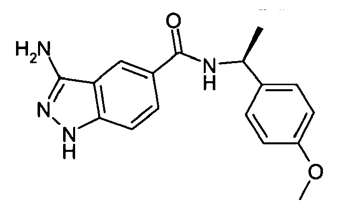 |
| 140 | 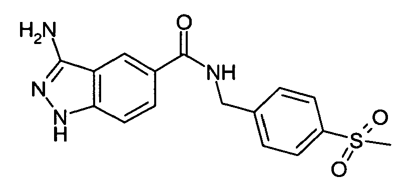 |
| 141 | 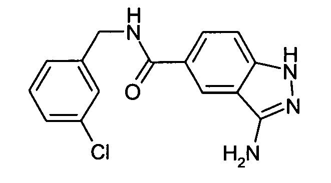 |
| 142 | 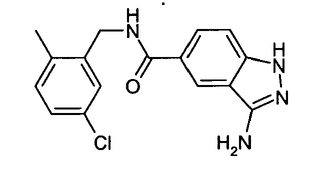 |
| 143 | 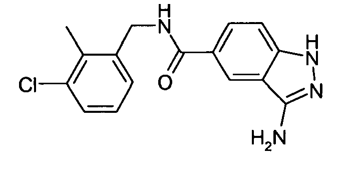 |
| 144 | 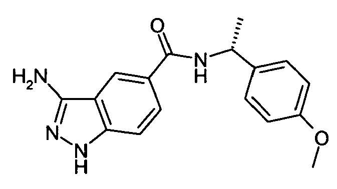 |
| 145 | 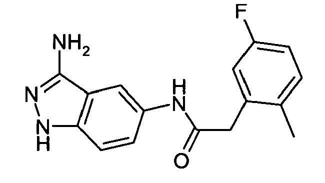 |
| 146 | 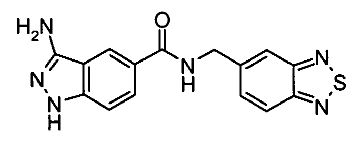 |
| 147 | 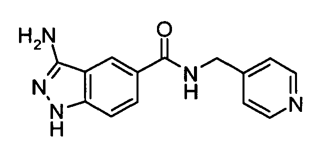 |
| 148 | 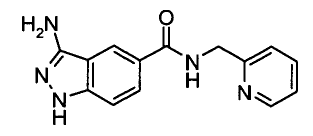 |
| 149 | 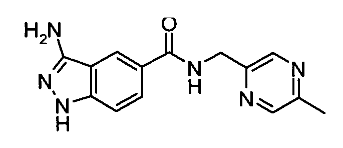 |
| 150 | 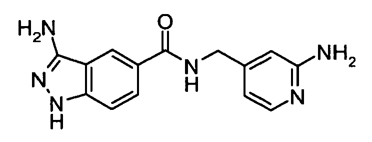 |
| 151 | 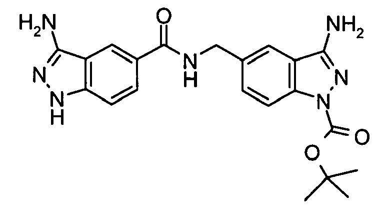 |
| 152 | 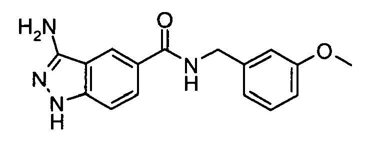 |
| 153 | 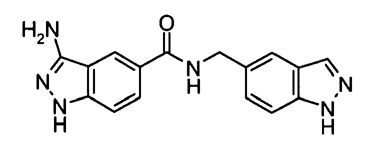 |
| 154 | 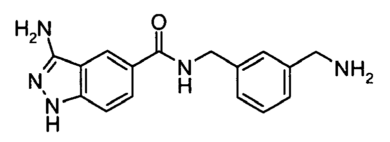 |
| 155 | 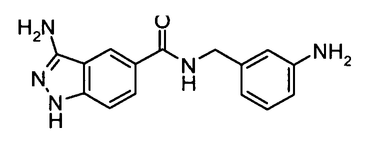 |
| 156 | 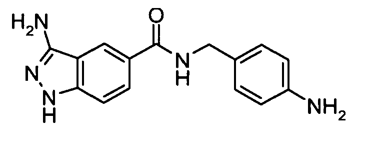 |
| 157 | 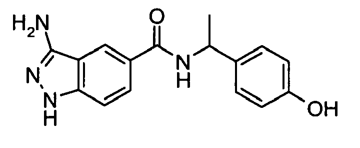 |
| 158 | 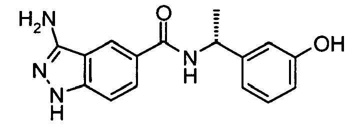 |
| 159 | 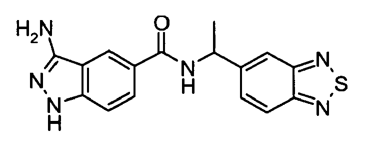 |
| 160 | 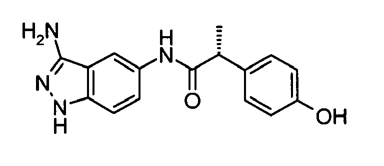 |
| 161 | 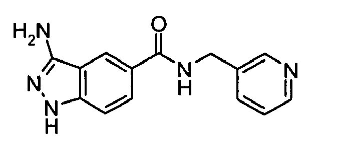 |
| 162 | 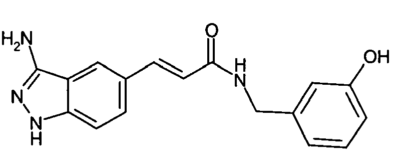 |
| 163 | 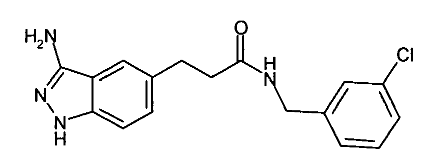 |
| 164 | 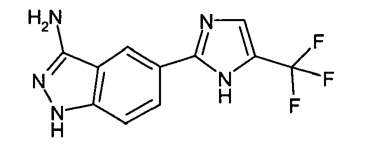 |
| 165 | 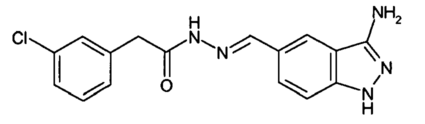 |
| 166 | 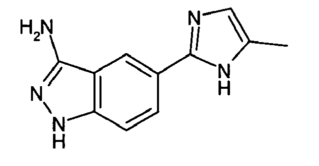 |
| 167 | 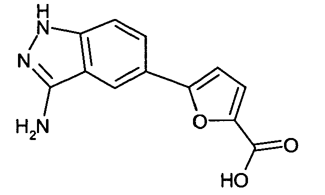 |
| 168 | 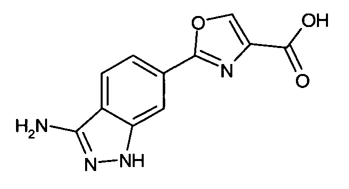 |
| 169 | 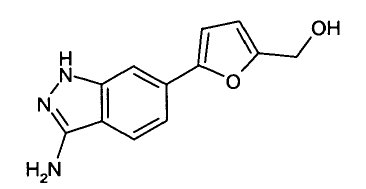 |
| 170 | 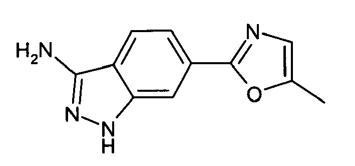 |
| 171 | 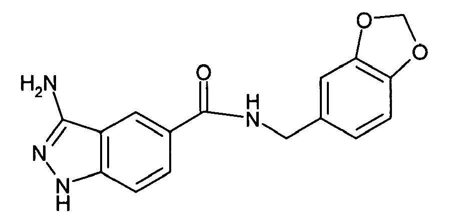 |
| 172 | 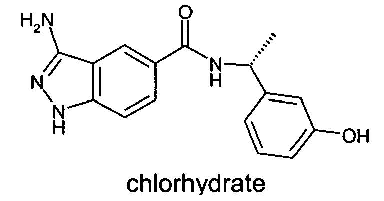 |
| 173 | 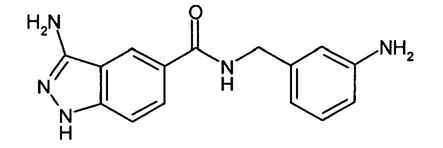 |
| 174 | 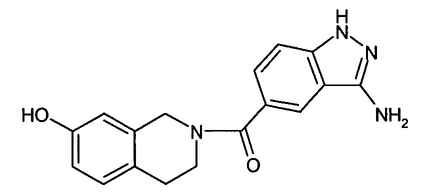 |
| 175 | 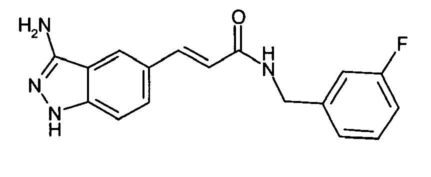 |
| 176 | 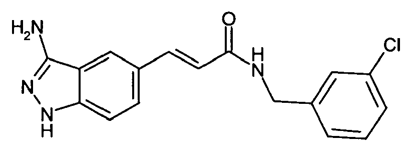 |
| 177 | 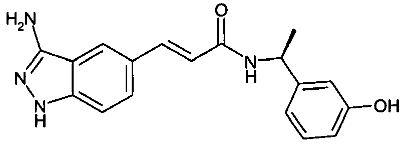 |
| 178 | 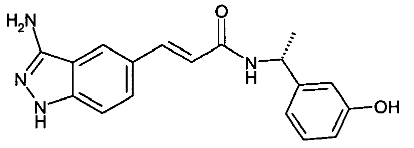 |
| 179 | 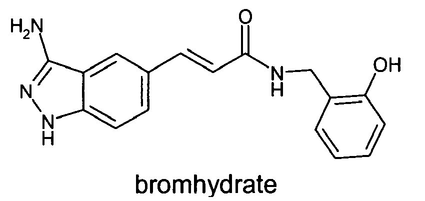 |
| 180 | 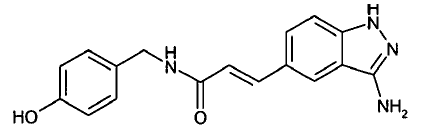 |
| 181 | 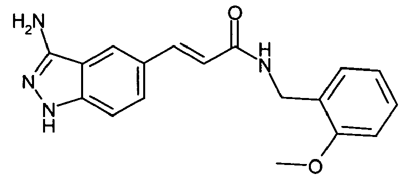 |
| 182 | 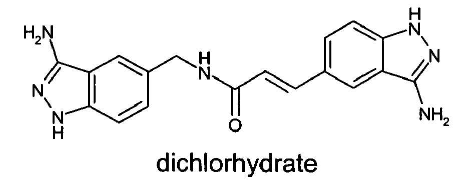 |
| 183 | 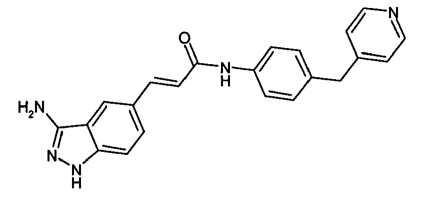 |
| 184 | 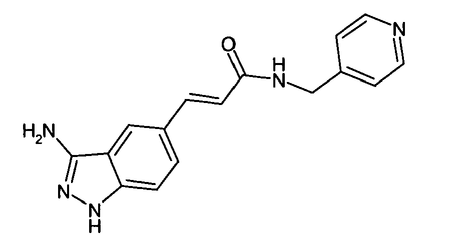 |
| 185 | 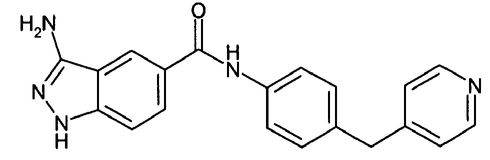 |
| 186 | 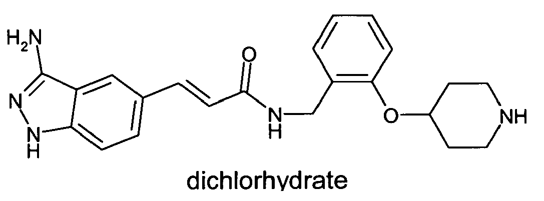 |
| 187 | 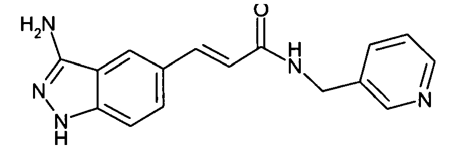 |
| 188 | 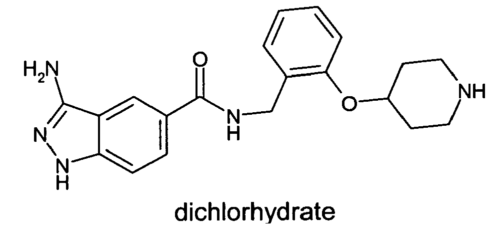 |
| 189 | 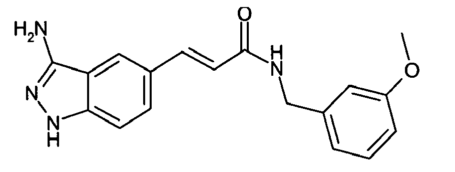 |
| 190 | 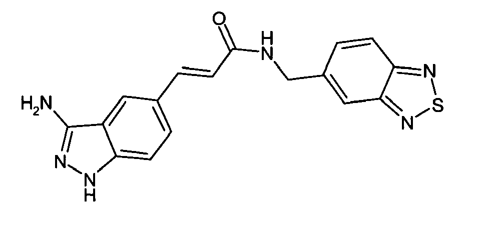 |
| 191 | 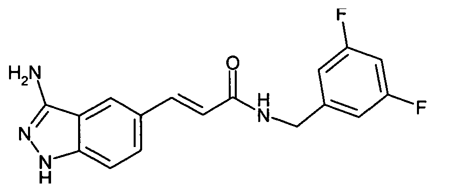 |
| 192 | 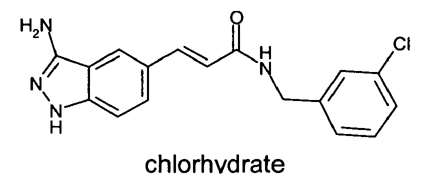 |
| 193 | 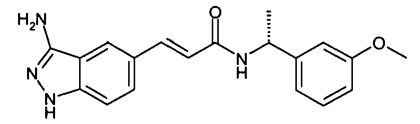 |
| 194 | 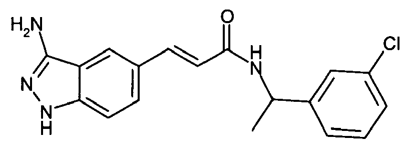 |
| 195 | 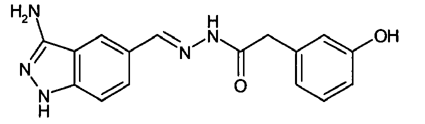 |
| 196 | 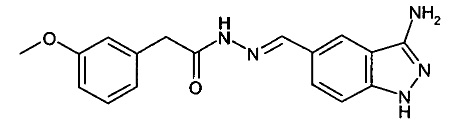 |
| 197 | 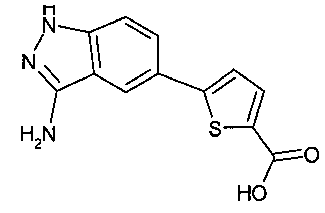 |
| 198 | 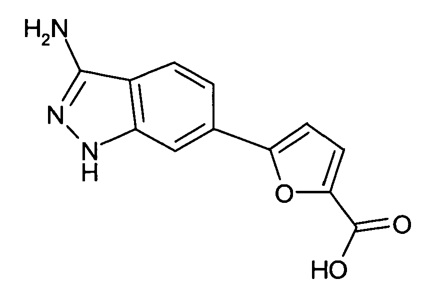 |
| 199 | 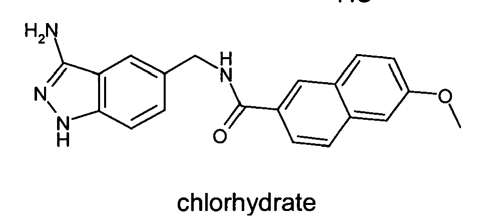 |
| 200 | 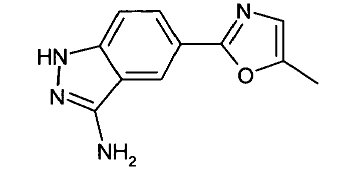 |
| 201 | 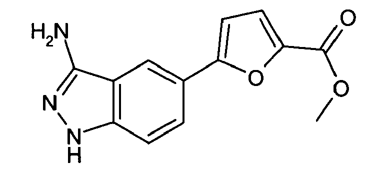 |
et les sels, isomères E,Z, diastéréoisomères, énantiomères, mono-, dihydrates et alcoolates pharmaceutiquement utilisables de ceux-ci, y compris des mélanges de ceux-ci en toutes proportions.

3. Procédé de préparation de composés de formule I selon les revendications 1-13 et de sels, isomères E,Z, diastéréoisomères, énantiomères, mono-, dihydrates et alcoolates pharmaceutiquement utilisables de ceux-ci, y compris des mélanges de ceux-ci en toutes proportions, **caractérisé en ce que**
a) pour la préparation de composés de formule I dans laquelle
W désigne
i) -[C(R³)₂]ₙCONH[C(R³)₂]ₙ- ou
ii) -[C(R³)₂]ₙCONHNH[C(R³)₂]ₙ-,
un composé de formule II 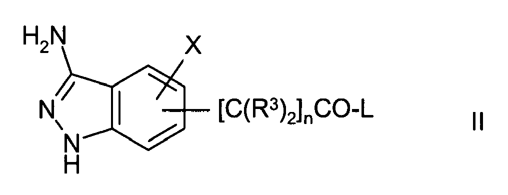 dans laquelle
X, R³ et n ont la signification indiquée selon la revendication 1,
et L désigne CI, Br, I ou un groupement OH libre ou
modifié de manière réactive et fonctionnelle, est réagi avec
i) un composé de formule IIIa
H₂N[C(R³)₂]ₙ-R¹ IIIa
dans laquelle
R¹, R³ et n ont les significations indiquées selon la revendication 1,
ou
ii) un composé de formule IIIb
H₂N-NH[C(R³)₂]ₙ-R¹ IIIb
dans laquelle
R¹, R³ et n ont les significations indiquées selon la revendication 1,
ou
b) pour la préparation de composés de formule I dans laquelle
W désigne -[C(R³)₂]ₙNHCO[C(R³)₂]ₙ-,
un composé de formule IV 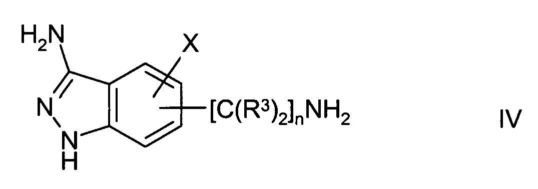 dans laquelle X, R³ et n ont les significations indiquées selon la revendication 1,
est réagi avec un composé de formule V
L-CO-[C(R³)₂]ₙ-R¹ V
dans laquelle
L désigne CI, Br, I ou un groupement OH libre ou modifié de manière réactive et fonctionnelle et
R¹, R³ et n ont les significations indiquées selon la revendication 1,
ou
c) pour la préparation de composés de formule I dans laquelle
W désigne -[C(R³)₂]ₙ-, -[C(R³)₂]ₙO[C(R³)₂]ₙ-, -[C(R³)₂]ₙNR³[C(R³)₂]ₙ- ou -[C(R³)₂]ₙNR¹[C(R³)₂]ₙ-,
un composé de formule VI 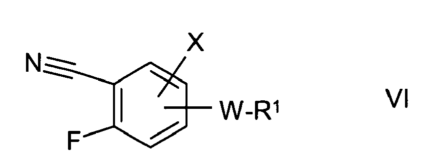 dans laquelle
R¹, W et X ont les significations indiquées selon la revendication 1,
est réagi avec de l'hydrazine,
ou
d) pour la préparation de composés de formule I dans laquelle
W désigne -[C(R³)₂]ₙNHCONH[C(R³)₂]ₙ-,
un composé de formule IV est réagi avec un composé de formule VII
O=C=N-[C(R³)₂]ₙ-R¹ VII
dans laquelle R¹, R³ et n ont les significations indiquées selon la revendication 1,
ou
e) **en ce qu'**ils sont libérés à partir de l'un de leurs dérivés fonctionnels par traitement par un agent de solvolyse ou d'hydrogénolyse,
et/ou
une base ou un acide de formule I est converti(e) en l'un de ses sels.

4. Médicaments comprenant au moins un composé selon les revendications 1-2 et/ou des sels, isomères E,Z, diastéréoisomères, énantiomères, mono-, dihydrates et alcoolates pharmaceutiquement utilisables de celui-ci, y compris des mélanges de ceux-ci en toutes proportions, et éventuellement des excipients et/ou adjuvants.

5. Utilisation de composés selon les revendications 1-2, et de sels, isomères E,Z, diastéréoisomères, énantiomères, mono-, dihydrates et alcoolates pharmaceutiquement utilisables de ceux-ci, y compris des mélanges de ceux-ci en toutes proportions, pour la préparation d'un médicament destiné au traitement et/ou la prophylaxie de maladies dans lesquelles l'inhibition, la régulation et/ou la modulation de la transduction des signaux de kinases joue un rôle.

6. Utilisation selon la revendication 5, où la kinase est la SGK.

7. Utilisation selon la revendication 6 de composés selon les revendications 1-2, et de sels, isomères E,Z, diastéréoisomères, énantiomères, mono-, dihydrates et alcoolates pharmaceutiquement utilisables de ceux-ci, y compris des mélanges de ceux-ci en toutes proportions, pour la préparation d'un médicament destiné au traitement de maladies qui sont influencées par l'inhibition des SGK par les composés selon les revendications 1-2.

8. Utilisation selon la revendication 7 de composés selon les revendications 1-2, et de sels, isomères E,Z, diastéréoisomères, énantiomères, mono-, dihydrates et alcoolates pharmaceutiquement utilisables de ceux-ci, y compris des mélanges de ceux-ci en toutes proportions, pour la préparation d'un médicament destiné au traitement ou à la prévention du diabète, de l'obésité, du syndrome métabolique (dyslipidémie), de l'hypertension systémique et pulmonaire, des maladies cardiovasculaires et des maladies rénales, généralement dans tous les types de fibroses et de processus inflammatoires, du cancer, des cellules tumorales, des métastases tumorales, des coagulopathies, de l'excitabilité neuronale, du glaucome, des cataractes, des infections bactériennes et de la thérapie anti-infectieuse, pour augmenter les capacités d'apprentissage et l'attention, et pour le traitement de l'acouphène.

9. Utilisation selon la revendication 8, dans laquelle le diabète est le diabète sucré, la néphropathie diabétique, la neuropathie diabétique, l'angiopathie diabétique et la microangiopathie.

10. Utilisation selon la revendication 8, dans laquelle les maladies cardiovasculaires sont les fibroses cardiaques après un infarctus du myocarde, l'hypertrophie cardiaque, l'insuffisance cardiaque et l'artériosclérose.

11. Utilisation selon la revendication 8, dans laquelle les maladies rénales sont la glomérulosclérose, la néphrosclérose, la néphrite, la néphropathie et l'altération de l'excrétion des électrolytes.

12. Utilisation selon la revendication 8, dans laquelle les fibroses et les processus inflammatoires sont la cirrhose hépatique, la fibrose pulmonaire, la pancréatite fibrosante, les rhumatismes et les arthroses, la maladie de Crohn, la bronchite chronique, la fibrose induite par une radiothérapie, la sclérodermie, la mucoviscidose, la cicatrisation et la maladie d'Alzheimer.

13. Médicaments comprenant au moins un composé selon les revendications 1-2 et/ou des sels, isomères E,Z, diastéréoisomères, énantiomères, mono-, dihydrates et alcoolates pharmaceutiquement utilisables de celui-ci, y compris des mélanges de ceux-ci en toutes proportions, et au moins un autre composé actif médicamenteux.

14. Ensemble (kit) constitué de conditionnements séparés
(a) d'une quantité efficace d'un composé selon les revendications 1-2 et/ou de sels, isomères E,Z, diastéréoisomères, énantiomères, mono-, dihydrates et alcoolates pharmaceutiquement utilisables de celui-ci, y compris des mélanges de ceux-ci en toutes proportions, et
(b) d'une quantité efficace d'un autre ingrédient actif médicamenteux.

15. Composés intermédiaires choisis parmi le groupe constitué par 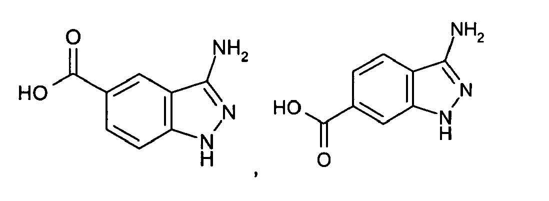 et les sels de ceux-ci.
